# EUROPEAN PATENT APPLICATION

(11) **EP 1 983 048 A1**
(43) Date of publication of application: **22.10.2008**
(21) Application number: 07706883.1
(22) Date of filing: 17.01.2007
(51) Int. Cl.: C12N 15/09, C12N 7/00, C12P 21/02

(54) **NOVEL PROTEIN EXPRESSION SYSTEM**

(30) Priority: 17.01.2006 JP 2006008965
(71) Applicant: Dnavec Corporation, Tsukuba-shi, Ibaraki 305-0856 (JP)
(72) Inventor: YOU, Jun, Tsukuba-shi, Ibaraki 305-0856 (JP); TABATA, Toshiaki, Tsukuba-shi, Ibaraki 305-0856 (JP); INOUE, Makoto, Tsukuba-shi, Ibaraki 305-0856 (JP); SHU, Tsugumine, Tsukuba-shi, Ibaraki 305-0856 (JP); HASEGAWA, Mamoru, Tsukuba-shi, Ibaraki 305-0856 (JP)
(74) Representative: Vossius & Partner
(86) International application number: PCT/JP2007/050556
(87) International publication number: WO 2007/083644

(57) **Abstract**

The present inventors devised a protein expression system with coexisting MiniSeV and SeV particles, and assessed the system for the ability to transfer a gene(s) of interest into target cells, and to express the gene(s) in the target cells. It was shown that the expression system of the present invention had a high ability to transfer a gene(s) of interest into target cells, and a high ability to express the gene(s) in the target cells.

SEQUENCE LISTING

<110> DNAVEC CORPORATION

<120> Novel protein expression system

<130> D4-A0511P

<150> JP 2006-008965
<151> 2006-01-17

<160> 44

<170> PatentIn version 3.3

<210> 1
<211> 24
<212> DNA
<213> Artificial

<220>
<223> an artificially synthesized primer sequence

<400> 1
caggtttgag gatattatac atag 24

<210> 2
<211> 28
<212> DNA
<213> Artificial

<220>
<223> an artificially synthesized primer sequence

<400> 2

caggatttta gtttttctta ctattgtc                                    28

<210>  3
<211>  30
<212>  DNA
<213>  Artificial

<220>
<223>  an artificially synthesized primer sequence

<400>  3
ctcttgtttg gtgggtcggc atggcatctc                                  30

<210>  4
<211>  25
<212>  DNA
<213>  Artificial

<220>
<223>  an artificially synthesized primer sequence

<400>  4
ttagctcact cattaggcac cccag                                       25

<210>  5
<211>  30
<212>  DNA
<213>  Artificial

<220>
<223>  an artificially synthesized primer sequence

<400>  5

gtaagaaaaa ctaaaatcct gtataacttc 30

<210> 6
<211> 30
<212> DNA
<213> Artificial

<220>
<223> an artificially synthesized primer sequence

<400> 6
gccgacccac caaacaagag aaaaaacatg 30

<210> 7
<211> 16362
<212> DNA
<213> Artificial

<220>
<223> an artificially synthesized nucleotide sequence

<400> 7
   accaaacaag agaaaaaaca tgtatgggat atgtaatgaa gttatacagg attttagggt 60
   caaagtatcc accctgagga gcaggttcca gaccctttgc tttgctgcca aagttcacgc 120
   ggccgcagat cttcacgatg gccgggttgt tgagcacctt cgatacattt agctctagga 180
   ggagcgaaag tattaataag tcgggaggag gtgctgttat ccccggccag aggagcacag 240
   tctcagtgtt cgtactaggc ccaagtgtga ctgatgatgc agacaagtta ttcattgcaa 300
   ctaccttcct agctcactca ttggacacag ataagcagca ctctcagaga ggggggttcc 360
   tcgtctctct gcttgccatg gcttacagta gtccagaatt gtacttgaca acaaacggag 420
   taaacgccga tgtcaaatat gtgatctaca acatagagaa agaccctaag aggacgaaga 480
   cagacggatt cattgtgaag acgagagata tggaatatga gaggaccaca gaatggctgt 540
   ttggacctat ggtcaacaag agcccactct tccagggtca acgggatgct gcagaccctg 600
   acacactcct tcaaatctat gggtatcctg catgcctagg agcaataatt gtccaagtct 660
   ggattgtgct ggtgaaggcc atcacaagca gcgccggctt aaggaaaggg ttcttcaaca 720
   ggttagaggc gttcagacaa gacggcaccg tgaaaggtgc cttagttttc actggggaga 780
   cagttgaggg gataggctcg gttatgagat ctcagcaaag ccttgtatct ctcatggttg 840
   agacccttgt gactatgaat actgcaagat ctgatctcac cacattagag aagaacatcc 900
   agatcgttgg gaactacatc cgagatgcag ggctggcttc cttcatgaac actattaaat 960
   atggggtgga aacaaagatg gcagctctaa cgttgtcaaa cctgaggccc gatattaata 1020
   agcttagaag cctcatagac acctacctgt caaaaggccc cagagctccc tttatctgta 1080
   tcctcaagga ccctgttcat ggtgaatttg ctccaggcaa ttatcctgca ctatggagtt 1140
   acgccatggg agtcgccgtc gtacagaaca aggcaatgca gcagtacgtc acagggagga 1200
   cataccttga tatggaaatg ttcttactag gacaagccgt ggcaaaggat gctgaatcga 1260
   agatcagcag tgccttggaa gatgagttag gagtgacgga tacagccaag gggaggctca 1320
   gacatcatct ggcaaacttg tccggtgggg atggtgctta ccacaaacca acaggcggtg 1380
   gtgcaattga ggtagctcta gacaatgccg acatcgacct agaaacaaaa gcccatgcgg 1440
   accaggacgc taggggttgg ggtggagata gtggtgaaag atgggcacgt caggtgagtg 1500
   gtggccactt tgtcacacta catggggctg aacggttaga ggaggaaacc aatgatgagg 1560
   atgtatcaga catagagaga agaatagcca tgagactcgc agagagacgg caagaggatt 1620
   ctgcaaccca tggagatgaa ggccgcaata acggtgtcga tcatgacgaa gatgacgatg 1680
   ccgcagcagt agctgggata ggaggaatct aggatcatac gaggcttcaa ggtacttgat 1740
   ccgtagtaag aaaaacttag ggtgaaagtt catccaccga tcggctcagg caaggccaca 1800
   cccaacccca ccgaccacac ccagcagtcg agacagccac ggcttcggct acacttaccg 1860
   catggatcaa gatgccttca ttcttaaaga agattctgaa gttgagaggg aggcgccagg 1920
   aggacgagag tcgctctcgg atgttatcgg attcctcgat gctgtcctgt cgagtgaacc 1980
   aactgacatc ggaggggaca gaagctggct ccacaacacc atcaacactc cccaaggacc 2040
   aggctctgct catagagcca aaagtgaggg cgaaggagaa gtctcaacac cgtcgaccca 2100
   agataatcga tcaggtgagg agagtagagt ctctgggaga acaagcaagc cagaggcaga 2160
   agcacatgct ggaaaccttg ataaacaaaa tatacaccgg gcctttgggg gaagaactgg 2220
   tacaaactct gtatctcagg atctgggcga tggaggagac tccggaatcc ttgaaaatcc 2280
   tccaaatgag agaggatatc cgagatcagg tattgaagat gaaaacagag agatggctgc 2340
   gcaccctgat aagaggggag aagaccaagc tgaaggactt ccagaagagg tacgaggaag 2400
   tacatcccta cctgatgaag gagaaggtgg agcaagtaat aatggaagaa gcatggagcc 2460
   tggcagctca catagtgcaa gagtaactgg ggtcctggtg attcctagcc ccgaacttga 2520
   agaggctgtg ctacggagga acaaaagaag acctaccaac agtgggtcca aacctcttac 2580
   tccagcaacc gtgcctggca cccggtcccc accgctgaat cgttacaaca gcacagggtc 2640
   accaccagga aaacccccat ctacacagga tgagcacatc aactctgggg acacccccgc 2700
   cgtcagggtc aaagaccgga aaccaccaat agggacccgc tctgtctcag attgtccagc 2760
   caacggccgc ccaatccacc cgggtctaga gaccgactca acaaaaaagg gcataggaga 2820
   gaacacatca tctatgaaag agatggctac attgttgacg agtcttggtg taatccagtc 2880
   tgctcaagaa ttcgaatcat cccgagacgc gagttatgtg tttgcaagac gtgccctaaa 2940
   gtctgcaaac tatgcagaga tgacattcaa tgtatgcggc ctgatccttt ctgccgagaa 3000
   atcttccgct cgtaaggtag atgagaacaa acaactgctc aaacagatcc aagagagcgt 3060
   ggaatcattc cgggatattt acaagagatt ctctgagtat cagaaagaac agaactcatt 3120
   gctgatgtcc aacctatcta cacttcatat catcacagat agaggtggca agactgacaa 3180
   cacagactcc cttacaaggt ccccctccgt ttttgcaaaa tcaaaagaga acaagactaa 3240
   ggctaccagg tttgacccat ctatggagac cctagaagat atgaagtaca aaccggacct 3300
   aatccgagag gatgaattta gagatgagat ccgcaacccg gtgtaccaag agagggacac 3360
   agaacccagg gcctcaaacg catcacgtct cctcccctcc aaagagaagc ccacaatgca 3420
   ctctctcagg ctcgtcatag agagcagtcc cctaagcaga gctgagaaag tagcatatgt 3480
   gaaatcatta tccaagtgca agacagacca agaggttaag gcagtcatgg aactcgtaga 3540
   agaggacata gagtcactga ccaactagat cccgggtgag gcatcctacc atcctcagtc 3600
   atagagagat ccaatctacc atcagcatca gccagtaaag attaagaaaa acttagggtg 3660
   aaagaaattt cacctaacac ggcgcaatgg cagatatcta tagattccct aagttctcat 3720
   atgaggataa cggtactgtg gagcccctgc ctctgagaac tggtccggat aagaaagcca 3780
   tcccccacat caggattgtc aaggtaggag accctcctaa acatggagtg agatacctag 3840
   atttattgct cttgggtttc tttgagacac cgaaacaaac aaccaatcta gggagcgtat 3900
   ctgacttgac agagccgacc agctactcaa tatgcggctc cgggtcgtta cccataggtg 3960
   tggccaaata ctacgggact gatcaggaac tcttaaaggc ctgcaccgat ctcagaatta 4020
   cggtgaggag gactgttcga gcaggagaga tgatcgtata catggtggat tcgattggtg 4080
   ctccactcct accatggtca ggcaggctga gacagggaat gatatttaat gcaaacaagg 4140
   tcgcactagc tccccaatgc ctccctgtgg acaaggacat aagactcaga gtggtgtttg 4200
   tcaatgggac atctctaggg gcaatcacca tagccaagat cccaaagacc cttgcagacc 4260
   ttgcattgcc caactctata tctgttaatt tactggtgac actcaagacc gggatctcca 4320
   cagaacaaaa gggggtactc ccagtacttg atgatcaagg ggagaaaaag ctcaatttta 4380
   tggtgcacct cgggttgatc aggagaaagg tcgggaagat atactctgtt gagtactgca 4440
   agagcaagat tgagagaatg cggctgattt tctcacttgg gttaatcggc ggtataagct 4500
   tccatgttca ggttaatggg acactatcta agacattcat gagtcagctc gcatggaaga 4560
   gggcagtctg cttcccatta atggatgtga atccccatat gaacatggtg atttgggcgg 4620
   catctgtaga aatcacaggc gtcgatgcgg tgttccaacc ggccatccct cgtgatttcc 4680
   gctactaccc taatgttgtg gctaagaaca tcggaaggat cagaaagctg taaatgtgca 4740
   cccatcagag acctgcgaca atgccccaag cagacaccac ctggcagtcg gagccaccgg 4800
   gtcactcctt gtcttaaata agaaaaactt agggataaag tcccttgtga gtgcttggtt 4860
   gcaaaactct ccacctggta caagcacaga tcatggatgg tgataggggc aaacgtgact 4920
   cgtactggtc tacttctcct agtggtagca ccacaaaacc agcatcaggt tgggagaggt 4980
   caagtaaagc cgacacatgg ttgctgattc tctcattcac ccagtgggct ttgtcaattg 5040
   ccacagtgat catctgtatc ataatttctg ctagacaagg gtatagtatg aaagagtact 5100
   caatgactgt agaggcattg aacatgagca gcagggaggt gaaagagtca cttaccagtc 5160
   taataaggca agaggttata gcaagggctg tcaacattca gagctctgtg caaaccggaa 5220
   tcccagtctt gttgaacaaa aacagcaggg atgtcatcca gatgattgat aagtcgtgca 5280
   gcagacaaga gctcactcag cactgtgaga gtacgatcgc agtccaccat gccgatggaa 5340
   ttgccccact tgagccacat agtttctgga gatgccctgt cggagaaccg tatcttagct 5400
   cagatcctga aatctcattg ctgcctggtc cgagcttgtt atctggttct acaacgatct 5460
   ctggatgtgt taggctccct tcactctcaa ttggcgaggc aatctatgcc tattcatcaa 5520
   atctcattac acaaggttgt gctgacatag ggaaatcata tcaggtcctg cagctagggt 5580
   acatatcact caattcagat atgttccctg atcttaaccc cgtagtgtcc cacacttatg 5640
   acatcaacga caatcggaaa tcatgctctg tggtggcaac cgggactagg ggttatcagc 5700
   tttgctccat gccgactgta gacgaaagaa ccgactactc tagtgatggt attgaggatc 5760
   tggtccttga tgtcctggat ctcaaaggga gaactaagtc tcaccggtat cgcaacagcg 5820
   aggtagatct tgatcacccg ttctctgcac tataccccag tgtaggcaac ggcattgcaa 5880
   cagaaggctc attgatattt cttgggtatg gtggactaac cacccctctg cagggtgata 5940
   caaaatgtag gacccaagga tgccaacagg tgtcgcaaga cacatgcaat gaggctctga 6000
   aaattacatg gctaggaggg aaacaggtgg tcagcgtgat catccaggtc aatgactatc 6060
   tctcagagag gccaaagata agagtcacaa ccattccaat cactcaaaac tatctcgggg 6120
   cggaaggtag attattaaaa ttgggtgatc gggtgtacat ctatacaaga tcatcaggct 6180
   ggcactctca actgcagata ggagtacttg atgtcagcca ccctttgact atcaactgga 6240
   cacctcatga agccttgtct agaccaggaa ataaagagtg caattggtac aataagtgtc 6300
   cgaaggaatg catatcaggc gtatacactg atgcttatcc attgtcccct gatgcagcta 6360
   acgtcgctac cgtcacgcta tatgccaata catcgcgtgt caacccaaca atcatgtatt 6420
   ctaacactac taacattata aatatgttaa ggataaagga tgttcaatta gaggctgcat 6480
   ataccacgac atcgtgtatc acgcattttg gtaaaggcta ctgctttcac atcatcgaga 6540
   tcaatcagaa gagcctgaat accttacagc cgatgctctt taagactagc atccctaaat 6600
   tatgcaaggc cgagtcttaa atttaactga ctagcaggct tgtcggcctt gctgacacta 6660
   gagtcatctc cgaacatcca caatatctct cagtctctta cgtctctcac agtattaaga 6720
   aaaacccagg gtgaatggga agcttgccat aggtcatgga tgggcaggag tcctcccaaa 6780
   acccttctga catactctat ccagaatgcc acctgaactc tcccatagtc agggggaaga 6840
   tagcacagtt gcacgtcttg ttagatgtga accagcccta cagactgaag gacgacagca 6900
   taataaatat tacaaagcac aaaattagga acggaggatt gtccccccgt caaattaaga 6960
   tcaggtctct gggtaaggct cttcaacgca caataaagga tttagaccga tacacgtttg 7020
   aaccgtaccc aacctactct caggaattac ttaggcttga tataccagag atatgtgaca 7080
   aaatccgatc cgtcttcgcg gtctcggatc ggctgaccag ggagttatct agtgggttcc 7140
   aggatctttg gttgaatatc ttcaagcaac taggcaatat agaaggaaga gaggggtacg 7200
   atccgttgca ggatatcggc accatcccgg agataactga taagtacagc aggaatagat 7260
   ggtataggcc attcctaact tggttcagca tcaaatatga catgcggtgg atgcagaaga 7320
   ccagaccggg gggacccctc gatacctcta attcacataa cctcctagaa tgcaaatcat 7380
   acactctagt aacatacgga gatcttgtca tgatactgaa caagttgaca ttgacagggt 7440
   atatcctaac ccctgagctg gtcttgatgt attgtgatgt tgtagaagga aggtggaata 7500
   tgtctgctgc agggcatcta gataagaagt ccattgggat aacaagcaaa ggtgaggaat 7560
   tatgggaact agtggattcc ctcttctcaa gtcttggaga ggaaatatac aatgtcatcg 7620
   cactattgga gcccctatca cttgctctca tacaactaaa tgatcctgtt atacctctac 7680
   gtggggcatt tatgaggcat gtgttgacag agctacagac tgttttaaca agtagagacg 7740
   tgtacacaga tgctgaagca gacactattg tggagtcgtt actcgccatt ttccatggaa 7800
   cctctattga tgagaaagca gagatctttt ccttctttag gacatttggc caccccagct 7860
   tagaggctgt cactgccgcc gacaaggtaa gggcccatat gtatgcacaa aaggcaataa 7920
   agcttaagac cctatacgag tgtcatgcag ttttttgcac tatcatcata aatgggtata 7980
   gagagaggca tggcggacag tggcccccct gtgacttccc tgatcacgtg tgtctagaac 8040
   taaggaacgc tcaagggtcc aatacggcaa tctcttatga atgtgctgta gacaactata 8100
   caagtttcat aggcttcaag tttcggaagt ttatagaacc acaactagat gaagatctca 8160
   caatatatat gaaagacaaa gcactatccc ccaggaagga ggcatgggac tctgtatacc 8220
   cggatagtaa tctgtactat aaagccccag agtctgaaga gacccggcgg cttattgaag 8280
   tgttcataaa tgatgagaat ttcaacccag aagaaattat caattatgtg gagtcaggag 8340
   attggttgaa agacgaggag ttcaacatct cgtacagtct caaagagaaa gagatcaagc 8400
   aagagggtcg tctattcgca aaaatgactt ataagatgcg agccgtacag gtgctggcag 8460
   agacactact ggctaaagga ataggagagc tattcagcga aaatgggatg gttaaaggag 8520
   agatagacct acttaaaaga ttgactactc tttctgtctc aggcgtcccc aggactgatt 8580
   cagtgtacaa taactctaaa tcatcagaga agagaaacga aggcatggaa aataagaact 8640
   ctggggggta ctgggacgaa aagaagaggt ccagacatga attcaaggca acagattcat 8700
   caacagacgg ctatgaaacg ttaagttgct tcctcacaac agacctcaag aaatactgct 8760
   taaactggag atttgagagt actgcattgt ttggtcagag atgcaacgag atatttggct 8820
   tcaagacctt ctttaactgg atgcatccag tccttgaaag gtgtacaata tatgttggag 8880
   atccttactg tccagtcgcc gaccggatgc atcgacaact ccaggatcat gcagactctg 8940
   gcattttcat acataatcct agggggggca tagaaggtta ctgccagaag ctgtggacct 9000
   taatctcaat cagtgcaatc cacctagcag ctgtgagagt gggtgtcagg gtctctgcaa 9060
   tggttcaggg tgacaatcaa gctatagccg tgacatcaag agtacctgta gctcagactt 9120
   acaagcagaa gaaaaatcat gtctatgagg agatcaccaa atatttcggt gctctaagac 9180
   acgtcatgtt tgatgtaggg cacgagctaa aattgaacga gaccatcatt agtagcaaga 9240
   tgtttgtcta tagtaaaagg atatactatg atgggaagat tttaccacag tgcctgaaag 9300
   ccttgaccaa gtgtgtattc tggtccgaga cactggtaga tgaaaacaga tctgcttgtt 9360
   cgaacatctc aacatccata gcaaaagcta tcgaaaatgg gtattctcct atactaggct 9420
   actgcattgc gttgtataag acctgtcagc aggtgtgcat atcactaggg atgactataa 9480
   atccaactat cagcccgacc gtaagagatc aatactttaa gggtaagaat tggctgagat 9540
   gtgcagtgtt gattccagca aatgttggag gattcaacta catgtctaca tctagatgct 9600
   ttgttagaaa tattggagac cccgcagtag cagccctagc tgatctcaaa agattcatca 9660
   gagcggatct gttagacaag caggtattat acagggtcat gaatcaagaa cccggtgact 9720
   ctagttttct agattgggct tcagaccctt attcgtgtaa cctcccgcat tctcagagta 9780
   taactacgat tataaagaat atcactgcta gatctgtgct gcaggaatcc ccgaatcctc 9840
   tactgtctgg tctcttcacc gagactagtg gagaagagga tctcaacctg gcctcgttcc 9900
   ttatggaccg gaaagtcatc ctgccgagag tggctcatga gatcctgggt aattccttaa 9960
   ctggagttag ggaggcgatt gcagggatgc ttgatacgac caagtctcta gtgagagcca 10020
   gcgttaggaa aggaggatta tcatatggga tattgaggag gcttgtcaat tatgatctat 10080
   tgcagtacga gacactgact agaactctca ggaaaccggt gaaagacaac atcgaatatg 10140
   agtatatgtg ttcagttgag ctagctgtcg gtctaaggca gaaaatgtgg atccacctga 10200
   cttacgggag acccatacat gggctagaaa caccagaccc tttagagctc ttgaggggaa 10260
   tatttatcga aggttcagag gtgtgcaagc tttgcaggtc tgaaggagca gaccccatct 10320
   atacatggtt ctatcttcct gacaatatag acctggacac gcttacaaac ggatgtccgg 10380
   ctataagaat cccctatttt ggatcagcca ctgatgaaag gtcggaagcc caactcgggt 10440
   atgtaagaaa tctaagcaaa cccgcaaagg cggccatccg gatagctatg gtgtatacgt 10500
   gggcctacgg gactgatgag atatcgtgga tggaagccgc tcttatagcc caaacaagag 10560
   ctaatctgag cttagagaat ctaaagctgc tgactcctgt ttcaacctcc actaatctat 10620
   ctcataggtt gaaagatacg gcaacccaga tgaagttctc tagtgcaaca ctagtccgtg 10680
   caagtcggtt cataacaata tcaaatgata acatggcact caaagaagca ggggagtcga 10740
   aggatactaa tctcgtgtat cagcagatta tgctaactgg gctaagcttg ttcgagttca 10800
   atatgagata taagaaaggt tccttaggga agccactgat attgcactta catcttaata 10860
   acgggtgctg tataatggag tccccacagg aggcgaatat ccccccaagg tccacattag 10920
   atttagagat tacacaagag aacaataaat tgatctatga tcctgatcca ctcaaggatg 10980
   tggaccttga gctatttagc aaggtcagag atgttgtaca cacagttgac atgacttatt 11040
   ggtcagatga tgaagttatc agagcaacca gtatctgtac tgcaatgacg atagctgata 11100
   caatgtctca attagataga gacaacttaa aagagatgat cgcactagta aatgacgatg 11160
   atgtcaacag cttgattact gagtttatgg tgattgatgt tcctttattt tgctcaacgt 11220
   tcgggggtat tctagtcaat cagtttgcat actcactcta cggcttaaac atcagaggaa 11280
   gggaagaaat atggggacat gtagtccgga ttcttaaaga tacctcccac gcagttttaa 11340
   aagtcttatc taatgctcta tctcatccca aaatcttcaa acgattctgg aatgcaggtg 11400
   tcgtggaacc tgtgtatggg cctaacctct caaatcagga taagatactc ttggccctct 11460
   ctgtctgtga atattctgtg gatctattca tgcacgattg gcaagggggt gtaccgcttg 11520
   agatctttat ctgtgacaat gacccagatg tggccgacat gaggaggtcc tctttcttgg 11580
   caagacatct tgcataccta tgcagcttgg cagagatatc tagggatggg ccaagattag 11640
   aatcaatgaa ctctctagag aggctcgagt cactaaagag ttacctggaa ctcacatttc 11700
   ttgatgaccc ggtactgagg tacagtcagt tgactggcct agtcatcaaa gtattcccat 11760
   ctactttgac ctatatccgg aagtcatcta taaaagtgtt aaggacaaga ggtataggag 11820
   tccctgaagt cttagaagat tgggatcccg aggcagataa tgcactgtta gatggtatcg 11880
   cggcagaaat acaacagaat attcctttgg gacatcagac tagagcccct ttttgggggt 11940
   tgagagtatc caagtcacag gtactgcgtc tccgggggta caaggagatc acaagaggtg 12000
   agataggcag atcaggtgtt ggtctgacgt taccattcga tggaagatat ctatctcacc 12060
   agctgaggct ctttggcatc aacagtacta gctgcttgaa agcacttgaa cttacctacc 12120
   tattgagccc cttagttgac aaggataaag ataggctata tttaggggaa ggagctgggg 12180
   ccatgctttc ctgttatgac gctactcttg gcccatgcat caactattat aactcagggg 12240
   tatactcttg tgatgtcaat gggcagagag agttaaatat atatcctgct gaggtggcac 12300
   tagtgggaaa gaaattaaac aatgttacta gtctgggtca aagagttaaa gtgttattca 12360
   acgggaatcc tggctcgaca tggattggga atgatgagtg tgaggctttg atttggaatg 12420
   aattacagaa tagctcgata ggcctagtcc actgtgacat ggagggagga gatcataagg 12480
   atgatcaagt tgtactgcat gagcattaca gtgtaatccg gatcgcgtat ctggtggggg 12540
   atcgagacgt tgtgcttata agcaagattg ctcccaggct gggcacggat tggaccaggc 12600
   agctcagcct atatctgaga tactgggacg aggttaacct aatagtgctt aaaacatcta 12660
   accctgcttc cacagagatg tatctcctat cgaggcaccc caaatctgac attatagagg 12720
   acagcaagac agtgttagct agtctcctcc ctttgtcaaa agaagatagc atcaagatag 12780
   aaaagtggat cttaatagag aaggcaaagg ctcacgaatg ggttactcgg gaattgagag 12840
   aaggaagctc ttcatcaggg atgcttagac cttaccatca agcactgcag acgtttggct 12900
   ttgaaccaaa cttgtataaa ttgagcagag atttcttgtc caccatgaac atagctgata 12960
   cacacaactg catgatagct ttcaacaggg ttttgaagga tacaatcttc gaatgggcta 13020
   gaataactga gtcagataaa aggcttaaac taactggtaa gtatgacctg tatcctgtga 13080
   gagattcagg caagttgaag acaatttcta gaagacttgt gctatcttgg atatctttat 13140
   ctatgtccac aagattggta actgggtcat tccctgacca gaagtttgaa gcaagacttc 13200
   aattgggaat agtttcatta tcatcccgtg aaatcaggaa cctgagggtt atcacaaaaa 13260
   ctttattaga caggtttgag gatattatac atagtataac gtatagattc ctcaccaaag 13320
   aaataaagat tttgatgaag attttagggg cagtcaagat gttcggggcc aggcaaaatg 13380
   aatacacgac cgtgattgat gatggatcac taggtgatat cgagccatat gacagctcgt 13440
   aataattagt ccctatcgtg cagaacgatc gaagctccgc ggtacctgga agtcttggac 13500
   ttgtccatat gacaatagta agaaaaacta aaatcctgta taacttcatt acatatccca 13560
   tacatgtttt ttctcttgtt tggtgggtcg gcatggcatc tccacctcct cgcggtccga 13620
   cctgggcatc cgaaggagga cgcacgtcca ctcggatggc taagggagag ctacgcgtgg 13680
   atccccggga attccgtaat catggtcata gctgtttcct gtgtgaaatt gttatccgct 13740
   cacaattcca cacaacatac gagccggaag cataaagtgt aaagcctggg gtgcctaatg 13800
   agtgagctaa ctcacattaa ttgcgttgcg ctcactgccc gctttccagt cgggaaacct 13860
   gtcgtgccag ctgcattaat gaatcggcca acgcgcgggg agaggcggtt tgcgtattgg 13920
   gcgctcttcc gcttcctcgc tcactgactc gctgcgctcg gtcgttcggc tgcggcgagc 13980
   ggtatcagct cactcaaagg cggtaatacg gttatccaca gaatcagggg ataacgcagg 14040
   aaagaacatg tgagcaaaag gccagcaaaa ggccaggaac cgtaaaaagg ccgcgttgct 14100
   ggcgtttttc cataggctcc gcccccctga cgagcatcac aaaaatcgac gctcaagtca 14160
   gaggtggcga aacccgacag gactataaag ataccaggcg tttccccctg gaagctccct 14220
   cgtgcgctct cctgttccga ccctgccgct taccggatac ctgtccgcct ttctcccttc 14280
   gggaagcgtg gcgctttctc atagctcacg ctgtaggtat ctcagttcgg tgtaggtcgt 14340
   tcgctccaag ctgggctgtg tgcacgaacc ccccgttcag cccgaccgct gcgccttatc 14400
   cggtaactat cgtcttgagt ccaacccggt aagacacgac ttatcgccac tggcagcagc 14460
   cactggtaac aggattagca gagcgaggta tgtaggcggt gctacagagt tcttgaagtg 14520
   gtggcctaac tacggctaca ctagaagaac agtatttggt atctgcgctc tgctgaagcc 14580
   agttaccttc ggaaaaagag ttggtagctc ttgatccggc aaacaaacca ccgctggtag 14640
   cggtggtttt tttgtttgca agcagcagat tacgcgcaga aaaaaaggat ctcaagaaga 14700
   tcctttgatc ttttctacgg ggtctgacgc tcagtggaac gaaaactcac gttaagggat 14760
   tttggtcatg agattatcaa aaaggatctt cacctagatc cttttaaatt aaaaatgaag 14820
   ttttaaatca atctaaagta tatatgagta aacttggtct gacagttacc aatgcttaat 14880
   cagtgaggca cctatctcag cgatctgtct atttcgttca tccatagttg cctgactccc 14940
   cgtcgtgtag ataactacga tacgggaggg cttaccatct ggccccagtg ctgcaatgat 15000
   accgcgagac ccacgctcac cggctccaga tttatcagca ataaaccagc cagccggaag 15060
   ggccgagcgc agaagtggtc ctgcaacttt atccgcctcc atccagtcta ttaattgttg 15120
   ccgggaagct agagtaagta gttcgccagt taatagtttg cgcaacgttg ttgccattgc 15180
   tacaggcatc gtggtgtcac gctcgtcgtt tggtatggct tcattcagct ccggttccca 15240
   acgatcaagg cgagttacat gatcccccat gttgtgcaaa aaagcggtta gctccttcgg 15300
   tcctccgatc gttgtcagaa gtaagttggc cgcagtgtta tcactcatgg ttatggcagc 15360
   actgcataat tctcttactg tcatgccatc cgtaagatgc ttttctgtga ctggtgagta 15420
   ctcaaccaag tcattctgag aatagtgtat gcggcgaccg agttgctctt gcccggcgtc 15480
   aatacgggat aataccgcgc cacatagcag aactttaaaa gtgctcatca ttggaaaacg 15540
   ttcttcgggg cgaaaactct caaggatctt accgctgttg agatccagtt cgatgtaacc 15600
   cactcgtgca cccaactgat cttcagcatc ttttactttc accagcgttt ctgggtgagc 15660
   aaaaacagga aggcaaaatg ccgcaaaaaa gggaataagg gcgacacgga aatgttgaat 15720
   actcatactc ttcctttttc aatattattg aagcatttat cagggttatt gtctcatgag 15780
   cggatacata tttgaatgta tttagaaaaa taaacaaata ggggttccgc gcacatttcc 15840
   ccgaaaagtg ccacctgacg tctaagaaac cattattatc atgacattaa cctataaaaa 15900
   taggcgtatc acgaggccct ttcgtctcgc gcgtttcggt gatgacggtg aaaacctctg 15960
   acacatgcag ctcccggaga cggtcacagc ttgtctgtaa gcggatgccg ggagcagaca 16020
   agcccgtcag ggcgcgtcag cgggtgttgg cgggtgtcgg ggctggctta actatgcggc 16080
   atcagagcag attgtactga gagtgcacca tatgcggtgt gaaataccgc acagatgcgt 16140
   aaggagaaaa taccgcatca ggcgccattc gccattcagg ctgcgcaact gttgggaagg 16200
   gcgatcggtg cgggcctctt cgctattacg ccagctggcg aaagggggat gtgctgcaag 16260
   gcgattaagt tgggtaacgc cagggttttc ccagtcacga cgttgtaaaa cgacggccag 16320
   tgccaagctc gcgtcgtacg ggccctaata cgactcacta ta 16362

<210> 8
<211> 24
<212> DNA
<213> Artificial

<220>
<223>  an artificially synthesized primer sequence

<400>  8
atgggtatcc tgcatgccta ggag                                              24

<210>  9
<211>  21
<212>  DNA
<213>  Artificial

<220>
<223>  an artificially synthesized primer sequence

<400>  9
atcgattatc ttgggtcgac g                                                 21

<210>  10
<211>  1493
<212>  DNA
<213>  Artificial

<220>
<223>  an artificially synthesized nucleotide sequence

<400>  10
atgggtatcc tgcatgccta ggagcaataa ttgtccaagt ctggattgtg ctggtgaagg      60

ccatcacaag cagcgccggc ttaaggaaag ggttcttcaa caggttagag gcgttcagac     120

aagacggcac cgtgaaaggt gccttagttt tcactgggga gacagttgag gggataggct     180

cggttatgag atctcagcaa agccttgtat ctctcatggt tgagaccctt gtgactatga     240

atactgcaag atctgatctc accacattag agaagaacat ccagatcgtt gggaactaca 300

tccgagatgc agggctggct tccttcatga acactattaa atatggggtg gaaacaaaga 360

tggcagctct aacgttgtca aacctgaggc ccgatattaa taagcttaga agcctcatag 420

acacctacct gtcaaaaggc cccagagctc cctttatctg tatcctcaag gaccctgttc 480

atggtgaatt tgctccaggc aattatcctg cactatggag ttacgccatg ggagtcgccg 540

tcgtacagaa caaggcaatg cagcagtacg tcacagggag gacatacctt gatatggaaa 600

tgttcttact aggacaagcc gtggcaaagg atgctgaatc gaagatcagc agtgccttgg 660

aagatgagtt aggagtgacg gatacagcca aggggaggct cagacatcat ctggcaaact 720

tgtccggtgg ggatggtgct taccacaaac caacaggcgg tggtgcaatt gaggtagctc 780

tagacaatgc cgacatcgac ctagaaacaa aagcccatgc ggaccaggac gctaggggtt 840

ggggtggaga tagtggtgaa agatgggcac gtcaggtgag tggtggccac tttgtcacac 900

tacatggggc tgaacggtta gaggaggaaa ccaatgatga ggatgtatca gacatagaga 960

gaagaatagc catgagactc gcagagagac ggcaagagga ttctgcaacc catggagatg 1020

aaggccgcaa taacggtgtc gatcatgacg aagatgacga tgccgcagca gtagctggga 1080

taggaggaat ctaggatcat acgaggcttc aaggtacttg atccgtagta agaaaaactt 1140

agggtgaaag ttcatccacc gatcggctca ggcaaggcca cacccaaccc caccgaccac 1200

acccagcagt cgagacagcc acggcttcgg ctacacttac cgcatggatc aagatgcctt 1260

cattctgggg gaggattctg gggtggagag gggggcgccg gggggacggg agtcgctctc 1320

ggacgtggtc gggttcctcg acgctgtcct gtcgggtggg ccggctgaca tcggggggga 1380

cgggggctgg ctccacagca ccatcggcgc tccccgggga ccgggctctg ctcatagagc 1440

caaaagtgag ggcgaaggag aagtctcaac accgtcgacc caagataatc gat 1493

<210> 11
<211> 16362
<212> DNA
<213> Artificial

<220>
<223> an artificially synthesized nucleotide sequence

<400> 11
   accaaacaag agaaaaaaca tgtatgggat atgtaatgaa gttatacagg attttagggt 60
   caaagtatcc accctgagga gcaggttcca gaccctttgc tttgctgcca aagttcacgc 120
   ggccgcagat cttcacgatg gccgggttgt tgagcacctt cgatacattt agctctagga 180
   ggagcgaaag tattaataag tcgggaggag gtgctgttat ccccggccag aggagcacag 240
   tctcagtgtt cgtactaggc ccaagtgtga ctgatgatgc agacaagtta ttcattgcaa 300
   ctaccttcct agctcactca ttggacacag ataagcagca ctctcagaga ggggggttcc 360
   tcgtctctct gcttgccatg gcttacagta gtccagaatt gtacttgaca acaaacggag 420
   taaacgccga tgtcaaatat gtgatctaca acatagagaa agaccctaag aggacgaaga 480
   cagacggatt cattgtgaag acgagagata tggaatatga gaggaccaca gaatggctgt 540
   ttggacctat ggtcaacaag agcccactct tccagggtca acgggatgct gcagaccctg 600
   acacactcct tcaaatctat gggtatcctg catgcctagg agcaataatt gtccaagtct 660
   ggattgtgct ggtgaaggcc atcacaagca gcgccggctt aaggaaaggg ttcttcaaca 720
   ggttagaggc gttcagacaa gacggcaccg tgaaaggtgc cttagttttc actggggaga 780
   cagttgaggg gataggctcg gttatgagat ctcagcaaag ccttgtatct ctcatggttg 840
   agacccttgt gactatgaat actgcaagat ctgatctcac cacattagag aagaacatcc 900
   agatcgttgg gaactacatc cgagatgcag ggctggcttc cttcatgaac actattaaat 960
   atggggtgga aacaaagatg gcagctctaa cgttgtcaaa cctgaggccc gatattaata 1020
   agcttagaag cctcatagac acctacctgt caaaaggccc cagagctccc tttatctgta 1080
   tcctcaagga ccctgttcat ggtgaatttg ctccaggcaa ttatcctgca ctatggagtt 1140
   acgccatggg agtcgccgtc gtacagaaca aggcaatgca gcagtacgtc acagggagga 1200
   cataccttga tatggaaatg ttcttactag gacaagccgt ggcaaaggat gctgaatcga 1260
   agatcagcag tgccttggaa gatgagttag gagtgacgga tacagccaag gggaggctca 1320
   gacatcatct ggcaaacttg tccggtgggg atggtgctta ccacaaacca acaggcggtg 1380
   gtgcaattga ggtagctcta gacaatgccg acatcgacct agaaacaaaa gcccatgcgg 1440
   accaggacgc taggggttgg ggtggagata gtggtgaaag atgggcacgt caggtgagtg 1500
   gtggccactt tgtcacacta catggggctg aacggttaga ggaggaaacc aatgatgagg 1560
   atgtatcaga catagagaga agaatagcca tgagactcgc agagagacgg caagaggatt 1620
   ctgcaaccca tggagatgaa ggccgcaata acggtgtcga tcatgacgaa gatgacgatg 1680
   ccgcagcagt agctgggata ggaggaatct aggatcatac gaggcttcaa ggtacttgat 1740
   ccgtagtaag aaaaacttag ggtgaaagtt catccaccga tcggctcagg caaggccaca 1800
   cccaacccca ccgaccacac ccagcagtcg agacagccac ggcttcggct acacttaccg 1860
   catggatcaa gatgccttca ttctggggga ggattctggg gtggagaggg gggcgccggg 1920
   gggacgggag tcgctctcgg acgtggtcgg gttcctcgac gctgtcctgt cgggtgggcc 1980
   ggctgacatc gggggggacg ggggctggct ccacagcacc atcggcgctc cccggggacc 2040
   gggctctgct catagagcca aaagtgaggg cgaaggagaa gtctcaacac cgtcgaccca 2100
   agataatcga tcaggtgagg agagtagagt ctctgggaga acaagcaagc cagaggcaga 2160
   agcacatgct ggaaaccttg ataaacaaaa tatacaccgg gcctttgggg gaagaactgg 2220
   tacaaactct gtatctcagg atctgggcga tggaggagac tccggaatcc ttgaaaatcc 2280
   tccaaatgag agaggatatc cgagatcagg tattgaagat gaaaacagag agatggctgc 2340
   gcaccctgat aagaggggag aagaccaagc tgaaggactt ccagaagagg tacgaggaag 2400
   tacatcccta cctgatgaag gagaaggtgg agcaagtaat aatggaagaa gcatggagcc 2460
   tggcagctca catagtgcaa gagtaactgg ggtcctggtg attcctagcc ccgaacttga 2520
   agaggctgtg ctacggagga acaaaagaag acctaccaac agtgggtcca aacctcttac 2580
   tccagcaacc gtgcctggca cccggtcccc accgctgaat cgttacaaca gcacagggtc 2640
   accaccagga aaacccccat ctacacagga tgagcacatc aactctgggg acacccccgc 2700
   cgtcagggtc aaagaccgga aaccaccaat agggacccgc tctgtctcag attgtccagc 2760
   caacggccgc ccaatccacc cgggtctaga gaccgactca acaaaaaagg gcataggaga 2820
   gaacacatca tctatgaaag agatggctac attgttgacg agtcttggtg taatccagtc 2880
   tgctcaagaa ttcgaatcat cccgagacgc gagttatgtg tttgcaagac gtgccctaaa 2940
   gtctgcaaac tatgcagaga tgacattcaa tgtatgcggc ctgatccttt ctgccgagaa 3000
   atcttccgct cgtaaggtag atgagaacaa acaactgctc aaacagatcc aagagagcgt 3060
   ggaatcattc cgggatattt acaagagatt ctctgagtat cagaaagaac agaactcatt 3120
   gctgatgtcc aacctatcta cacttcatat catcacagat agaggtggca agactgacaa 3180
   cacagactcc cttacaaggt ccccctccgt ttttgcaaaa tcaaaagaga acaagactaa 3240
   ggctaccagg tttgacccat ctatggagac cctagaagat atgaagtaca aaccggacct 3300
   aatccgagag gatgaattta gagatgagat ccgcaacccg gtgtaccaag agagggacac 3360
   agaacccagg gcctcaaacg catcacgtct cctcccctcc aaagagaagc ccacaatgca 3420
   ctctctcagg ctcgtcatag agagcagtcc cctaagcaga gctgagaaag tagcatatgt 3480
   gaaatcatta tccaagtgca agacagacca agaggttaag gcagtcatgg aactcgtaga 3540
   agaggacata gagtcactga ccaactagat cccgggtgag gcatcctacc atcctcagtc 3600
   atagagagat ccaatctacc atcagcatca gccagtaaag attaagaaaa acttagggtg 3660
   aaagaaattt cacctaacac ggcgcaatgg cagatatcta tagattccct aagttctcat 3720
   atgaggataa cggtactgtg gagcccctgc ctctgagaac tggtccggat aagaaagcca 3780
   tcccccacat caggattgtc aaggtaggag accctcctaa acatggagtg agatacctag 3840
   atttattgct cttgggtttc tttgagacac cgaaacaaac aaccaatcta gggagcgtat 3900
   ctgacttgac agagccgacc agctactcaa tatgcggctc cgggtcgtta cccataggtg 3960
   tggccaaata ctacgggact gatcaggaac tcttaaaggc ctgcaccgat ctcagaatta 4020
   cggtgaggag gactgttcga gcaggagaga tgatcgtata catggtggat tcgattggtg 4080
   ctccactcct accatggtca ggcaggctga gacagggaat gatatttaat gcaaacaagg 4140
   tcgcactagc tccccaatgc ctccctgtgg acaaggacat aagactcaga gtggtgtttg 4200
   tcaatgggac atctctaggg gcaatcacca tagccaagat cccaaagacc cttgcagacc 4260
   ttgcattgcc caactctata tctgttaatt tactggtgac actcaagacc gggatctcca 4320
   cagaacaaaa gggggtactc ccagtacttg atgatcaagg ggagaaaaag ctcaatttta 4380
   tggtgcacct cgggttgatc aggagaaagg tcgggaagat atactctgtt gagtactgca 4440
   agagcaagat tgagagaatg cggctgattt tctcacttgg gttaatcggc ggtataagct 4500
   tccatgttca ggttaatggg acactatcta agacattcat gagtcagctc gcatggaaga 4560
   gggcagtctg cttcccatta atggatgtga atccccatat gaacatggtg atttgggcgg 4620
   catctgtaga aatcacaggc gtcgatgcgg tgttccaacc ggccatccct cgtgatttcc 4680
   gctactaccc taatgttgtg gctaagaaca tcggaaggat cagaaagctg taaatgtgca 4740
   cccatcagag acctgcgaca atgccccaag cagacaccac ctggcagtcg gagccaccgg 4800
   gtcactcctt gtcttaaata agaaaaactt agggataaag tcccttgtga gtgcttggtt 4860
   gcaaaactct ccacctggta caagcacaga tcatggatgg tgataggggc aaacgtgact 4920
   cgtactggtc tacttctcct agtggtagca ccacaaaacc agcatcaggt tgggagaggt 4980
   caagtaaagc cgacacatgg ttgctgattc tctcattcac ccagtgggct ttgtcaattg 5040
   ccacagtgat catctgtatc ataatttctg ctagacaagg gtatagtatg aaagagtact 5100
   caatgactgt agaggcattg aacatgagca gcagggaggt gaaagagtca cttaccagtc 5160
   taataaggca agaggttata gcaagggctg tcaacattca gagctctgtg caaaccggaa 5220
   tcccagtctt gttgaacaaa aacagcaggg atgtcatcca gatgattgat aagtcgtgca 5280
   gcagacaaga gctcactcag cactgtgaga gtacgatcgc agtccaccat gccgatggaa 5340
   ttgccccact tgagccacat agtttctgga gatgccctgt cggagaaccg tatcttagct 5400
   cagatcctga aatctcattg ctgcctggtc cgagcttgtt atctggttct acaacgatct 5460
   ctggatgtgt taggctccct tcactctcaa ttggcgaggc aatctatgcc tattcatcaa 5520
   atctcattac acaaggttgt gctgacatag ggaaatcata tcaggtcctg cagctagggt 5580
   acatatcact caattcagat atgttccctg atcttaaccc cgtagtgtcc cacacttatg 5640
   acatcaacga caatcggaaa tcatgctctg tggtggcaac cgggactagg ggttatcagc 5700
   tttgctccat gccgactgta gacgaaagaa ccgactactc tagtgatggt attgaggatc 5760
   tggtccttga tgtcctggat ctcaaaggga gaactaagtc tcaccggtat cgcaacagcg 5820
   aggtagatct tgatcacccg ttctctgcac tataccccag tgtaggcaac ggcattgcaa 5880
   cagaaggctc attgatattt cttgggtatg gtggactaac cacccctctg cagggtgata 5940
   caaaatgtag gacccaagga tgccaacagg tgtcgcaaga cacatgcaat gaggctctga 6000
   aaattacatg gctaggaggg aaacaggtgg tcagcgtgat catccaggtc aatgactatc 6060
   tctcagagag gccaaagata agagtcacaa ccattccaat cactcaaaac tatctcgggg 6120
   cggaaggtag attattaaaa ttgggtgatc gggtgtacat ctatacaaga tcatcaggct 6180
   ggcactctca actgcagata ggagtacttg atgtcagcca ccctttgact atcaactgga 6240
   cacctcatga agccttgtct agaccaggaa ataaagagtg caattggtac aataagtgtc 6300
   cgaaggaatg catatcaggc gtatacactg atgcttatcc attgtcccct gatgcagcta 6360
   acgtcgctac cgtcacgcta tatgccaata catcgcgtgt caacccaaca atcatgtatt 6420
   ctaacactac taacattata aatatgttaa ggataaagga tgttcaatta gaggctgcat 6480
   ataccacgac atcgtgtatc acgcattttg gtaaaggcta ctgctttcac atcatcgaga 6540
   tcaatcagaa gagcctgaat accttacagc cgatgctctt taagactagc atccctaaat 6600
   tatgcaaggc cgagtcttaa atttaactga ctagcaggct tgtcggcctt gctgacacta 6660
   gagtcatctc cgaacatcca caatatctct cagtctctta cgtctctcac agtattaaga 6720
   aaaacccagg gtgaatggga agcttgccat aggtcatgga tgggcaggag tcctcccaaa 6780
   acccttctga catactctat ccagaatgcc acctgaactc tcccatagtc agggggaaga 6840
   tagcacagtt gcacgtcttg ttagatgtga accagcccta cagactgaag gacgacagca 6900
   taataaatat tacaaagcac aaaattagga acggaggatt gtccccccgt caaattaaga 6960
   tcaggtctct gggtaaggct cttcaacgca caataaagga tttagaccga tacacgtttg 7020
   aaccgtaccc aacctactct caggaattac ttaggcttga tataccagag atatgtgaca 7080
   aaatccgatc cgtcttcgcg gtctcggatc ggctgaccag ggagttatct agtgggttcc 7140
   aggatctttg gttgaatatc ttcaagcaac taggcaatat agaaggaaga gaggggtacg 7200
   atccgttgca ggatatcggc accatcccgg agataactga taagtacagc aggaatagat 7260
   ggtataggcc attcctaact tggttcagca tcaaatatga catgcggtgg atgcagaaga 7320
   ccagaccggg gggacccctc gatacctcta attcacataa cctcctagaa tgcaaatcat 7380
   acactctagt aacatacgga gatcttgtca tgatactgaa caagttgaca ttgacagggt 7440
   atatcctaac ccctgagctg gtcttgatgt attgtgatgt tgtagaagga aggtggaata 7500
   tgtctgctgc agggcatcta gataagaagt ccattgggat aacaagcaaa ggtgaggaat 7560
   tatgggaact agtggattcc ctcttctcaa gtcttggaga ggaaatatac aatgtcatcg 7620
   cactattgga gcccctatca cttgctctca tacaactaaa tgatcctgtt atacctctac 7680
   gtggggcatt tatgaggcat gtgttgacag agctacagac tgttttaaca agtagagacg 7740
   tgtacacaga tgctgaagca gacactattg tggagtcgtt actcgccatt ttccatggaa 7800
   cctctattga tgagaaagca gagatctttt ccttctttag gacatttggc caccccagct 7860
   tagaggctgt cactgccgcc gacaaggtaa gggcccatat gtatgcacaa aaggcaataa 7920
   agcttaagac cctatacgag tgtcatgcag ttttttgcac tatcatcata aatgggtata 7980
   gagagaggca tggcggacag tggcccccct gtgacttccc tgatcacgtg tgtctagaac 8040
   taaggaacgc tcaagggtcc aatacggcaa tctcttatga atgtgctgta gacaactata 8100
   caagtttcat aggcttcaag tttcggaagt ttatagaacc acaactagat gaagatctca 8160
   caatatatat gaaagacaaa gcactatccc ccaggaagga ggcatgggac tctgtatacc 8220
   cggatagtaa tctgtactat aaagccccag agtctgaaga gacccggcgg cttattgaag 8280
   tgttcataaa tgatgagaat ttcaacccag aagaaattat caattatgtg gagtcaggag 8340
   attggttgaa agacgaggag ttcaacatct cgtacagtct caaagagaaa gagatcaagc 8400
   aagagggtcg tctattcgca aaaatgactt ataagatgcg agccgtacag gtgctggcag 8460
   agacactact ggctaaagga ataggagagc tattcagcga aaatgggatg gttaaaggag 8520
   agatagacct acttaaaaga ttgactactc tttctgtctc aggcgtcccc aggactgatt 8580
   cagtgtacaa taactctaaa tcatcagaga agagaaacga aggcatggaa aataagaact 8640
   ctgggggcta ctgggacgaa aagaagaggt ccagacatga attcaaggca acagattcat 8700
   caacagacgg ctatgaaacg ttaagttgct tcctcacaac agacctcaag aaatactgct 8760
   taaactggag atttgagagt actgcattgt ttggtcagag atgcaacgag atatttggct 8820
   tcaagacctt ctttaactgg atgcatccag tccttgaaag gtgtacaata tatgttggag 8880
   atccttactg tccagtcgcc gaccggatgc atcgacaact ccaggatcat gcagactctg 8940
   gcattttcat acataatcct agggggggca tagaaggtta ctgccagaag ctgtggacct 9000
   taatctcaat cagtgcaatc cacctagcag ctgtgagagt gggtgtcagg gtctctgcaa 9060
   tggttcaggg tgacaatcaa gctatagccg tgacatcaag agtacctgta gctcagactt 9120
   acaagcagaa gaaaaatcat gtctatgagg agatcaccaa atatttcggt gctctaagac 9180
   acgtcatgtt tgatgtaggg cacgagctaa aattgaacga gaccatcatt agtagcaaga 9240
   tgtttgtcta tagtaaaagg atatactatg atgggaagat tttaccacag tgcctgaaag 9300
   ccttgaccaa gtgtgtattc tggtccgaga cactggtaga tgaaaacaga tctgcttgtt 9360
   cgaacatctc aacatccata gcaaaagcta tcgaaaatgg gtattctcct atactaggct 9420
   actgcattgc gttgtataag acctgtcagc aggtgtgcat atcactaggg atgactataa 9480
   atccaactat cagcccgacc gtaagagatc aatactttaa gggtaagaat tggctgagat 9540
   gtgcagtgtt gattccagca aatgttggag gattcaacta catgtctaca tctagatgct 9600
   ttgttagaaa tattggagac cccgcagtag cagccctagc tgatctcaaa agattcatca 9660
   gagcggatct gttagacaag caggtattat acagggtcat gaatcaagaa cccggtgact 9720
   ctagttttct agattgggct tcagaccctt attcgtgtaa cctcccgcat tctcagagta 9780
   taactacgat tataaagaat atcactgcta gatctgtgct gcaggaatcc ccgaatcctc 9840
   tactgtctgg tctcttcacc gagactagtg gagaagagga tctcaacctg gcctcgttcc 9900
   ttatggaccg gaaagtcatc ctgccgagag tggctcatga gatcctgggt aattccttaa 9960
   ctggagttag ggaggcgatt gcagggatgc ttgatacgac caagtctcta gtgagagcca 10020
   gcgttaggaa aggaggatta tcatatggga tattgaggag gcttgtcaat tatgatctat 10080
   tgcagtacga gacactgact agaactctca ggaaaccggt gaaagacaac atcgaatatg 10140
   agtatatgtg ttcagttgag ctagctgtcg gtctaaggca gaaaatgtgg atccacctga 10200
   cttacgggag acccatacat gggctagaaa caccagaccc tttagagctc ttgaggggaa 10260
   tatttatcga aggttcagag gtgtgcaagc tttgcaggtc tgaaggagca gaccccatct 10320
   atacatggtt ctatcttcct gacaatatag acctggacac gcttacaaac ggatgtccgg 10380
   ctataagaat cccctatttt ggatcagcca ctgatgaaag gtcggaagcc caactcgggt 10440
   atgtaagaaa tctaagcaaa cccgcaaagg cggccatccg gatagctatg gtgtatacgt 10500
   gggcctacgg gactgatgag atatcgtgga tggaagccgc tcttatagcc caaacaagag 10560
   ctaatctgag cttagagaat ctaaagctgc tgactcctgt ttcaacctcc actaatctat 10620
   ctcataggtt gaaagatacg gcaacccaga tgaagttctc tagtgcaaca ctagtccgtg 10680
   caagtcggtt cataacaata tcaaatgata acatggcact caaagaagca ggggagtcga 10740
   aggatactaa tctcgtgtat cagcagatta tgctaactgg gctaagcttg ttcgagttca 10800
   atatgagata taagaaaggt tccttaggga agccactgat attgcactta catcttaata 10860
   acgggtgctg tataatggag tccccacagg aggcgaatat ccccccaagg tccacattag 10920
   atttagagat tacacaagag aacaataaat tgatctatga tcctgatcca ctcaaggatg 10980
   tggaccttga gctatttagc aaggtcagag atgttgtaca cacagttgac atgacttatt 11040
   ggtcagatga tgaagttatc agagcaacca gtatctgtac tgcaatgacg atagctgata 11100
   caatgtctca attagataga gacaacttaa aagagatgat cgcactagta aatgacgatg 11160
   atgtcaacag cttgattact gagtttatgg tgattgatgt tcctttattt tgctcaacgt 11220
   tcgggggtat tctagtcaat cagtttgcat actcactcta cggcttaaac atcagaggaa 11280
   gggaagaaat atggggacat gtagtccgga ttcttaaaga tacctcccac gcagttttaa 11340
   aagtcttatc taatgctcta tctcatccca aaatcttcaa acgattctgg aatgcaggtg 11400
   tcgtggaacc tgtgtatggg cctaacctct caaatcagga taagatactc ttggccctct 11460
   ctgtctgtga atattctgtg gatctattca tgcacgattg gcaagggggt gtaccgcttg 11520
   agatctttat ctgtgacaat gacccagatg tggccgacat gaggaggtcc tctttcttgg 11580
   caagacatct tgcataccta tgcagcttgg cagagatatc tagggatggg ccaagattag 11640
   aatcaatgaa ctctctagag aggctcgagt cactaaagag ttacctggaa ctcacatttc 11700
   ttgatgaccc ggtactgagg tacagtcagt tgactggcct agtcatcaaa gtattcccat 11760
   ctactttgac ctatatccgg aagtcatcta taaaagtgtt aaggacaaga ggtataggag 11820
   tccctgaagt cttagaagat tgggatcccg aggcagataa tgcactgtta gatggtatcg 11880
   cggcagaaat acaacagaat attcctttgg gacatcagac tagagcccct ttttgggggt 11940
   tgagagtatc caagtcacag gtactgcgtc tccgggggta caaggagatc acaagaggtg 12000
   agataggcag atcaggtgtt ggtctgacgt taccattcga tggaagatat ctatctcacc 12060
   agctgaggct ctttggcatc aacagtacta gctgcttgaa agcacttgaa cttacctacc 12120
   tattgagccc cttagttgac aaggataaag ataggctata tttaggggaa ggagctgggg 12180
   ccatgctttc ctgttatgac gctactcttg gcccatgcat caactattat aactcagggg 12240
   tatactcttg tgatgtcaat gggcagagag agttaaatat atatcctgct gaggtggcac 12300
   tagtgggaaa gaaattaaac aatgttacta gtctgggtca aagagttaaa gtgttattca 12360
   acgggaatcc tggctcgaca tggattggga atgatgagtg tgaggctttg atttggaatg 12420
   aattacagaa tagctcgata ggcctagtcc actgtgacat ggagggagga gatcataagg 12480
   atgatcaagt tgtactgcat gagcattaca gtgtaatccg gatcgcgtat ctggtggggg 12540
   atcgagacgt tgtgcttata agcaagattg ctcccaggct gggcacggat tggaccaggc 12600
   agctcagcct atatctgaga tactgggacg aggttaacct aatagtgctt aaaacatcta 12660
   accctgcttc cacagagatg tatctcctat cgaggcaccc caaatctgac attatagagg 12720
   acagcaagac agtgttagct agtctcctcc ctttgtcaaa agaagatagc atcaagatag 12780
   aaaagtggat cttaatagag aaggcaaagg ctcacgaatg ggttactcgg gaattgagag 12840
   aaggaagctc ttcatcaggg atgcttagac cttaccatca agcactgcag acgtttggct 12900
   ttgaaccaaa cttgtataaa ttgagcagag atttcttgtc caccatgaac atagctgata 12960
   cacacaactg catgatagct ttcaacaggg ttttgaagga tacaatcttc gaatgggcta 13020
   gaataactga gtcagataaa aggcttaaac taactggtaa gtatgacctg tatcctgtga 13080
   gagattcagg caagttgaag acaatttcta gaagacttgt gctatcttgg atatctttat 13140
   ctatgtccac aagattggta actgggtcat tccctgacca gaagtttgaa gcaagacttc 13200
   aattgggaat agtttcatta tcatcccgtg aaatcaggaa cctgagggtt atcacaaaaa 13260
   ctttattaga caggtttgag gatattatac atagtataac gtatagattc ctcaccaaag 13320
   aaataaagat tttgatgaag attttagggg cagtcaagat gttcggggcc aggcaaaatg 13380
   aatacacgac cgtgattgat gatggatcac taggtgatat cgagccatat gacagctcgt 13440
   aataattagt ccctatcgtg cagaacgatc gaagctccgc ggtacctgga agtcttggac 13500
   ttgtccatat gacaatagta agaaaaacta aaatcctgta taacttcatt acatatccca 13560
   tacatgtttt ttctcttgtt tggtgggtcg gcatggcatc tccacctcct cgcggtccga 13620
   cctgggcatc cgaaggagga cgcacgtcca ctcggatggc taagggagag ctacgcgtgg 13680
   atccccggga attccgtaat catggtcata gctgtttcct gtgtgaaatt gttatccgct 13740
   cacaattcca cacaacatac gagccggaag cataaagtgt aaagcctggg gtgcctaatg 13800
   agtgagctaa ctcacattaa ttgcgttgcg ctcactgccc gctttccagt cgggaaacct 13860
   gtcgtgccag ctgcattaat gaatcggcca acgcgcgggg agaggcggtt tgcgtattgg 13920
   gcgctcttcc gcttcctcgc tcactgactc gctgcgctcg gtcgttcggc tgcggcgagc 13980
   ggtatcagct cactcaaagg cggtaatacg gttatccaca gaatcagggg ataacgcagg 14040
   aaagaacatg tgagcaaaag gccagcaaaa ggccaggaac cgtaaaaagg ccgcgttgct 14100
   ggcgtttttc cataggctcc gcccccctga cgagcatcac aaaaatcgac gctcaagtca 14160
   gaggtggcga aacccgacag gactataaag ataccaggcg tttccccctg gaagctccct 14220
   cgtgcgctct cctgttccga ccctgccgct taccggatac ctgtccgcct ttctcccttc 14280
   gggaagcgtg gcgctttctc atagctcacg ctgtaggtat ctcagttcgg tgtaggtcgt 14340
   tcgctccaag ctgggctgtg tgcacgaacc ccccgttcag cccgaccgct gcgccttatc 14400
   cggtaactat cgtcttgagt ccaacccggt aagacacgac ttatcgccac tggcagcagc 14460
   cactggtaac aggattagca gagcgaggta tgtaggcggt gctacagagt tcttgaagtg 14520
   gtggcctaac tacggctaca ctagaagaac agtatttggt atctgcgctc tgctgaagcc 14580
   agttaccttc ggaaaaagag ttggtagctc ttgatccggc aaacaaacca ccgctggtag 14640
   cggtggtttt tttgtttgca agcagcagat tacgcgcaga aaaaaaggat ctcaagaaga 14700
   tcctttgatc ttttctacgg ggtctgacgc tcagtggaac gaaaactcac gttaagggat 14760
   tttggtcatg agattatcaa aaaggatctt cacctagatc cttttaaatt aaaaatgaag 14820
   ttttaaatca atctaaagta tatatgagta aacttggtct gacagttacc aatgcttaat 14880
   cagtgaggca cctatctcag cgatctgtct atttcgttca tccatagttg cctgactccc 14940
   cgtcgtgtag ataactacga tacgggaggg cttaccatct ggccccagtg ctgcaatgat 15000
   accgcgagac ccacgctcac cggctccaga tttatcagca ataaaccagc cagccggaag 15060
   ggccgagcgc agaagtggtc ctgcaacttt atccgcctcc atccagtcta ttaattgttg 15120
   ccgggaagct agagtaagta gttcgccagt taatagtttg cgcaacgttg ttgccattgc 15180
   tacaggcatc gtggtgtcac gctcgtcgtt tggtatggct tcattcagct ccggttccca 15240
   acgatcaagg cgagttacat gatcccccat gttgtgcaaa aaagcggtta gctccttcgg 15300
   tcctccgatc gttgtcagaa gtaagttggc cgcagtgtta tcactcatgg ttatggcagc 15360
   actgcataat tctcttactg tcatgccatc cgtaagatgc ttttctgtga ctggtgagta 15420
   ctcaaccaag tcattctgag aatagtgtat gcggcgaccg agttgctctt gcccggcgtc 15480
   aatacgggat aataccgcgc cacatagcag aactttaaaa gtgctcatca ttggaaaacg 15540
   ttcttcgggg cgaaaactct caaggatctt accgctgttg agatccagtt cgatgtaacc 15600
   cactcgtgca cccaactgat cttcagcatc ttttactttc accagcgttt ctgggtgagc 15660
   aaaaacagga aggcaaaatg ccgcaaaaaa gggaataagg gcgacacgga aatgttgaat 15720
   actcatactc ttcctttttc aatattattg aagcatttat cagggttatt gtctcatgag 15780
   cggatacata tttgaatgta tttagaaaaa taaacaaata ggggttccgc gcacatttcc 15840
   ccgaaaagtg ccacctgacg tctaagaaac cattattatc atgacattaa cctataaaaa 15900
   taggcgtatc acgaggccct ttcgtctcgc gcgtttcggt gatgacggtg aaaacctctg 15960
   acacatgcag ctcccggaga cggtcacagc ttgtctgtaa gcggatgccg ggagcagaca 16020
   agcccgtcag ggcgcgtcag cgggtgttgg cgggtgtcgg ggctggctta actatgcggc 16080
   atcagagcag attgtactga gagtgcacca tatgcggtgt gaaataccgc acagatgcgt 16140
   aaggagaaaa taccgcatca ggcgccattc gccattcagg ctgcgcaact gttgggaagg 16200
   gcgatcggtg cgggcctctt cgctattacg ccagctggcg aaagggggat gtgctgcaag 16260
   gcgattaagt tgggtaacgc cagggttttc ccagtcacga cgttgtaaaa cgacggccag 16320
   tgccaagctc gcgtcgtacg ggccctaata cgactcacta ta 16362

<210> 12
<211> 50
<212> DNA
<213> Artificial

<220>
<223> an artificially synthesized primer sequence

<400> 12
agttcacgcg gccgcagatc ttcacgatgg tgagcaaggg cgaggagctg 50

<210> 13

<211>  65
<212>  DNA
<213>  Artificial

<220>
<223>  an artificially synthesized primer sequence

<400>  13
caggtaccgc ggagcttcga tcgttctgca cgatagggac taattacttg tacagctcgt      60

ccatg                                                                  65



<210>  14
<211>  3010
<212>  DNA
<213>  Artificial

<220>
<223>  an artificially synthesized nucleotide sequence

<400>  14
tcgcgcgttt cggtgatgac ggtgaaaacc tctgacacat gcagctcccg gagacggtca      60

cagcttgtct gtaagcggat gccgggagca gacaagcccg tcagggcgcg tcagcgggtg     120

ttggcgggtg tcggggctgg cttaactatg cggcatcaga gcagattgta ctgagagtgc     180

accatatgcg gtgtgaaata ccgcacagat gcgtaaggag aaaataccgc atcaggcgcc     240

attcgccatt caggctgcgc aactgttggg aagggcgatc ggtgcgggcc tcttcgctat     300

tacgccagct ggcgaaaggg ggatgtgctg caaggcgatt aagttgggta acgccagggt     360

tttcccagtc acgacgttgt aaaacgacgg ccagtgccaa gctcgcgtcg tacgggccct     420

aatacgactc actataacca aacaagagaa aaaacatgta tgggatatgt aatgaagtta 480

tacaggattt tagggtcaaa gtatccaccc tgaggagcag gttccagacc ctttgctttg 540

ctgccaaagt tcacgcggcc gcccgcggta cctggaagtc ttggacttgt ccatatgaca 600

atagtaagaa aaacttacaa gaagacaaga aaatttaaaa ggatacatat ctcttaaact 660

cttgtctggt gggtcggcat ggcatctcca cctcctcgcg gtccgacctg ggcatccgaa 720

ggaggacgca cgtccactcg gatggctaag ggagagctac gcgtggatcc ccgggaattc 780

gtaatcatgt catagctgtt tcctgtgtga aattgttatc cgctcacaat tccacacaac 840

atacgagccg gaagcataaa gtgtaaagcc tggggtgcct aatgagtgag ctaactcaca 900

ttaattgcgt tgcgctcact gcccgctttc cagtcgggaa acctgtcgtg ccagctgcat 960

taatgaatcg gccaacgcgc ggggagaggc ggtttgcgta ttgggcgctc ttccgcttcc 1020

tcgctcactg actcgctgcg ctcggtcgtt cggctgcggc gagcggtatc agctcactca 1080

aaggcggtaa tacggttatc cacagaatca ggggataacg caggaaagaa catgtgagca 1140

aaaggccagc aaaaggccag gaaccgtaaa aaggccgcgt tgctggcgtt tttccatagg 1200

ctccgccccc ctgacgagca tcacaaaaat cgacgctcaa gtcagaggtg gcgaaacccg 1260

acaggactat aaagatacca ggcgtttccc cctggaagct ccctcgtgcg ctctcctgtt 1320

ccgaccctgc cgcttaccgg atacctgtcc gcctttctcc cttcgggaag cgtggcgctt 1380

tctcaaagct cacgctgtag gtatctcagt tcggtgtagg tcgttcgctc caagctgggc 1440

tgtgtgcacg aaccccccgt tcagcccgac cgctgcgcct tatccggtaa ctatcgtctt 1500

gagtccaacc cggtaagaca cgacttatcg ccactggcag cagccactgg taacaggatt 1560

agcagagcga ggtatgtagg cggtgctaca gagttcttga agtggtggcc taactacggc 1620

tacactagaa gaacagtatt tggtatctgc gctctgctga agccagttac cttcggaaaa 1680

agagttggta gctcttgatc cggcaaacaa accaccgctg gtagcggtgg tttttttgtt 1740

tgcaagcagc agattacgcg cagaaaaaaa ggatctcaag aagatccttt gatcttttct 1800

acggggtctg acgctcagtg gaacgaaaac tcacgttaag ggattttggt catgagatta 1860

tcaaaaagga tcttcaccta gatcctttta aattaaaaat gaagttttaa atcaatctaa 1920

agtatatatg agtaaacttg gtctgacagt taccaatgct taatcagtga ggcacctatc 1980

tcagcgatct gtctatttcg ttcatccata gttgcctgac tccccgtcgt gtagataact 2040

acgatacggg agggcttacc atctggcccc agtgctgcaa tgataccgcg agacccacgc 2100

tcaccggctc cagatttatc agcaataaac cagccagccg gaagggccga gcgcagaagt 2160

ggtcctgcaa ctttatccgc ctccatccag tctattaatt gttgccggga agctagagta 2220

agtagttcgc cagttaatag tttgcgcaac gttgttgcca ttgctacagg catcgtggtg 2280

tcacgctcgt cgtttggtat ggcttcattc agctccggtt cccaacgatc aaggcgagtt 2340

acatgatccc ccatgttgtg caaaaaagcg gttagctcct tcggtcctcc gatcgttgtc 2400

agaagtaagt tggccgcagt gttatcactc atggttatgg cagcactgca taattctctt 2460

actgtcatgc catccgtaag atgcttttct gtgactggtg agtactcaac caagtcattc 2520

tgagaatagt gtatgcggcg accgagttgc tcttgcccgg cgtcaatacg ggataatacc 2580

gcgccacata gcagaacttt aaaagtgctc atcattggaa aacgttcttc ggggcgaaaa 2640

ctctcaagga tcttaccgct gttgagatcc agttcgatgt aacccactcg tgcacccaac 2700

tgatcttcag catcttttac tttcaccagc gtttctgggt gagcaaaaac aggaaggcaa 2760

aatgccgcaa aaaagggaat aagggcgaca cggaaatgtt gaatactcat actcttcctt 2820

tttcaatatt attgaagcat ttatcagggt tattgtctca tgagcggata catatttgaa 2880

tgtatttaga aaaataaaca aataggggtt ccgcgcacat ttccccgaaa agtgccacct 2940

gacgtctaag aaaccattat tatcatgaca ttaacctata aaaataggcg tatcacgagg 3000

ccctttcgtc 3010

<210> 15
<211> 996
<212> DNA
<213> Artificial

<220>
<223> an artificially synthesized nucleotide sequence

<400> 15
accaaacaag agaaaaaaca tgtatgggat atgtaatgaa gttatacagg attttagggt 60

caaagtatcc accctgagga gcaggttcca gaccctttgc tttgctgcca aagttcacgc 120

ggccgcagat cttcacgatg gtgagcaagg gcgaggagct gttcaccggg gtggtgccca 180

tcctggtcga gctggacggc gacgtaaacg gccacaagtt cagcgtgtcc ggcgagggcg 240

agggcgatgc cacctacggc aagctgaccc tgaagttcat ctgcaccacc ggcaagctgc 300

ccgtgccctg gcccaccctc gtgaccaccc tgacctacgg cgtgcagtgc ttcagccgct 360

accccgacca catgaagcag cacgacttct tcaagtccgc catgcccgaa ggctacgtcc 420

aggagcgcac catcttcttc aaggacgacg gcaactacaa gacccgcgcc gaggtgaagt 480

tcgagggcga caccctggtg aaccgcatcg agctgaaggg catcgacttc aaggaggacg 540

gcaacatcct ggggcacaag ctggagtaca actacaacag ccacaacgtc tatatcatgg 600

ccgacaagca gaagaacggc atcaaggtga acttcaagat ccgccacaac atcgaggacg 660

gcagcgtgca gctcgccgac cactaccagc agaacacccc catcggcgac ggccccgtgc 720

tgctgcccga caaccactac ctgagcaccc agtccgccct gagcaaagac cccaacgaga 780

agcgcgatca catggtcctg ctggagttcg tgaccgccgc cgggatcact ctcggcatgg 840

acgagctgta caagtaatta gtccctatcg tgcagaacga tcgaagctcc gcggtacctg 900

gaagtcttgg acttgtccat atgacaatag taagaaaaac ttacaagaag acaagaaaat 960

ttaaaaggat acatatctct taaactcttg tctggt 996

<210> 16
<211> 26
<212> DNA
<213> Artificial

<220>
<223> an artificially synthesized primer sequence

<400> 16
agttcacgcg gccgcagatc ttcacg 26

<210> 17
<211> 43
<212> DNA
<213> Artificial

<220>
<223> an artificially synthesized primer sequence

<400> 17
caggtaccgc ggagcttcga tcgttctgca cgatagggac taa 43

<210> 18
<211> 22
<212> DNA
<213> Artificial

<220>
<223> an artificially synthesized primer sequence

<400> 18
ataccgcatc aggcgccatt cg 22

<210> 19
<211> 57
<212> DNA
<213> Artificial

<220>
<223> an artificially synthesized primer sequence

<400> 19
gttttttagg gtctggaacc tgctcctcag ggtggatact ttgacactaa aatcctg 57

<210> 20
<211> 34
<212> DNA
<213> Artificial

<220>
<223> an artificially synthesized primer sequence

<400> 20
ggttccagac cctaaaaaac atgtatggga tatg 34

<210> 21
<211> 3856
<212> DNA
<213> Artificial

<220>
<223> an artificially synthesized nucleotide sequence

<400> 21
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgccaa gctcgcgtcg tacgggccct 420

aatacgactc actataacca aacaagagaa aaaacatgta tgggatatgt aatgaagtta 480

tacaggattt tagtgtcaaa gtatccaccc tgaggagcag gttccagacc ctaaaaaaca 540

tgtatgggat atgtaatgaa gttatacagg attttagggt caaagtatcc accctgagga 600

gcaggttcca gaccctttgc tttgctgcca aagttcacgc ggccgcagat cttcacgatg 660

gtgagcaagg gcgaggagct gttcaccggg gtggtgccca tcctggtcga gctggacggc 720

gacgtaaacg gccacaagtt cagcgtgtcc ggcgagggcg agggcgatgc cacctacggc 780

aagctgaccc tgaagttcat ctgcaccacc ggcaagctgc ccgtgccctg gcccaccctc 840

gtgaccaccc tgacctacgg cgtgcagtgc ttcagccgct accccgacca catgaagcag 900

cacgacttct tcaagtccgc catgcccgaa ggctacgtcc aggagcgcac catcttcttc 960

aaggacgacg gcaactacaa gacccgcgcc gaggtgaagt tcgagggcga caccctggtg 1020

aaccgcatcg agctgaaggg catcgacttc aaggaggacg gcaacatcct ggggcacaag 1080

ctggagtaca actacaacag ccacaacgtc tatatcatgg ccgacaagca gaagaacggc 1140

atcaaggtga acttcaagat ccgccacaac atcgaggacg gcagcgtgca gctcgccgac 1200

cactaccagc agaacacccc catcggcgac ggccccgtgc tgctgcccga caaccactac 1260

ctgagcaccc agtccgccct gagcaaagac cccaacgaga agcgcgatca catggtcctg 1320

ctggagttcg tgaccgccgc cgggatcact ctcggcatgg acgagctgta caagtaatta 1380

gtccctatcg tgcagaacga tcgaagctcc gcggtacctg gaagtcttgg acttgtccat 1440

atgacaatag taagaaaaac ttacaagaag acaagaaaat ttaaaaggat acatatctct 1500

taaactcttg tctggtgggt cggcatggca tctccacctc ctcgcggtcc gacctgggca 1560

tccgaaggag gacgcacgtc cactcggatg gctaagggag agctacgcgt ggatccccgg 1620

gaattcgtaa tcatgtcata gctgtttcct gtgtgaaatt gttatccgct cacaattcca 1680

cacaacatac gagccggaag cataaagtgt aaagcctggg gtgcctaatg agtgagctaa 1740

ctcacattaa ttgcgttgcg ctcactgccc gctttccagt cgggaaacct gtcgtgccag 1800

ctgcattaat gaatcggcca acgcgcgggg agaggcggtt tgcgtattgg gcgctcttcc 1860

gcttcctcgc tcactgactc gctgcgctcg gtcgttcggc tgcggcgagc ggtatcagct 1920

cactcaaagg cggtaatacg gttatccaca gaatcagggg ataacgcagg aaagaacatg 1980

tgagcaaaag gccagcaaaa ggccaggaac cgtaaaaagg ccgcgttgct ggcgtttttc 2040

cataggctcc gcccccctga cgagcatcac aaaaatcgac gctcaagtca gaggtggcga 2100

aacccgacag gactataaag ataccaggcg tttccccctg gaagctccct cgtgcgctct 2160

cctgttccga ccctgccgct taccggatac ctgtccgcct ttctcccttc gggaagcgtg 2220

gcgctttctc aaagctcacg ctgtaggtat ctcagttcgg tgtaggtcgt tcgctccaag 2280

ctgggctgtg tgcacgaacc ccccgttcag cccgaccgct gcgccttatc cggtaactat 2340

cgtcttgagt ccaacccggt aagacacgac ttatcgccac tggcagcagc cactggtaac 2400

aggattagca gagcgaggta tgtaggcggt gctacagagt tcttgaagtg gtggcctaac 2460

tacggctaca ctagaagaac agtatttggt atctgcgctc tgctgaagcc agttaccttc 2520

ggaaaaagag ttggtagctc ttgatccggc aaacaaacca ccgctggtag cggtggtttt 2580

tttgtttgca agcagcagat tacgcgcaga aaaaaaggat ctcaagaaga tcctttgatc 2640

ttttctacgg ggtctgacgc tcagtggaac gaaaactcac gttaagggat tttggtcatg 2700

agattatcaa aaaggatctt cacctagatc cttttaaatt aaaaatgaag ttttaaatca 2760

atctaaagta tatatgagta aacttggtct gacagttacc aatgcttaat cagtgaggca 2820

cctatctcag cgatctgtct atttcgttca tccatagttg cctgactccc cgtcgtgtag 2880

ataactacga tacgggaggg cttaccatct ggccccagtg ctgcaatgat accgcgagac 2940

ccacgctcac cggctccaga tttatcagca ataaaccagc cagccggaag ggccgagcgc 3000

agaagtggtc ctgcaacttt atccgcctcc atccagtcta ttaattgttg ccgggaagct 3060

agagtaagta gttcgccagt taatagtttg cgcaacgttg ttgccattgc tacaggcatc 3120

gtggtgtcac gctcgtcgtt tggtatggct tcattcagct ccggttccca acgatcaagg 3180

cgagttacat gatcccccat gttgtgcaaa aaagcggtta gctccttcgg tcctccgatc 3240

gttgtcagaa gtaagttggc cgcagtgtta tcactcatgg ttatggcagc actgcataat 3300

tctcttactg tcatgccatc cgtaagatgc ttttctgtga ctggtgagta ctcaaccaag 3360

tcattctgag aatagtgtat gcggcgaccg agttgctctt gcccggcgtc aatacgggat 3420

aataccgcgc cacatagcag aactttaaaa gtgctcatca ttggaaaacg ttcttcgggg 3480

cgaaaactct caaggatctt accgctgttg agatccagtt cgatgtaacc cactcgtgca 3540

cccaactgat cttcagcatc ttttactttc accagcgttt ctgggtgagc aaaaacagga 3600

aggcaaaatg ccgcaaaaaa gggaataagg gcgacacgga aatgttgaat actcatactc 3660

ttcctttttc aatattattg aagcatttat cagggttatt gtctcatgag cggatacata 3720

tttgaatgta tttagaaaaa taaacaaata ggggttccgc gcacatttcc ccgaaaagtg 3780

ccacctgacg tctaagaaac cattattatc atgacattaa cctataaaaa taggcgtatc 3840

acgaggccct ttcgtc 3856

<210> 22
<211> 10
<212> RNA
<213> Sendai virus

<400> 22
ucccwvuuwc 10

<210> 23
<211> 10
<212> RNA
<213> Sendai virus

<400> 23
ucccaguuuc 10

<210> 24
<211> 10
<212> RNA
<213> Sendai virus

<400> 24
ucccacuuac 10

<210> 25
<211> 10
<212> RNA
<213> Sendai virus

<400> 25
ucccacuuuc 10

<210> 26
<211> 10
<212> DNA
<213> Sendai virus

<400> 26
agggtcaaag 10

<210> 27
<211> 10
<212> DNA
<213> Sendai virus

<400> 27
agggtgaatg 10

<210> 28
<211> 10
<212> DNA
<213> Sendai virus

<400> 28
agggtgaaag 10

<210> 29
<211> 9
<212> RNA
<213> Sendai virus

<400> 29
auucuuuuu 9

<210> 30
<211> 9
<212> DNA
<213> Sendai virus

<400> 30
taagaaaaa 9

<210> 31
<211> 33
<212> DNA
<213> Artificial

<220>
<223> An artificially synthesized primer sequence

<400> 31
gtacgggccc gtacgtctga ggccgaggga aag 33

<210> 32
<211> 34
<212> DNA
<213> Artificial

<220>

<223>  An artificially synthesized primer sequence

<400>  32
ctcttgtttg gtacctatct ccaggtccaa tagg                                    34

<210>  33
<211>  34
<212>  DNA
<213>  Artificial

<220>
<223>  An artificially synthesized primer sequence

<400>  33
gacctggaga taggtaccaa acaagagaaa aaac                                    34

<210>  34
<211>  27
<212>  DNA
<213>  Artificial

<220>
<223>  An artificially synthesized primer sequence

<400>  34
gactagcggc cgcgtgaact ttggcag                                            27

<210>  35
<211>  34
<212>  DNA
<213>  Artificial

<220>

<223>  An artificially synthesized primer sequence

<400>  35
atatgcggcc gcaatggtga gcaagggcga ggag                                34

<210>  36
<211>  32
<212>  DNA
<213>  Artificial

<220>
<223>  An artificially synthesized primer sequence

<400>  36
tggcgtcttc cttgtacagc tcgtccatgc cg                                  32

<210>  37
<211>  35
<212>  DNA
<213>  Artificial

<220>
<223>  An artificially synthesized primer sequence

<400>  37
acgagctgta caaggaagac gccaaaaaca taaag                               35

<210>  38
<211>  32
<212>  DNA
<213>  Artificial

<220>

<223>  An artificially synthesized primer sequence

<400>  38
atatgcggcc gcttacacgg cgatctttcc gc                                    32

<210>  39
<211>  33
<212>  DNA
<213>  Artificial

<220>
<223>  An artificially synthesized primer sequence

<400>  39
tcacgcggcc gctagtccct atcgtgcaga acg                                   33

<210>  40
<211>  27
<212>  DNA
<213>  Artificial

<220>
<223>  An artificially synthesized primer sequence

<400>  40
gactagcggc cgcgtgaact ttggcag                                          27

<210>  41
<211>  2384
<212>  DNA
<213>  Artificial

<220>

<223>  An artificially synthesized nucleotide sequence

<400>  41
gcggccgcaa tggtgagcaa gggcgaggag ctgttcaccg gggtggtgcc catcctggtc      60

gagctggacg gcgacgtaaa cggccacaag ttcagcgtgt ccggcgaggg cgagggcgat     120

gccacctacg gcaagctgac cctgaagttc atctgcacca ccggcaagct gcccgtgccc     180

tggcccaccc tcgtgaccac cctgacctac ggcgtgcagt gcttcagccg ctaccccgac     240

cacatgaagc agcacgactt cttcaagtcc gccatgcccg aaggctacgt ccaggagcgc     300

accatcttct tcaaggacga cggcaactac aagacccgcg ccgaggtgaa gttcgagggc     360

gacaccctgg tgaaccgcat cgagctgaag ggcatcgact tcaaggagga cggcaacatc     420

ctggggcaca agctggagta caactacaac agccacaacg tctatatcat ggccgacaag     480

cagaagaacg gcatcaaggt gaacttcaag atccgccaca acatcgagga cggcagcgtg     540

cagctcgccg accactacca gcagaacacc cccatcggcg acggccccgt gctgctgccc     600

gacaaccact acctgagcac ccagtccgcc ctgagcaaag accccaacga gaagcgcgat     660

cacatggtcc tgctggagtt cgtgaccgcc gccgggatca ctctcggcat ggacgagctg     720

tacaaggaag acgccaaaaa cataaagaaa ggcccggcgc cattctatcc gctggaagat     780

ggaaccgctg gagagcaact gcataaggct atgaagagat acgccctggt tcctggaaca     840

attgctttta cagatgcaca tatcgaggtg gacatcactt acgctgagta cttcgaaatg     900

tccgttcggt tggcagaagc tatgaaacga tatgggctga atacaaatca cagaatcgtc     960

gtatgcagtg aaaactctct tcaattcttt atgccggtgt tgggcgcgtt atttatcgga    1020

gttgcagttg cgcccgcgaa cgacatttat aatgaacgtg aattgctcaa cagtatgggc 1080

atttcgcagc ctaccgtggt gttcgtttcc aaaaaggggt tgcaaaagat tttgaacgtg 1140

caaaagaagc tcccaatcat ccaaaaaatt attatcatgg attctaaaac ggattaccag 1200

ggatttcagt cgatgtacac gttcgtcaca tctcatctac ctcccggttt taatgaatac 1260

gattttgtgc cagagtcctt cgatagggac aagacaattg cactgatcat gaactcctct 1320

ggatctactg gtctgcctaa aggtgtcgct ctgcctcata gaactgcctg cgtgagattc 1380

tcgcatgcca gagatcctat ttttggcaat caaatcattc cggatactgc gattttaagt 1440

gttgttccat tccatcacgg ttttggaatg tttactacac tcggatattt gatatgtgga 1500

tttcgagtcg tcttaatgta tagatttgaa gaagagctgt ttctgaggag ccttcaggat 1560

tacaagattc aaagtgcgct gctggtgcca accctattct ccttcttcgc caaaagcact 1620

ctgattgaca aatacgattt atctaattta cacgaaattg cttctggtgg cgctcccctc 1680

tctaaggaag tcggggaagc ggttgccaag aggttccatc tgccaggtat caggcaagga 1740

tatgggctca ctgagactac atcagctatt ctgattacac ccgaggggga tgataaaccg 1800

ggcgcggtcg gtaaagttgt tccatttttt gaagcgaagg ttgtggatct ggataccggg 1860

aaaacgctgg gcgttaatca aagaggcgaa ctgtgtgtga gaggtcctat gattatgtcc 1920

ggttatgtaa acaatccgga agcgaccaac gccttgattg acaaggatgg atggctacat 1980

tctggagaca tagcttactg ggacgaagac gaacacttct tcatcgttga ccgcctgaag 2040

tctctgatta agtacaaagg ctatcaggtg gctcccgctg aattggaatc catcttgctc 2100

caacacccca acatcttcga cgcaggtgtc gcaggtcttc ccgacgatga cgccggtgaa 2160

cttcccgccg ccgttgttgt tttggagcac ggaaagacga tgacggaaaa agagatcgtg 2220

gattacgtcg ccagtcaagt aacaaccgcg aaaaagttgc gcggaggagt tgtgtttgtg 2280

gacgaagtac cgaaaggtct taccggaaaa ctcgacgcaa gaaaaatcag agagatcctc 2340

ataaaggcca agaagggcgg aaagatcgcc gtgtaagcgg ccgc 2384

<210> 42
<211> 63
<212> DNA
<213> Artificial

<220>
<223> An artificially synthesized primer sequence

<400> 42
atagcggccg caatgtaccc atacgacgtc ccagactacg ctgtgagcaa gggcgaggag 60

ctg 63

<210> 43
<211> 33
<212> DNA
<213> Artificial

<220>
<223> An artificially synthesized primer sequence

<400> 43
tatgcggccg cttacttgta cagctcgtcc atg 33

<210> 44
<211> 63
<212> DNA
<213> Artificial

<220>
<223> An artificially synthesized primer sequence

<400> 44
tatgcggccg cttaagcgta gtctgggacg tcgtatgggt acttgtacag ctcgtccatg 60

ccg 63

## Description

### Technical Fiend

The present invention relates to methods of expressing a foreign gene(s) in target cells, which methods comprise the step of introducing into the target cells viral particles in which either a helper minus-strand RNA viral vector or a minus-strand RNA viral minigenome that carries a foreign gene(s) is packaged. The present invention also relates to helper minus-strand RNA viral vectors and minus-strand RNA viral minigenomes. The present invention further relates to methods of producing viral particles in which either a helper minus-strand RNA viral vector or a minus-strand RNA viral minigenome is packaged. The present invention also relates to the viral particles.

### Background Art

Conventional protein expression systems (for protein production, functional analysis, etc.) can be roughly grouped into two types: non-viral protein expression systems and viral protein expression systems. Non-viral protein expression systems have advantages such as simplicity in preparation, low cost, capacity of large-scale production in a short time, high biosafety, and such. However, the gene transfer efficiency *in vitro* largely depends on the transfer reagents, the cell types, and such, and the gene transfer efficiency *in vivo* is very low. Meanwhile, viral protein expression systems often achieve high gene transfer efficiency both *in vitro* and *in vivo.* However, these systems have disadvantages such as higher cost, need of a longer preparation time, and necessity of paying more attention to biosafety, as compared to non-viral vectors. Since these two types of protein expression systems have both advantages and disadvantages, there has been a need to develop a novel protein expression system with a higher-performance.

Sendai virus vectors (hereinafter sometimes referred to as "SeV") are cytoplasmic-type viral vectors. The vectors have superior characteristics such as high gene transfer efficiency and high expression efficiency both *in vitro* and *in vivo,* and the ability to achieve long-term sustained expression *in vitro.* However, the vectors also have disadvantages that are common to virus vectors, such as high preparation costs, and difficulty in large-scale production in a short time.

Meanwhile, it is known that there are defective interfering (DI) particles, which lack a part of the SeV genome and thus require a normal virus as a helper for their propagation, and which have a property to suppress (interfere with) propagation of the normal virus. Non-Patent Document 1 reports an example of expressing a foreign protein of interest based on this property of DI particles, in which an artificial DI (SeV minigenome) cDNA plasmid that carries a foreign gene was constructed and introduced into target cells together with a helper SeV or a helper plasmid expressing NP, P, and L. This enables protein expression by minigenome systems, in which vector cDNAs are easier to prepare as compared to recombinant SeV vectors. However, the efficiency of gene transfer and expression needs to be improved as it is low both *in vivo* and *in vitro.*

In view of the above, there is a need to develop a novel expression system that has the advantages of both expression systems, using viral vectors and minigenomes.

Prior art document information relating to the present invention is indicated below: Non-Patent Document 1: Vullienoz et al., Journal of General Virology, 86:171-180, 2005

### Disclosure of the Invention

### [Problems to be Solved by the Invention]

The present invention was achieved in view of the above circumstances. An objective of the present invention is to provide methods of expressing a foreign gene(s) in target cells, which methods comprise the step of introducing into the target cells viral particles in which either a helper minus-strand RNA viral vector or a minus-strand RNA viral minigenome that carries a foreign gene(s) is packaged. Another objective of the present invention is to provide helper minus-strand RNA viral vectors and minus-strand RNA viral minigenomes. A further objective of the present invention is to provide methods of producing viral particles in which either a helper minus-strand RNA viral vector or a minus-strand RNA viral minigenome is packaged. Another objective of the present invention is to provide the viral particles.

### [Means for Solving the Problems]

To achieve the above objectives, the present inventors used a SeV minigenome (MiniSeV) system to develop a novel protein expression system that can be simply prepared, which enables low-cost and large-scale production in a short time, while exploiting the superior characteristics of SeV vectors. The SeV minigenome system has advantageous characteristics such as preparation simplicity and high transcription/replication efficiency. However, this SeV minigenome system has been difficult to complete as a high-expression vector, since it requires a normal virus or a plasmid expressing NP, P, and L, as a helper for intracellular autonomous replication.

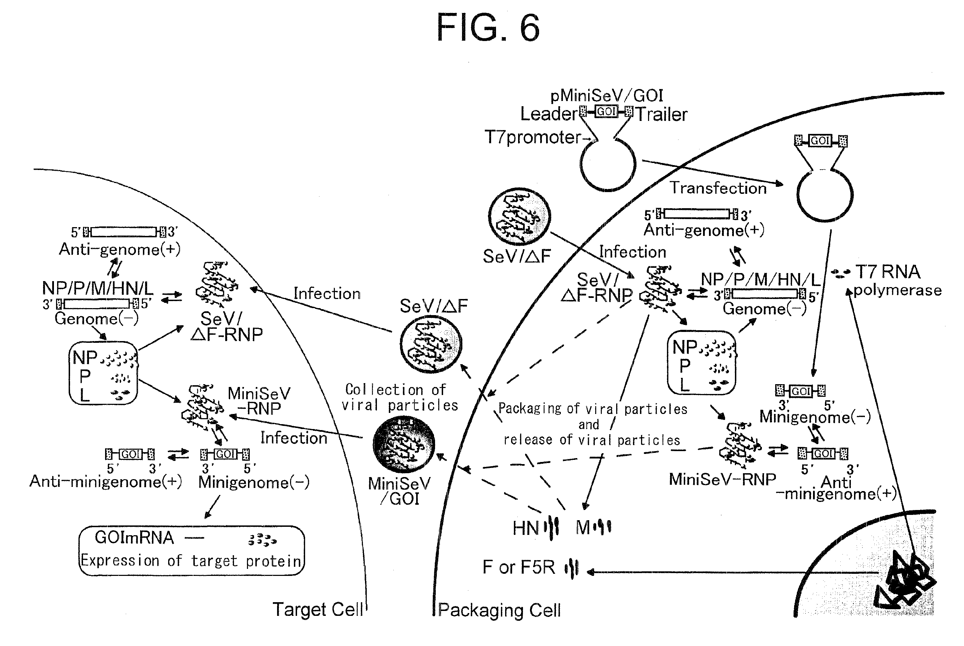

The present inventors devised a protein expression system in which both MiniSeV and SeV particles coexisted (Fig. 6), and assessed the system for the ability to introduce a gene(s) of interest into target cells and the ability to express the gene(s) in the target cells. First, SeV/ΔF (SeV lacking the SeV fusion protein (F protein)) and a SeV minigenome cDNA plasmid that carries a gene(s) of interest (GOI) were introduced into production cells expressing T7 RNA polymerase and the SeV fusion protein (F protein). Then, culture supernatants in which MiniSeV/GOI and SeV/ΔF particles coexisted were collected. By using the coexisting MiniSeV/GOI and SeV/ΔF particles, the inventors achieved a high gene transfer efficiency of a gene(s) of interest (GOI) into target cells and high expression efficiency of the genes in the cells, both *in vitro* and *in vivo*. In the present invention, GFP is used as an example of the gene of interest (GOI).

Furthermore, the present inventors discovered that high gene transfer efficiency of a gene(s) of interest into target cells and high expression efficiency of the gene(s) in the cells can be achieved by using the RNA polymerase I promoter or attaching a tag sequence (HA-Tag) to the gene(s) of interest in the SeV minigenome of the above-described system with coexisting particles.

That is, the present inventors succeeded in constructing a protein expression system in which MiniSeV and SeV particles coexisted, which has a high ability to transfer a gene(s) of interest into target cells and high ability to express the gene(s) in the cells. Thus, the present inventors completed the present invention.

More specifically, the present invention provides [1] to [51] shown below:
[1] a method of expressing a foreign gene(s) in target cells, said method comprising the steps of:
   (a) introducing into production cells a helper minus-strand RNA viral vector and a minus-strand RNA viral minigenome that carries the foreign gene(s);
   (b) collecting viral particles in which either the helper minus-strand RNA viral vector or the minus-strand RNA viral minigenome is packaged, wherein the vector and the minigenome are produced in said production cells; and
   (c) introducing into the target cells the viral particles collected in step (b);
[2] the method of [1], wherein step (a) comprises further introducing into the production cells a vector that expresses NP, P, and L proteins;
[3] the method of [1], wherein the helper minus-strand RNA viral vector is a ribonucleoprotein (RNP) complex of a virus-like particle (VLP);
[4] the method of [1], wherein the minus-strand RNA viral minigenome has one or more promoters;
[5] the method of [4], wherein at least one of the promoters is the RNA polymerase I promoter;
[6] the method of any one of [1] to [5], wherein the minus-strand RNA virus is a paramyxovirus;
[7] the method of [6], wherein paramyxovirus NP, P, and L proteins are expressed from the helper minus-strand RNA viral vector or the minus-strand RNA viral minigenome;
[8] the method of [6], wherein the helper minus-strand RNA viral vector is a vector comprising a single-stranded minus-strand RNA that has been altered so as not to express one or more of paramyxovirus F, M, and HN proteins, and wherein the 5'-end trailer region of the single-stranded minus-strand RNA has been replaced with a leader region;
[9] the method of [8], wherein the production cells express one or more of F, M, and HN proteins, and wherein the helper minus-strand RNA viral vector has been altered so as not to express proteins expressed by the production cells;
[10] the method of [8] or [9], wherein the helper minus-strand RNA viral vector has been altered so as not to express F protein;
[11] the method of any one of [8] to [10], wherein the helper minus-strand RNA viral vector has a mutation(s) in P gene and/or C gene;
[12] the method of [11], wherein the helper minus-strand RNA viral vector comprises:
   (a) a single-stranded minus-strand RNA comprising the nucleotide sequence of SEQ ID NO: 10; or
   (b) a single-stranded minus-strand RNA that hybridizes under stringent conditions to the complementary strand of a minus-strand RNA comprising the nucleotide sequence of SEQ ID NO: 10;
[13] the method of [6], wherein the paramyxovirus is Sendai virus;
[14] the method of [6], wherein the helper minus-strand RNA viral vector is a wild type Sendai virus vector;
[15] the method of any one of [1] to [14], wherein the production cells express T7 RNA polymerase;
[16] the method of any one of [1] to [15], wherein the foreign gene(s) is expressed in target cells *in vitro*;
[17] the method of [16], wherein the target cells are 293T cells, A549 cells, BHK-21 cells, C2C12 cells, HeLa cells, LLC-MK₂ cells, MDCK cells, or NIH3T3 cells;
[18] the method of any one of [1] to [15], wherein the foreign gene(s) is expressed in target cells *in vivo*;
[19] the method of any one of [1] to [18], wherein a tag sequence is attached to the foreign gene(s);
[20] the method of [19], wherein the tag sequence is an HA-Tag;
[21] a helper minus-strand RNA viral vector comprising a single-stranded minus-strand RNA that has been altered so as not to express one or more of paramyxovirus F, M, and HN proteins, and wherein the 5'-end trailer region of the single-stranded minus-strand RNA has been replaced with a leader region;
[22] the vector of [21], wherein the altered protein is F protein;
[23] the vector of [21] or [22], wherein P gene and/or C gene have a mutation(s);
[24] the vector of [23], wherein the single-stranded minus-strand RNA is:
   (a) a single-stranded minus-strand RNA comprising the nucleotide sequence of SEQ ID NO: 10; or
   (b) a single-stranded minus-strand RNA that hybridizes under stringent conditions to the complementary strand of a minus-strand RNA comprising the nucleotide sequence of SEQ ID NO: 10;
[25] the vector of any one of [21] to [24], wherein the helper minus-strand RNA viral vector is a ribonucleoprotein (RNP) complex or a virus-like particle (VLP);
[26] the vector of any one of [21] to [25], wherein the minus-strand RNA virus is a paramyxovirus;
[27] the vector of [26], wherein the paramyxovirus is Sendai virus;
[28] a minus-strand RNA viral minigenome that carries a foreign gene(s);
[29] the minus-strand RNA viral minigenome of [28], wherein the viral minigenome has one or more promoters;
[30] the minus-strand RNA viral minigenome of [29], wherein at least one of the promoters is the RNA polymerase I promoter;
[31] the minus-strand RNA viral minigenome of any one of [28] to [30], wherein the minus-strand RNA virus is a paramyxovirus;
[32] the minus-strand RNA viral minigenome of [31], wherein the paramyxovirus is Sendai virus;
[33] the minus-strand RNA viral minigenome of any one of claims 28 to 32, wherein a tag sequence is attached to the foreign gene;
[34] the minus-strand RNA viral minigenome of claim 33, wherein the tag sequence is an HA-Tag;
[35] a method of expressing a foreign gene(s) in target cells, said method comprises the step of introducing into the target cells the helper minus-strand RNA viral vector of any one of [21] to [27] and the minus-strand RNA viral minigenome of any one of [28] to [34];
[36] a method of producing viral particles, said method comprises the steps of:
   (a) introducing into production cells a helper minus-strand RNA viral vector and a minus-strand RNA viral minigenome that carries a foreign gene(s); and
   (b) collecting viral particles in which either the helper minus-strand RNA viral vector or the minus-strand RNA viral minigenome is packaged, wherein the vector and the minigenome are produced in said production cells;
[37] the method of [36], wherein step (a) comprises the step of further introducing into production cells a vector that expresses NP, P, and L proteins;
[38] the method of [36], wherein the helper minus-strand RNA viral vector is a ribonucleoprotein (RNP) complex or a virus-like particle (VLP);
[39] the method of [36], wherein the minus-strand RNA viral minigenome has one or more promoters;
[40] the method of [39], wherein at least one of the promoters is the RNA polymerase I promoter;
[41] the method of any one of [36] to [40], wherein the minus-strand RNA virus is a paramyxovirus;
[42] the method of [41], wherein paramyxovirus NP, P, and L proteins are expressed from the helper minus-strand RNA viral vector or the minus-strand RNA viral minigenome;
[43] the method of [41], wherein the helper minus-strand RNA viral vector is a vector comprising a single-stranded minus-strand RNA that has been altered so as not to express one or more of paramyxovirus F, M, and HN proteins, and wherein the 5'-end trailer region of the single-stranded minus-strand RNA has been replaced with a leader region;
[44] the method of [43], wherein the production cells express one or more of F, M, and HN proteins, and wherein the helper minus-strand RNA viral vector has been altered so as not to express the proteins expressed in the production cells.
[45] the method of [43] or [44], wherein the altered protein of the helper minus-strand RNA viral vector is F protein;
[46] the method of any one of [43] to [45], wherein the helper minus-strand RNA viral vector has a mutation(s) in P gene and/or C gene;
[47] the method of [46], wherein the helper minus-strand RNA viral vector comprises:
   (a) a single-stranded minus-strand RNA comprising the nucleotide sequence of SEQ ID NO: 10;
   (b) a single-stranded minus-strand RNA that hybridizes under stringent conditions to the complementary strand of a minus-strand RNA comprising the nucleotide sequence of SEQ ID NO: 10;
[48] the method of [41], wherein the paramyxovirus is Sendai virus;
[49] the method of [41], wherein the helper minus-strand RNA viral vector is a wild type Sendai virus vector;
[50] the method of any one of [36] to [49], wherein the production cells express T7 RNA polymerase; and
[51] viral particles collected by the method of any one of [36] to [50], wherein either a helper minus-strand RNA viral vector or a minus-strand RNA viral minigenome is packaged in the particles.

### Brief Description of the Drawings

Fig. 1 depicts the construction of pSeVagp55/ΔF.
Fig. 2 depicts the construction of pSeVagp55/(P/C)m/ΔF.
Fig. 3 depicts the design of a SeV minigenome. (A) Single-promoter SeV minigenome; MiniSeV_{SP}/GOI. (B) Double-promoter SeV minigenome; MiniSeV_{DP}/GOI. (1) The nucleotide C was mutated to A at position 58 of GP. This mutation significantly impaired the function of S. (2) The deletion of the first 12 nucleotides of GP significantly impaired the function of the 3' promoter.
Fig. 4 depicts the construction of a single-promoter SeV minigenome cDNA.
Fig. 5 depicts the construction of a double-promoter SeV minigenome cDNA.
Fig. 6 depicts the mechanism of the protein expression system with coexisting MiniSeV and SeV/ΔF particles.
Fig. 7 indicates photographs showing the effect of the design of helper SeV on the productivity of coexisting particles. (A) Production of coexisting particles in packaging cells. (B) Infection of target cells with coexisting particles.
Fig. 8 indicates photographs and a diagram showing the effect of the design of MiniSeV cDNA on the productivity of coexisting particles. (A) Production of coexisting particles in packaging cells. (B) Titers of coexisting particles produced.
Fig. 9 indicates photographs and a diagram showing comparison of the productivity of coexisting particles between SeVagp55/ΔF and SeVagp55/(P/C)m/ΔF. (A) Production of coexisting particles in packaging cells (B) Infection of target cells with coexisting particles. (C) Titers of coexisting particles produced.
Fig. 10 indicates photographs and a diagram showing improvement of the amount ratio between MiniSeV and helper SeV in coexisting particles. (A) Production of coexisting particles in packaging cells. (B) Titers of coexisting particles produced and the amount ratio of helper SeV/MiniSeV.
Fig. 11 depicts photographs showing *in vitro* expression using MiniSeV_{DP}/GFP and SeVagp55/ΔF coexisting particles.
Fig. 12 depicts photographs showing *in vivo* expression (intranasal GFP expression) using the coexisting particles.
Fig. 13 depicts photographs showing protein expression using the MiniSeV_{DP}/GFP cDNA plasmid and infectious SeVagp55/ΔF particles.
Fig. 14 depicts construction of a minigenome cDNA using the RNA polymerase I promoter.
Fig. 15 depicts construction of a minigenome cDNA that carries an HA-Tag-attached gene (GFP).
Fig. 16 indicates photographs showing *in vitro* expression by the coexisting particle system using the RNA polymerase I promoter.
Fig. 17 indicates photographs showing *in vitro* expression by the coexisting particle system, which carries an HA-Tag-attached gene.

### Best Mode for Carrying Out the Invention

The present inventors discovered that high-efficiency transfer and expression of a gene(s) of interest could be achieved by introducing into production cells a helper minus-strand RNA viral vector and a minus-strand RNA viral minigenome that carries a foreign gene(s), and then introducing into target cells viral particles in which either the vector or the minigenome is packaged in the production cells. The present invention is based on these findings.

The present invention relates to methods of expressing a foreign gene(s) in target cells, which methods comprise the steps of:
(a) introducing into production cells a helper minus-strand RNA viral vector and a minus-strand RNA viral minigenome that carries a foreign gene(s);
(b) collecting viral particles in which either the helper minus-strand RNA viral vector or the minus-strand RNA viral minigenome is packaged, which vector and minigenome are produced in the production cells; and
(c) introducing into the target cells the viral particles collected in step (b).

Herein, "minus-strand RNA virus" refers to a virus comprising as its genome a minus-strand (antisense strand complementary to a sense strand encoding viral proteins) RNA. "Minus-strand RNA virus" is also referred to as "negative-strand RNA virus". Particularly preferred minus-strand RNA viruses used in the present invention are single-stranded minus-strand RNA viruses (also referred to as non-segmented minus-strand RNA viruses). "Single-stranded negative-strand RNA virus" refers to a virus comprising as its genome a single-stranded negative strand (i.e., minus strand) RNA. Such viruses include those belonging to *Paramyxoviridae* (including the genera *Paramyxovirus*, *Morbillivirus*, *Rubulavirus*, *Pneumovirus*, etc.); *Rhabdoviridae* (including the genera *Vesiculovirus*, *Lyssavirus*, *Ephemerovirus*, etc.); *Filoviridae*; *Bunyaviridae* (including the genera *Bunyavirus*, *Hantavirus*, *Nairovirus*, *Phlebovirus*, etc.); *Arenaviridae*, and such. The minus-strand RNA virus vector used in the present invention may be a transmissible vector or may be a non-transmissible defective vector. "Transmissible" means that when a host cell is infected with a viral vector, the virus replicates in the cell to produce infectious viral particles. Defective vectors include, for example, vectors lacking at least one of the genes encoding envelope-constituting proteins. Specifically, such vectors include vectors lacking at least one of the genes encoding the envelope-constituting proteins, such as the F, H, HN, G, M, and M1 proteins (WO 00/70055 and WO 00/70070; Li, H.-O. et al., J. Virol. 74(14) 6564-6569 (2000)), while the types of proteins may vary depending on the virus types.

Specific examples of particularly preferred minus-strand RNA viruses suitable for use in the context of the present invention include, for example, Sendai virus, Newcastle disease virus, mumps virus, measles virus, respiratory syncytial virus (RS virus), rinderpest virus, distemper virus, simian parainfluenza virus (SV5), and human parainfluenza viruses 1, 2, and 3 belonging to Paramyxoviridae; vesicular stomatitis virus and rabies virus belonging to Rhabdoviridae. In the present invention, preferred paramyxoviruses include viruses belonging to Paramyxovirinae (including Respirovirus, Rubulavirus, and Morbillivirus), more preferably those belonging to the genus Respirovirus (also referred to as Paramyxovirus) or derivatives thereof. The derivatives include viruses that are genetically-modified or chemically-modified in a manner not to impair their gene-transferring ability. Examples of viruses of the genus Respirovirus applicable to this invention are human parainfluenza virus-1 (HPIV-1), human parainfluenza virus-3 (HPIV-3), bovine parainfluenza virus-3 (BPIV-3), Sendai virus (also referred to as murine parainfluenza virus-1), and simian parainfluenza virus-10 (SPIV-10). In the context of the present invention, a more preferred paramyxovirus is the Sendai virus. These viruses may be derived from natural strains, wild type strains, mutant strains, laboratory-passaged strains, artificially constructed strains, or the like.

For example, in Sendai virus (SeV), the genome size of a naturally occurring virus is about 15,000 nucleotides. On its negative strand, six genes are aligned following a short 3' leader region, which genes encode NP (Nucleocapsid), P (Phospho), M (Matrix), F (Fusion), HN (Hemagglutinin-neuraminidase), and L (Large) proteins. The negative strand further has a short 5' trailer region at its 5' end.

Paramyxovirus "NP, P, M, F, HN, and L genes" refer to the genes encoding Nucleocapsid, Phospho, Matrix, Fusion, Hemagglutinin-neuraminidase, and Large proteins, respectively. The Phospho (P) protein is a phosphorylated protein that is a small subunit of RNA polymerase. The Matrix (M) protein functions to support the viral particle structure from the inside. The Fusion (F) protein is a protein for membrane fusion, which is involved in the viral invasion into host cells. The Hemagglutinin-neuraminidase (HN) protein is involved in the viral adhesion to host cells. The Large protein is a large subunit of RNA polymerase. Each of the above genes has an individual transcription regulatory unit, and thus mRNA is independently transcribed from each gene, which is transcribed into a protein. In addition to the P protein, the P gene is translated into, a non-structural protein (C) using an ORF other than that of the P protein, and also another protein (V) via RNA edition of the P protein mRNA during transcription. Respective genes of the viruses belonging to the paramyxovirus subfamily are commonly represented from the 3' end in as shown below. In general, the NP gene is also referred to as "N gene".
The genus *Respirovirus*: N P/C/V M F HN - L
The genus *Rubulavirus*: N P/V M F HN (SH) L
The genus *Morbillivirus*: N P/C/V M F H - L

For example, the database accession numbers for the nucleotide sequences of each of the Sendai virus genes are as follows:
M29343, M30202, M30203, M30204, M51331, M55565, M69046, and X17218 for the N gene; M30202, M30203, M30204, M55565, M69046, X00583, X17007, and X17008 for the P gene; D11446, K02742, M30202, M30203, M30204, M69046, U31956, X00584, and X53056 for the M gene;
D00152, D11446, D17334, D17335, M30202, M30203, M30204, M69046, X00152, and X02131 for the F gene;
D26475, M12397, M30202, M30203, M30204, M69046, X00586, X02808, and X56131 for the HN gene; and
D00053, M30202, M30203, M30204, M69040, X00587, and X58886 for the L gene.

Examples of viral genes encoded by other viruses are as follows:
CDV, AF014953; DMV, X75961; HPIV-1, D01070; HPIV-2, M55320; HPIV-3, D10025; Mapuera, X85128; Mumps, D86172; MV, K01711; NDV, AF064091; PDPR, X74443; PDV, X75717; RPV, X68311; SeV, X00087; SV5, M81442; and Tupaia, AF079780 for the N gene; CDV, X51869; DMV, Z47758; HPIV-1, M74081; HPIV-3, X04721; HPIV-4a, M55975; HPIV-4b, M55976; Mumps, D86173; MV, M89920; NDV, M20302; PDV, X75960; RPV, X68311; SeV, M30202; SV5, AF052755; and Tupaia, AF079780 for the P gene;
CDV, AF014953; DMV, Z47758; HPIV-1, M74081; HPIV-3, D00047; MV, ABO16162; RPV, X68311; SeV, AB005796; and Tupaia, AF079780 for the C gene;
CDV, M12669; DMV Z30087; HPIV-1, S38067; HPIV-2, M62734; HPIV-3, D00130; HPIV-4a, D10241; HPIV-4b, D10242; Mumps, D86171; MV, AB012948; NDV, AF089819; PDPR, Z47977; PDV, X75717; RPV, M34018; SeV, U31956; and SV5, M32248 for the M gene;
CDV, M21849; DMV, AJ224704; HPN-1, M22347; HPIV-2, M60182; HPIV-3, X05303, HPIV-4a, D49821; HPIV-4b, D49822; Mumps, D86169; MV, AB003178; NDV, AF048763; PDPR, Z37017; PDV, AJ224706; RPV, M21514; SeV, D17334; and SV5, AB021962 for the F gene;
CDV, AF112189; DMV, AJ224705; HPIV-1, U709498; HPIV-2, D000865; HPIV-3, AB012132; HPIV-4A, M34033; HPIV-4B, AB006954; Mumps, X99040; MV, K01711; NDV, AF204872; PDPR, X74443; PDV, Z36979; RPV, AF132934; SeV, U06433; and SV-5, S76876 for the HN (H or G) gene; and
CDV, AF014953; DMV, AJ608288; HPIV-1, AF117818; HPIV-2, X57559; HPIV-3, AB012132; Mumps, AB040874; MV, K01711; NDV, AY049766; PDPR, AJ849636; PDV, Y09630; RPV, Z30698; and SV-5, D13868 for the L gene.

However, since multiple strains are known for each virus, there are genes consisting of sequences other than those examples listed above depending on the strain.

Herein, "minus-strand RNA viral minigenome" refers to a genome derived from interfering defective particles of a minus-strand RNA virus, which particles lack part of their genome and thus require for their propagation a helper virus that functions to complement the lacking genes, and inhibit the propagation of the helper virus.

Desired foreign genes can be inserted into the genomic RNA of a minus-strand RNA viral minigenome. Such foreign genes are not particularly limited, and may be those that encode full-length natural proteins, or partial fragments thereof (seven or more amino acids, preferably eight, ten, fifteen or more amino acids), or fusion polypeptides in which the above proteins/fragments and other polypeptides are fused, or such. Recombinant viral vectors into which a foreign gene(s) is introduced are obtained by inserting an antisense gene(s) complementary to the sense strand(s) of the gene(s) into the genome of a minus-strand RNA viral minigenome.

Transcription/replication efficiency is higher if the number of genes carried by a minigenome is smaller (*i*.*e*., if the genome size is smaller). The minus-strand RNA viral minigenome of the present invention may lack more than one of the proteins, most preferably lacks all of the proteins that constitute a single-stranded minus-strand RNA virus.

Any site within the non-protein-coding regions of a viral genome can be selected for the position of gene insertion. For example, the position of insertion may be between the 3' leader region of genome RNA and the viral protein ORF closest to the 3' end, between the viral protein ORFs, and/or between the viral protein ORF closest to the 5' end and the 5' trailer region of genomic RNA. Alternatively, in a minus-strand RNA viral minigenome in which a plurality of genes is deleted, the position of gene insertion may be within the deleted regions. When a foreign gene is inserted into the viral minigenome of *Paramyxoviridae*, the chain length of a polynucleotide fragment inserted into the genome is preferably a multiple of six (Kolakofski, D. et al., J. Virol. 1998: 72; 891-899; Calain, P. and Roux, L. J. Virol. 1993: 67; 4822-4830). An E-I-S sequence should be arranged between the inserted foreign gene and an ORF of the minus-strand RNA viral minigenome. Two or more foreign genes can be inserted in tandem via E-I-S sequences.

The minus-strand RNA viral minigenome of the present invention may have one or more genomic promoters. In the present invention, such genomic promoters, which are not particularly limited, include, for example, the RNA polymerase I promoter. When cells expressing RNA polymerase I are used as production cells, there is no need to supply RNA polymerase I using a foreign vector (helper minus-strand RNA viral vector) and therefore the supply cost can be saved. In addition, high-efficiency gene expression can be achieved, as with the T7 system.

The expression level of a foreign gene can be controlled by altering the type of transcription initiation sequence added to the upstream of the gene (on the 3' side of the minus strand (negative strand)) (WO 01/18223). The level can also be controlled by altering the position of the foreign gene within the genome. The closer the position of insertion is to the 3' end of the minus strand, the higher the expression level is. The closer the position of insertion is to the 5' end, the lower the expression level is. To achieve a high level of foreign gene expression, the gene encoding a foreign polypeptide is preferably linked to a high-efficiency transcription initiation sequence and inserted near the 3' end of the minus-strand genome. Specifically, the gene may be inserted between the 3' leader region and the viral protein ORF closest to the 3' end. Alternatively, the gene may be inserted between the ORFs of viral protein genes that are first- and second-closest to the 3' end, or between the viral protein genes that are second- and third-closest to the 3' end.

For example, a desired S sequence of a minus-strand RNA virus may be used as the S sequence attached to a nucleic acid encoding a foreign gene when the nucleic acid is inserted into a genome. The consensus sequence 3'-UCCCWVUUWC-5' (W=A or C; V=A, C, or G) (SEQ ID NO: 22) can be preferably used for Sendai viruses. Particularly preferred sequences are: 3'-UCCCAGUUUC-5' (SEQ ID NO: 23), 3'-UCCCACUUAC-5' (SEQ ID NO: 24), and 3'-UCCCACUUUC-5' (SEQ ID NO: 25). When shown as plus strand-encoding DNA sequences, these sequences are respectively: 5'-AGGGTCAAAG-3' (SEQ ID NO: 26), 5'-AGGGTGAATG-3' (SEQ ID NO: 27), and 5'-AGGGTGAAAG-3' (SEQ ID NO: 28). A preferred E sequence for Sendai viral vectors is, for example, 3'-AUUCUUUUU-5' (SEQ ID NO: 29) (the sequence of the plus strand-encoding DNA is 5'-TAAGAAAAA-3' (SEQ ID NO: 30)). An I sequence may be, for example, any three nucleotides. Specifically, 3'-GAA-5' (5'-CTT-3' in the plus-strand DNA) may be used.

A nucleotide sequence encoding a tag sequence may be attached to the foreign gene(s) of the present invention. Even when the function of a foreign gene(s) is unknown, the expression of the foreign gene(s) can be confirmed by attaching a tag sequence. The position to which a tag sequence is attached is not particularly limited. A tag sequence may be attached to the amino or carboxyl terminus of the foreign gene(s). The types of tag sequences are not particularly limited. For example, an HA-Tag sequence can be used.

Herein, "helper minus-strand RNA viral vector" refers to a minus-strand RNA viral vector that complements genes defective in a minus-strand RNA viral minigenome in order to allow the minus-strand RNA viral minigenome to propagate.

The helper minus-strand RNA viral vector of the present invention may be a ribonucleoprotein (RNP) complex or a virus-like particle (VLP).

In general, paramyxoviruses contain complexes consisting of RNA and proteins (ribonucleoproteins; RNPs) inside their envelope. The RNA comprised in the RNPs is a single-stranded (-) strand (negative strand) RNA, which is the genome of paramyxovirus. The single-stranded RNA binds to the NP, P, and L proteins, and thus forms the RNPs. The RNA comprised in the RNPs serves as a template for transcription/replication of the viral genome (Lamb, R.A., and D. Kolakofsky, 1996, Paramyxoviridae: The viruses and their replication. pp.1177-1204. In Fields Virology, 3rd edn. Fields, B. N., D. M. Knipe, and P. M. Howley et al. (ed.), Raven Press, New York, N. Y).

Typically, a single-stranded minus-strand RNA (genomic RNA) of the helper minus-strand viral vector of the present invention preferably expresses NP, P, and L proteins. However, the single-stranded minus-strand RNA is not limited to those expressing the above proteins. In the present invention, paramyxovirus NP, P, and L proteins may be expressed by a helper minus-strand RNA viral vector or a minus-strand RNA viral minigenome.

The single-stranded minus-strand RNA (genomic RNA) of the helper minus-strand viral vector of the present invention may be altered so as not to express all or some of the F, M, and/or HN proteins. Paramyxovirus F protein is involved in membrane fusion with the host cell membrane, while HN protein is involved in membrane adhesion. M protein supports the viral particle structure from the inside. The viral vector of the present invention is incapable of particle formation and transmission, since the genomic RNA expresses none of the F, M, and HN proteins. That is, when host cells are infected with the viral vector of the present invention, the vector cannot produce infectious viral particles in the host cells and thus cannot allow any daughter viruses to invade cells adjacent to the cells infected with the viral vector.
Alternatively, the viral vector of the present invention may produce infectious particles. As described below, when the F, M, and/or HN proteins are co-expressed in the reconstruction of the viral vector of the present invention, host cells can be infected with the reconstructed viral vector.

The vector of the present invention can be altered so as not to express envelope proteins by designing a genome lacking F, HN, and M genes. Deletion of F gene is particularly preferred. NP, P, and L genes encoded by the vector genome may retain the gene sequences of the original virus. However, the genes may have mutations, as long as the proteins encoded by the genes bind to the genomic RNA and have the activity to replicate RNPs in cells. Furthermore, single-stranded minus-strand RNAs encoding polypeptides having a deletion, substitution, insertion, or addition of one or more amino acids in the polypeptides encoded by a minus-strand RNA comprising the nucleotide sequence of the genomic RNA, and single-stranded minus-strand RNAs that hybridize under stringent conditions to the complementary strand of a minus-strand RNA comprising the nucleotide sequence of the genomic RNA can also be used as a genomic RNA of the vector of the present invention, as long as the NP, P, and L proteins encoded by the genes of the RNA bind to the genomic RNA and have the RNP-replicating activity. Such mutations can be introduced by techniques well-known to those skilled in the art. For example, site-directed mutations can be introduced by the PCR method, cassette mutagenesis, or such. Alternatively, random mutations can be introduced by using chemical reagents, random nucleotides, or such. The above stringent hybridization conditions can be appropriately selected by those skilled in the art. For example, pre-hybridization is carried out overnight at 42°C using a hybridization solution containing 25% formamide, or 50% formamide under more stringent conditions, and 4x SSC, 50 mM Hepes (pH 7.0), 10x Denhardt's solution, and 20 µg/ml denatured salmon sperm DNA. Then, hybridization is carried out overnight at 42°C. Post-hybridization washes can be carried out using the following washing solutions and temperature conditions: "1x SSC, 0.1% SDS, 37°C" or the like, "0.5x SSC, 0.1% SDS, 42°C" or the like for highly stringent conditions, and "0.2x SSC, 0.1% SDS, 65°C" or the like for more highly stringent conditions. In general, polypeptides encoded by polynucleotides isolated using such hybridization techniques have a high amino acid sequence homology to the polypeptides encoded by the (-) strand RNA described above. "High homology" refers to a sequence homology of at least 40% or more, preferably 60% or more, more preferably 80% or more, even more preferably 90% or more, still more preferably at least 95% or more, and yet more preferably at least 97% or more (for example, 98% to 99%). Amino acid sequence identity can be determined, for example, using the BLAST algorithm by Karlin and Altschul (Proc. Natl. Acad. Sci. USA 87:2264-2268, 1990; Proc. Natl. Acad. Sci. USA 90:5873-5877, 1993). When amino acid sequences are analyzed using BLASTX (Altschul et al. J. Mol. Biol. 215:403-410, 1990) developed based on this algorithm, the parameters may be set at: score=50 and wordlength=3, for example. When the BLAST and Gapped BLAST programs are used, the default parameters for each program are used. Specific procedures for these analytical methods are known (http://www.ncbi.nlm.nih.gov.).

The helper minus-strand RNA viral vector of the present invention may be a vector comprising a single-stranded minus-strand RNA in which the 5' trailer region has been replaced with a leader region. Since the genome transcription/replication efficiency of the antigenomic promoter in the 5' trailer region is higher than that of the genomic promoter in the 3' leader region, the replacement of the 5' trailer region with the 3' leader region leads to the reduction in the genome transcription/replication efficiency of a helper virus. This reduction prevents the competition between the helper virus and a minigenome, and thus results in higher expression of minigenome proteins (Le Mercier P. et al., J Virol. 2002 76(11): 5492-5502).

The helper minus-strand RNA viral vector of the present invention may have a mutation(s) in P gene and/or C gene. The mutations may be those reducing the cytotoxicity of the minus-strand RNA virus. Sites of such mutations are not particularly limited. Examples of preferred substitutions include substitutions of a different amino acid for the following amino acids: Lys at position 9 (K9), Glu at position 13 (E13), Glu at position 17 (E17), Ile at position 29 (129), Ser at position 38 (S38), Glu at position 39 (E39), Thr at position 41 (T41), Arg at position 47 (R47), Ser at position 48 (S48), Asn at position 52 (N52), Asn at position 55 (N55), Thr at position 56 (T56), or Gln at position 58 (Q58) of SeV P protein; and substitutions of a different amino acid for the following amino acids: Leu at position 16 (L16), Lys at position (K17), Lys at position (K18), Lys at position 21 (K21), Leu at position 22 (L22), Arg at position 25 (R25), Gln at position 27 (Q27), Glu at position 28 (E28), Glu at position 30 (E30), Met at position 35 (M35), Leu at position 36 (L36), Asp at position 38 (D38), Met at position 41 (M41), Asn at position 47 (N47), Gln at position 48 (Q48), Glu at position 52 (E52), Glu at position 55 (E55), Thr at position 63 (T63), Lys at position 66 (K66), or Gln at position 68 (Q68) of SeV C protein. A mutation may be introduced at a single site or multiple sites. While such amino acid mutations may be substitutions by a desired different amino acid, preferable mutations are substitutions between amino acids whose side chains have different chemical properties. Amino acids can be categorized into groups, such as basic amino acids (for example, lysine, arginine, and histidine), acidic amino acids (for example, aspartic acid and glutamic acid), non-charged polar amino acids (for example, glycine, asparagine, glutamine, serine, threonine, tyrosine, and cysteine), non-polar amino acids (for example, alanine, valine, leucine, isoleucine, proline, phenylalanine, methionine, and tryptophan), β-branched amino acids (for example, threonine, valine, and isoleucine), and aromatic amino acids (for example, tyrosine, phenylalanine, tryptophan, and histidine). Substitutions include substitutions of an amino acid with another amino acid that belongs to a group other than that of the former amino acid. Specifically, such substitutions include, but are not limited to, substitutions of an acidic or neutral amino acid for a basic amino acid; substitutions of a non-polar amino acid for a polar amino acid; substitutions of an amino acid whose molecular weight is lower than the average molecular weight of the twenty naturally occurring amino acids for an amino acid whose molecular weight is higher than the average molecular weight; substitutions of an amino acid whose molecular weight is higher than the average molecular weight for an amino acid whose molecular weight is lower than the average molecular weight. Specifically, the mutations include, for example, the substitution of Gly for K9 (K9G), the substitution of Gly for E13 (E13G), the substitution of Gly for E17 (E17G), the substitution of Val for I29 (I29V), the substitution of Gly for S38 (S38G), the substitution of Gly for E39 (E39G), the substitution of Ala for T41 (T41A), the substitution of Gly for R47 (R47G), the substitution of Gly for S48 (S48G), the substitution of Ser for N52 (N52S), the substitution of Gly for N55 (N55G), the substitution of Ala for T56 (T56A), and the substitution ofArg for Q58 (Q58R) in SeV P protein. The mutations also include, for example, the mutation of L16 into Trp (L16W), the mutation of K17 into Gly (K17G), the mutation of K18 into Arg (K18R), the mutation of K21 into Gly (K21G), the mutation of L22 into Trp (L22W), the mutation of R25 into Gly (R25G), the mutation of Q27 into Arg (Q27R), the mutation of E28 into Gly (E28G), the mutation of E30 into Gly (E30G), the mutation of M35 into Thr (M35T), the mutation of L36 into Trp (L36W), the mutation of D38 into Gly (D38G), the mutation of M41 into Thr (M41T), the mutation of N47 into Gly (N47G), the mutation of Q48 into Arg (Q48R), the mutation of E52 into Gly (E53G), the mutation of E55 into Gly (E55G), the mutation of T63 into Ala (T63A), the mutation of K66 into Gly (K66G), and the mutation of Q68 into Arg (Q68R).

In the present invention, mutations may be introduced into the L protein. Such mutations of the L protein include substitutions of a different amino acid for Asn at position 1197 (N1197) and/or Lys at position 1795 (K1795), and substitutions at sites homologous to those of the L proteins of other minus-strand RNA viruses. A particularly preferred L protein gene possesses both of the mutation types in the L protein. While the amino acid mutations may be substitutions by a desired different amino acid, the mutations are preferably substitutions between amino acids whose side chains have different chemical properties, as described above. Such substitutions include, for example, substitutions between amino acids of different groups, as described above. Specifically, the substitutions include, for example, the substitution of Ser for N1197 (N1197S) and substitution of Glu for K1795 (K1795E).

The coexistence of mutations in P gene and C gene significantly potentiates the effects such as persistent infectivity, suppressed release of secondary particles, or suppressed cytotoxicity. Combinations of the above mutations with the above recombinant minus-strand RNA virus that has been altered so as not to express F, HN, and/or M proteins can dramatically potentiate these effects. In particular, viruses having mutations in both P and C genes are more preferred. Viruses having a mutation at the above-indicated position in P gene and a mutation at the above-described position in C gene are most preferred.

The helper minus-strand RNA viral vector of the present invention may comprise:
(a) a single-stranded minus-strand RNA comprising the nucleotide sequence of SEQ ID NO: 10; or
(b) a single-stranded minus-strand RNA that hybridizes under stringent conditions to the complementary strand of a minus-strand RNA comprising the nucleotide sequence of SEQ ID NO: 10.

The stringent hybridization conditions in the present invention refers to "6 M urea, 0.4% SDS, 0.5x SSC" or a hybridization condition with a stringency equivalent thereto. DNAs with higher homology can be expected to be isolated when conditions with higher stringency, for example, 6 M urea, 0.4% SDS, and 0.1x SSC, are used. The amino acid sequences encoded by the DNAs isolated as described above are expected to have a high homology to the amino acid sequence of a protein of interest. "High homology" refers to a sequence identity of at least 50% or more, more preferably 70% or more, and still more preferably 90% or more (for example, 95%, 96%, 97%, 98%, 99% or more) in the entire amino acid sequence. Such amino acid sequence identity and nucleotide sequence identity can be determined using the BLAST algorithm by Karin and Altschul (Proc. Natl. Acad. Sci. USA 87:2264-2268, 1990, Proc Natl Acad Sci USA 90: 5873, 1993). Programs called BLASTN and BLASTX have been developed based on the BLAST algorithm (Altschul SF, et al., J Mol Biol 215: 403, 1990). When nucleotide sequences are analyzed using BLASTN, parameters may be set at: score=100 and wordlength=12, for example. Alternatively, when amino acid sequences are analyzed using BLASTX, parameters may be set at: score=50 and wordlength=3, for example. When the BLAST and Gapped BLAST programs are used, default parameters for each program are used. Specific procedures for these analytical methods are known.

Furthermore, the helper minus-strand RNA viral vector of the present invention may be a wild type Sendai virus vector.

Each step of the expression system of the present invention is described below.

In the first step of the expression system of the present invention, the above-described helper minus-strand RNA viral vector and the minus-strand RNA viral minigenome that carries a foreign gene(s) are introduced into production cells.

In the above step of the present invention, a vector that expresses the NP, P, and L proteins may be further introduced into the production cells.

In the present invention, desired mammalian cells or such can be preferably used as the "production cells". Specifically, the cells include, for example, culture cells such as LLC-MK₂ cells (ATCC CCL-7) and CV-1 cells (for example, ATCC CCL-70) derived from simian kidney, and BHK-21 cells (for example, ATCC CCL-10) derived from hamster kidney, and human-derived cells. In the present invention, an example of more preferred production cells is BHK-21 cells. However, the production cells are not particularly limited.

Methods of introducing minus-strand RNA viral minigenome cDNAs into cells include the following: (i) methods of preparing DNA precipitates that can be internalized by targeted cells; (ii) methods of preparing positively-charged complexes comprising DNAs that are suitable for internalization by targeted cells, and that have low cytotoxicity; and (iii) methods of instantaneously creating holes with a size sufficient for DNA molecules to pass through, in the membrane of targeted cells using electric pulses.

In the methods of (ii), various transfection reagents can be used. Such reagents include, for example, Lipofectamine 2000 (Invitrogen Cat. No. 11668-019), DOTMA (Boehringer), Superfect (QIAGEN #301305), DOTAP, DOPE, and DOSPER (Boehringer #1811169). The methods of (i) include, for example, transfection methods using calcium phosphate. While DNA introduced into cells by this method is known to be phagocytosed, a sufficient amount of DNA also enters the nucleus (Graham, F. L. and Van Der Eb, J., 1973, Virology 52: 456; Wigler, M. and Silverstein, S., 1977, Cell 11: 223). Chen and Okayama investigated the optimization of transfer techniques, and reported the following: (1) incubation conditions for cells and co-precipitates are 2 to 4% CO₂, 35°C, and 15 to 24 hours; (2) the activity of circular DNA is higher than that of linear DNA; and (3) optimal precipitation is achieved when the DNA concentration in the precipitate mixture is 20 to 30 µg/ml (Chen, C. and Okayama, H., 1987, Mol. Cell. Biol. 7: 2745). The methods of (ii) are suitable for transient transfection. In previously known methods, transfection is performed by preparing a DEAE-dextran (Sigma #D-9885 M.W. 5 x 10⁵) mixture with a desired DNA concentration. Since most complexes are degraded in endosomes, chloroquine may be added to enhance the effect (Calos, M. P., 1983, Proc. Natl. Acad. Sci. USA 80: 3015). The methods of (iii) are referred to as electroporation methods, which are not cell-selective, and thus have high versatility as compared to the methods of (i) and (ii). The efficiency of these methods is assumed to be high under optimal conditions of duration of pulse electric current, pulse shape, electric field potency (gap between electrodes, voltage), buffer conductivity, DNA concentration, and cell density.

Of the above three categories, the methods of (ii) are simpler to perform and capable of testing many samples using a large amount of cells. Thus, Transfection reagents are suitable in the present invention. Lipofectamine 2000 (Invitrogen Cat. No.11668-019), Superfect Transfection Reagent (QIAGEN, Cat No. 301305), or DOSPER Liposomal Transfection Reagent (Boehringer Mannheim, Cat No. 1811169) is preferably used.

A helper minus-strand RNA viral vector can be reconstituted by known methods.

Titers of collected viruses can be determined, for example, by measuring CIU (Cell-Infected Unit) or hemagglutination activity (HA) (WO 00/70070; Kato, A. et al., 1996, Genes Cells 1: 569-579; Yonemitsu, Y. & Kaneda, Y., Hemaggulutinating virus of Japan-liposome-mediated gene delivery to vascular cells. Ed. by Baker AH. Molecular Biology of Vascular Diseases. Method in Molecular Medicine: Humana Press: pp. 295-306, 1999). Regarding viruses with vectors that carry a marker gene such as a green fluorescent protein (GFP) gene, titers can be quantified by directly counting infected cells using the marker as an indicator (for example, as GFP-CIU). Titers thus determined can be treated in the same way as CIU (WO 00/70070).

The methods of expressing T7 RNA polymerase and F protein (which can be F5R mutant) are not particularly limited in the expression system of the present invention. The methods include, for example, transient expression methods using plasmids or such, and methods using cells with sustained expression of T7 RNA polymerase and F protein (F5R) as production cells. In the present invention, the production cells may express one or more of the F, M, and HN proteins, which are not expressed in a helper minus-strand RNA viral vector that has been altered so as not to express the proteins expressed in the production cells. The amino acid sequences of these proteins supplied from a source other than a viral genome are not necessarily identical to the original viral sequences. As long as the activity resulting from introduction of the nucleic acids is comparable to or greater than the activity of the natural proteins, mutations may be introduced into the sequences or homologous genes of other viruses may be used instead of the sequences. Such proteins of other viruses include, for example, vesicular stomatitis virus (VSV) G protein (VSV-G). In general, envelope proteins often exhibit cytotoxicity. Thus, the proteins may be expressed only at the time of vector reconstruction under the control of an inducible promoter.

When envelope proteins are expressed in production cells at the time of intracellular vector reconstitution, the envelope proteins are incorporated into minigenome viral particles. Thus, viral vectors with infectiveness due to the envelope proteins can be produced.

Once cells are infected with such vectors, they can allow amplification of RNP within the cells. However, the vectors cannot reproduce viruses similar to the original viruses having the envelope proteins, since the vectors themselves have no envelope genes. Such vectors are very useful in fields such as gene therapy, in which a high safety is required.

When the above first step is carried out, viruses which have a helper minus-strand RNA viral vector or a minus-strand RNA viral minigenome that carries a foreign gene(s) are reconstituted and released from production cells.

Specifically, as shown in the Examples (Fig. 6), helper SeV/ΔF (SeVagp55/ΔF is preferred) and a SeV minigenome cDNA plasmid that carries a foreign gene(s) of interest (GOI) (pSeVmini/GOI) are introduced into production cells expressing T7 RNA polymerase and the SeV fusion protein, F protein (which can be F5R mutant). Then, the RNA genome of MiniSeV/GOI is transcribed from pSeVmini/GOI by the action of T7 RNA polymerase, and RNPs of MiniSeV/GOI are formed and replicated using the NP, P, and L proteins expressed from SeV/ΔF serving as a helper. These RNPs of MiniSeV/GOI are packaged into infectious MiniSeV/GOI particles with M and HN expressed from helper SeV/ΔF and F/F5R expressed in production cells, which particles are released along with infectious helper SeV/ΔF particles into the culture supernatant.

In the second step of the expression system of the present invention, viral particles in which either the helper minus-strand RNA viral vector or the minus-strand RNA viral minigenome is packaged are collected, which vector and minigenome are produced within the production cells.

The collected viral particles (viral vectors) can be purified to be substantially pure.
The purification can be achieved using known purification/separation methods including filtration, centrifugation, adsorption, column purification, and such, or any combinations thereof. "Substantially pure" means that vector components constitute a major proportion of a solution including the vector. For example, a viral vector composition can be confirmed to be substantially pure by the fact that the proportion of proteins contained as the viral vector components to the total proteins (excluding proteins added as carriers and stabilizers) in a solution is 10% (w/w) or more, preferably 20% or more, more preferably 50% or more, preferably 70% or more, still more preferably 80% or more, and yet more preferably 90% or more. For example, specific purification methods for paramyxovirus vectors include, but are not limited to, methods using cellulose sulfate esters or cross-linked polysaccharide sulfate esters (Japanese Patent Application Kokoku Publication No. (JP-B) S62-30752 (examined, approved Japanese patent application published for opposition), JP-B S62-33879, and JP-B S62-30753), and methods including adsorbing the viruses to fucose sulfate-containing polysaccharides and/or degradation products thereof (WO 97/32010).

The present invention relates to methods of producing viral particles in which either a helper minus-strand RNA viral vector or a minus-strand RNA viral minigenome is packaged, which methods comprise the above first and second steps. The quantity of released viral particles in which a minus-strand RNA viral minigenome is packaged should be increased to achieve high-efficiency transfer of the minus-strand RNA viral minigenome and high expression of genes of interest. The quantity of released viral particles in which a minus-strand RNA viral minigenome is packaged can be increased by using a variant such as SeVagp55ΔF, as a helper minus-strand RNA viral vector, as described herein.

Furthermore, the ratio of viral particles released from production cells ([helper minus-strand RNA viral vector]: [minus-strand RNA viral minigenome]) should be close to one (it is preferred that the proportion of minus-strand RNA viral minigenome to helper minus-strand RNA viral vector be increased) to reduce the toxicity (cytotoxicity) of viral particles in which a helper minus-strand RNA viral vector is packaged. The proportion of released viral particles in which a minus-strand RNA viral minigenome is packaged can be increased by using a variant such as SeVagp55/(P/C)m/ΔF, as a helper minus-strand RNA viral vector, as described herein.

Various purposes (improvement of transfer/expression, reduction of toxicity) can be achieved by selecting suitable helper minus-strand RNA viral vectors.

In the third step of the expression system of the present invention, the viral particles collected in the above second step are introduced into target cells.

Herein, "target cells" refer to desired cells in which a foreign gene(s) is intended to be expressed by introducing the viral particles of the present invention.

In the present invention, target cells into which the viral particles are introduced may be *in vitro* target cells or *in vivo* target cells (that is, a foreign gene(s) is expressed directly in animals). Such *in vitro* target cells include 293T cells, A549 cells, BHK-21 cells, C2C12 cells, HeLa cells, LLC-MK₂ cells, MDCK cells, and NIH3T3 cells, but are not limited thereto.

When viral particles are introduced into target cells *in vitro*, for example, the target cells are infected with the viral particles in a desired physiological aqueous solution, such as culture medium or physiological saline. In this case, the multiplicity of infection (MOI; the number of infecting viruses per cell) is preferably in the range of 1 to 1000, more preferably 2 to 500, even more preferably 3 to 300, and still more preferably 5 to 100. It is sufficient to contact the viral particles with the target cells only for a short period of time. For example, the contact time may be 1 minute or longer, preferably 3 minutes or longer, 5 minutes or longer, 10 minutes or longer, or 20 minutes or longer, about 1 to about 60 minutes, and more specifically about 5 to about 30 minutes. As a matter of course, the contact time may be longer, for example, several days or longer.

Well-known transfection methods can be used to introduce RNPs or non-infectious viral particles (virus-like particles (VLPs)) that contain viral genomic RNA into cells. Specifically, such transfection of cells can be achieved by various techniques known to those skilled in the art, such as methods that utilize calcium phosphate (Chen, C. & Okayama, H. (1988) BioTechniques 6:632-638; Chen, C. and Okayama, H., 1987, Mol. Cell. Biol. 7: 2745), DEAE-dextran (Rosenthal, N. (1987) Methods Enzymol. 152:704-709), various liposome-based transfection reagents (Sambrook, J. et al. (1989) Molecular Cloning: A Laboratory Manual (Cold Spring Harbor Laboratory, Cold Spring Harbor, NY)), or electroporation (Ausubel, F. et al. (1994) In Current Protocols in Molecular Biology (John Wiley and Sons, NY), Vol. 1, Ch. 5 and 9). Chloroquine may be added to the transfection to suppress the degradation in endosomes (Calos, M. P., 1983, Proc. Natl. Acad. Sci. USA 80: 3015). Transfection reagents include, for example, DOTMA (Roche), Superfect Transfection Reagent (QIAGEN, Cat No. 301305), DOTAP, DOPE, DOSPER (Roche #1811169), TransIT-LT1 (Mirus, Product No. MIR 2300), CalPhosTM Mammalian Transfection Kit (Clontech #K2051-1), and CLONfectin^{™} (Clontech #8020-1). Enveloped viruses are known to incorporate host cell-derived proteins during virion formation, and such proteins can potentially cause antigenicity and cytotoxicity when introduced into cells (J. Biol. Chem. (1997) 272, 16578-16584). It is thus advantageous to use RNPs without the envelope (WO 00/70055).

Furthermore, a foreign gene(s) can be expressed in target cells by introducing into target cells the helper minus-strand RNA viral vector and the minus-strand RNA viral minigenome of the present invention, thereby directly forming viral RNPs in the cells.

The foreign gene(s) is expressed by culturing cells, into which the viral particles (or viral vectors) of the present invention are introduced, for about twelve hours to five days (preferably one to three days).

When the viral particles of the present invention are introduced into target cells *in vivo,* a foreign gene(s) is expressed by directly administering the viral particles to animals.

Cells infected with the above-described viral particles of the present invention (cells into which the particles have been introduced) may be administered to animals (*ex vivo* administration). Cells are inoculated into animals directly, or in the form of a lysate after cell lysing. A lysate of cells infected with vectors can be prepared by methods of lysing the cell membrane using surfactants, methods in which freeze-thaw cycles are repeated, or the like. Nonionic surfactants such as Triton X-100 and Nonidet P-40 are used at a concentration of 0.1 to 1%. Such a lysate can be prepared, for example, by washing with PBS, centrifuging the cells, resuspending the collected cell pellet in TNE buffer [25 mM Tris-HCl (pH 7.5), 150 mM NaCl, 1 mM EDTA, 1% Nonidet P-40], and then allowing the cells to be placed on ice for 10 to 30 minutes. When proteins to be used as immunogens are soluble in the cytoplasm, the yielded lysate is centrifuged (at 10,000 x g for 10 minutes) to precipitate and remove the unnecessary insoluble fraction, and the resulting supernatant can be used for immunization. When used for administration at a site for which surfactants are unfavorable, a lysate is prepared by resuspending the cells in PBS after washing, and then lysing the cells by repeating 5 or 6 cycles of freeze-thawing.

The inoculation route is not particularly limited. A preferred administration includes, for example, intramuscular injection (for example, gastrocnemial muscle), subcutaneous administration, intranasal administration (rhinenchysis), intracutaneous administration to the palm or footpad, direct administration into the spleen, and intraperitoneal administration. The number of inoculation sites may be one or more (for example, two to fifteen sites). The inoculation dose varies depending on the type of disease; patient's weight, age, sex, and symptoms; the purpose of administration, the form of composition to be administered, the administration method, the gene(s) to be introduced, and such. Those skilled in the art can appropriately determine the inoculation dose. At the time of administration, the subject animal for inoculation, the inoculation site, the frequency of inoculation, and the like may be appropriately selected. For example, when converted into a virus titer, the inoculation dose per site may be 1 x 10⁴ CIU to 5 x 10¹¹ CIU (cell infectious unit), preferably 1 x 10⁶ CIU to 1 x 10¹⁰ CIU. In inoculation via cells (*ex vivo* administration), for example, a culture cell line derived from the same species (for example, sarcoma cell line) can be infected with the virus of the present invention, and then 10⁴ to 10⁹ cells, preferably 10⁵ to 10⁸ cells, or a lysate thereof can be inoculated into animals.

For example, in humans, a single dose is preferably 2 x 10⁵ CIU to 2 x 10¹⁰ CIU and the frequency of administration can be once or more, as long as the side effects are within a clinically acceptable range. The same applies to the daily administration frequency. When protein preparations are produced by using the vector of the present invention, the preferred protein dose is, for example, in the range of 10 ng/kg to 100 µg/kg, preferably 100 ng/kg to 50 µg/kg, and more preferably 1 µg/kg to 5 µg/kg. For non-human animals, the above-described doses can be converted, for example, based on the ratio of body weight or the ratio of volume of the target site for administration (e.g. the average ratio) between the animal of interest and human. The converted doses can be administered to the animals. Subjects to which compositions comprising the vector of the present invention are administered include all mammals such as humans, monkeys, mice, rats, rabbits, sheep, bovines, and dogs.

The helper minus-strand RNA viral vector and the minus-strand RNA viral minigenome of the present invention, viral particles in which either of the above is packaged, and cells into which the viral particles are introduced, and a lysate thereof may be used in combination with desired pharmaceutically acceptable carriers or media.

Such desired pharmaceutically acceptable carriers or media include, for example, sterile water, physiological saline, stabilizers, excipients, buffers, preservatives, surfactants, chelating agents (EDTA and such), and binders.

In the present invention, surfactants include nonionic surfactants. Typical examples of such nonionic surfactants include: sorbitan fatty acid esters such as sorbitan monocaprylate, sorbitan monolaurate, and sorbitan monopalmitate; glycerin fatty acid esters such as glycerin monocaprylate, glycerin monomyristate and glycerin monostearate; polyglycerin fatty acid esters such as decaglyceryl monostearate, decaglyceryl distearate, and decaglyceryl monolinolate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, and polyoxyethylene sorbitan tristearate; polyoxyethylene sorbit fatty acid esters such as polyoxyethylene sorbit tetrastearate, and polyoxyethylene sorbit tetraoleate; polyoxyethylene glycerin fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol, polyoxyethylene polyoxypropylene propyl ether, and polyoxyethylene polyoxypropylene cetyl ether; polyoxyethylene alkyl phenyl ethers such as polyoxyethylene nonylphenyl ether; polyoxyethylene hardened castor oils such as polyoxyethylene castor oil and polyoxyethylene hardened castor oil (polyoxyethylene hydrogenated castor oil); polyoxyethylene beeswax derivatives such as polyoxyethylene sorbit beeswax; polyoxyethylene lanolin derivatives such as polyoxyethylene lanolin; and polyoxyethylene fatty acid amides and the like with an HLB of 6 to 18, such as polyoxyethylene stearic acid amide.

Surfactants also include anionic surfactants. Typical examples of such anionic surfactants include: alkylsulfates having an alkyl group with 10 to 18 carbon atoms, such as sodium cetyl sulfate, sodium lauryl sulfate, and sodium oleyl sulfate; polyoxyethylene alkyl ether sulfates such as sodium polyoxyethylene lauryl sulfate, which have an alkyl group with 10 to 18 carbon atoms, and a two to four average molar number of added ethylene oxide; alkyl sulfosuccinate ester salts having an alkyl group with 8 to 18 carbon atoms, such as sodium lauryl sulfosuccinate ester; natural surfactants such as lecithin; glycerophospholipids; sphingophospholipids such as sphingomyelin; and sucrose fatty acid esters in which the fatty acids have 12 to 18 carbon atoms.

In the present invention, one, two, or more of the surfactants described above can be added in combination. Surfactants that are preferably used in the formulations of the present invention include polyoxyethylene sorbitan fatty acid esters such as Polysorbates 20, 40, 60, and 80. Polysorbates 20 and 80 are particularly preferred. Polyoxyethylene polyoxypropylene glycols such as poloxamer (Pluronic® F-68 and such), are also preferred.

The amount of surfactant added varies depending on the type of surfactant used. When Polysorbate 20 or 80 is used, the concentration is generally in the range of 0.001 to 100 mg/ml, preferably 0.003 to 50 mg/ml, and more preferably 0.005 to 2 mg/ml.

In the present invention, buffers include phosphate, citrate buffer, acetic acid, malic acid, tartaric acid, succinic acid, lactic acid, potassium phosphate, gluconic acid, caprylic acid, deoxycholic acid, salicylic acid, triethanolamine, fumaric acid, and other organic acids; carbonate buffer, Tris buffer, histidine buffer, and imidazole buffer.

Alternatively, liquid formulations may be prepared by dissolution in aqueous buffers known in the field of liquid formulation. In general, the buffer concentration is in the range of 1 to 500 mM, preferably 5 to 100 mM, and more preferably 10 to 20 mM.

Furthermore, the solutions of the present invention may also comprise other low-molecular-weight polypeptides; proteins such as serum albumin, gelatin, and immunoglobulin; amino acids; sugars and carbohydrates such as polysaccharides and monosaccharides; sugar alcohols, and such.

Herein, amino acids include basic amino acids such as arginine, lysine, histidine, ornithine, and inorganic salts of these amino acids (preferably in the form of hydrochloride or phosphate, namely phosphate amino acids). When free amino acids are used, the pH is adjusted to a preferred value by adding appropriate physiologically acceptable buffering substances, for example, inorganic acids, in particular hydrochloric acid, phosphoric acid, sulfuric acid, acetic acid, and formic acid, and salts thereof. In this case, the use of phosphate is especially beneficial since particularly stable freeze-dried products can be obtained. Phosphate is particularly advantageous when the preparations do not substantially contain organic acids such as malic acid, tartaric acid, citric acid, succinic acid, and fumaric acid, or do not contain anions thereof (malate ion, tartrate ion, citrate ion, succinate ion, fumarate ion, and such). Preferred amino acids are arginine, lysine, histidine, and ornithine. Furthermore, it is possible to use acidic amino acids such as glutamic acid and aspartic acid, and salts thereof (preferably sodium salts); neutral amino acids such as isoleucine, leucine, glycine, serine, threonine, valine, methionine, cysteine, and alanine; and aromatic amino acids such as phenylalanine, tyrosine, tryptophan, and its derivative, N-acetyl tryptophan.

Herein, sugars and carbohydrates such as polysaccharides and monosaccharides include, for example, dextran, glucose, fructose, lactose, xylose, mannose, maltose, sucrose, trehalose, and raffinose.

Herein, sugar alcohols include, for example, mannitol, sorbitol, and inositol.

When aqueous solutions for injection are prepared, physiological saline, an isotonic solution containing glucose or other auxiliary agents, such as D-sorbitol, D-mannose, D-mannitol, and sodium chloride may be used. The aqueous solutions may be combined with appropriate solubilizing agents such as alcohols (ethanol and such), polyalcohols (propylene glycol, PEG, and such), or nonionic surfactants (Polysorbate 80 and HCO-50).

If required, diluents, solubilizers, pH adjusters, soothing agents, sulfur-containing reducing agents, antioxidants, and such may be comprised in the solutions.

Herein, the sulfur-containing reducing agents include, for example, those containing sulfhydryl groups, such as N-acetylcysteine, N-acetylhomocysteine, thioctic acid, thiodiglycol, thioethanolamine, thioglycerol, thiosorbitol, thioglycolic acid and salts thereof, sodium thiosulfate, glutathione, and thioalkanoic acids having one to seven carbon atoms.

Herein, the antioxidants include, for example, erythorbic acid, dibutylhydroxytoluene, butylhydroxyanisole, α-tocopherol, tocopherol acetate, L-ascorbic acid and salts thereof, L-ascorbic acid palmitate, L-ascorbic acid stearate, sodium hydrogen sulfite, sodium sulfite, triamyl gallate, propyl gallate, and chelating agents such as disodium ethylenediamine tetraacetate (EDTA), sodium pyrophosphate, and sodium metaphosphate.

If required, the formulations may be encapsulated within microcapsules (microcapsules of hydroxymethylcellulose, gelatin, poly[methylmethacrylic acid], or such). Alternatively, the formulations may be prepared for colloidal drug delivery systems (liposomes, albumin microspheres, microemulsions, nano-particles, nano-capsules, and such) (see "Remington's Pharmaceutical Science 16th edition", Oslo Ed., 1980). Furthermore, methods of preparing medicaments as sustained-release medicaments are also known, which can be applied to the present invention (Langer et al., J. Biomed. Mater. Res. 1981, 15: 167-277; Langer, Chem. Tech. 1982, 12: 98-105; United States Patent No. 3,773,919; European Patent Application Publication (EP) No. 58,481; Sidman et al., Biopolymers 1983, 22: 547-556; and EP 133,988).

Desired pharmaceutically acceptable carriers used are appropriately selected from those described above or combined depending on the intended use, but are not limited thereto.

When viral particles are prepared using a therapeutic gene as a foreign gene, gene therapy can be performed by administering the viral particles. In the application of the viral particles of the present invention to gene therapy, foreign genes that are expected for their therapeutic effects, endogenous genes that are insufficiently supplied in the patient's body, or the like can be expressed using gene expression methods by either direct administration or indirect (*ex vivo*) administration. Such foreign genes are not particularly limited. The genes may be non-protein-coding nucleic acids such as antisense and ribozyme, as well as protein-encoding nucleic acids.

When a gene encoding an antigen of a bacterium or a virus involved in an infection is used as a foreign gene, immunity can be induced in animals by administering the gene to the animals. That is, the gene can be used as a vaccine.

When used as vaccines, the viral particles of the present invention may be applied to, for example, tumors, infections, and other general diseases. For instance, to treat tumors, genes having therapeutic effects can be expressed in tumor cells or antigen-presenting cells (APC) such as DC cells, using the vector of the present invention. Such genes include cancer antigen Muc-1 or Muc-1-like mucin tandem repeat peptide (United States Patent No. 5,744,144), and melanoma antigen gp100. Treatment using such genes has been shown to be widely applicable to, for example, breast cancer, colon cancer, pancreatic cancer, prostate cancer, lung cancer, and such. It is also effective to combine the genes with cytokines that improve the adjuvant effect. Such genes include, for example, (i) the combination of IL-2 and single-stranded IL-12 (Proc. Natl. Acad. Sci. USA 96 (15): 8591-8596, 1999); (ii) the combination of IL-2 and interferon-y (United States Patent No. 5,798,100); (iii) granulocyte colony stimulating factor (GM-CSF) used alone; and (iv) the combination of GM-CSF and IL-4 aiming at treating brain tumors (J. Neurosurgery 90 (6), 1115-1124 (1999)).

Targets of the treatment of infections include, for example, for influenza, the envelope of highly-virulent H5N1 strain; for Japanese encephalitis, an envelope chimera (Vaccine, vol. 17, No. 15-16, 1869-1882 (1999)); for AIDS, HIV gag and SIV gag proteins (J. Immunology (2000) vol. 164, 4968-4978), as well as HIV envelope proteins, which are orally administered in vaccine treatment, or coated with polylactate-glycol copolymer for administration (Kaneko, H. et al., Virology 267: 8-16 (2000)); regarding cholera, the B subunit (CTB) of cholera toxin (Arakawa T, et al., Nature Biotechnology (1998) 16(10): 934-8; Arakawa T, et al., Nature Biotechnology (1998) 16(3): 292-7); for rabies, the glycoprotein of rabies virus (Lodmell, D. L. et al., 1998, Nature Medicine 4(8): 949-52); and for cervical carcinoma, capsid protein L1 of human papilloma virus 6 (J. Med. Virol., 60, 200-204 (2000)).

All prior art documents cited in the present specification are incorporated herein by reference.

### [Examples]

Hereinbelow, the present invention will be specifically described with reference to Examples, but it is not to be construed as being limited thereto.

### [Example 1] Construction of helper SeV cDNA

### 1.1 Construction of pSeVagp55/ΔF

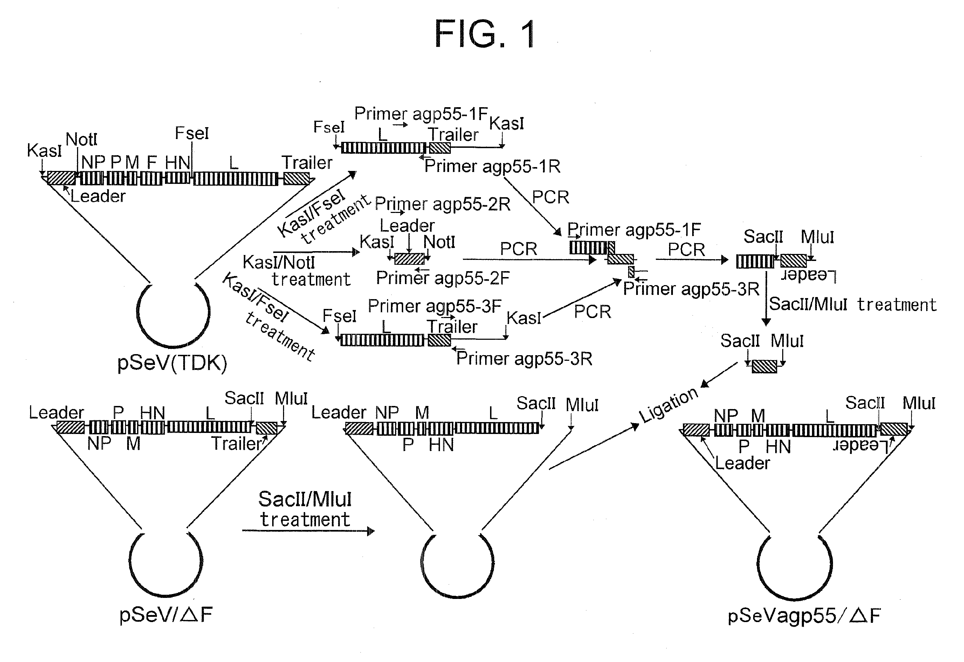

As shown in Fig. 1, pSeV(TDK) was digested with the restriction enzymes *Kas* I and *Fse* I, and purified. The resulting fragment containing the SeV trailer sequence (genomic 5' promoter) was used as a template for PCR. Fragment 1 was obtained by PCR using the following primers: agp55-1F (5'-caggtttgaggatattatacatag, 24 mer (SEQ ID NO: 1)) and agp55-1R (5'-caggattttagtttttcttactattgtc, 28 mer (SEQ ID NO: 2)). Fragment 3 was obtained by PCR using the following primers: agp55-3F (5'-ctcttgtttggtgggtcggcatggcatctc, 30 mer (SEQ ID NO: 3)) and agp55-3R (5'-ttagctcactcattaggcaccccag, 25 mer (SEQ ID NO: 4)). Furthermore, pSeV (TDK) was digested with the restriction enzymes *Kas* I and *Not* I, and purified. The resulting fragment containing the SeV leader sequence (genomic 3' promoter) was used as a template for PCR. Fragment 2 was obtained by PCR using the following primers: agp55-2F (5'-gtaagaaaaactaaaatcctgtataacttc, 30 mer (SEQ ID NO: 5)) and agp55-2R (5'-gccgacccaccaaacaagagaaaaaacatg, 30 mer (SEQ ID NO: 6)). Then, a fragment containing the *Sac*II and *Mlu*I restriction sites was obtained by PCR using Fragments 1, 2, and 3 as templates and the primers agp55-1F and agp55-3R. Finally, the resulting PCR fragment was digested with *Sac* II and *Mlu* I, and then inserted into pSeV/ΔF vector which had also been digested with *Sac* II and *Mlu* I to remove the fragment containing the SeV trailer sequence.
Thus, pSeVagp55/ΔF (SEQ ID NO: 7) was obtained. In the above description, agp55 represents the leader-leader type (in which the trailer has been replaced with the leader), while ΔF represents the deletion of F gene.

### 1.2 Construction of pSeVagp55/(P/C)m/ΔF

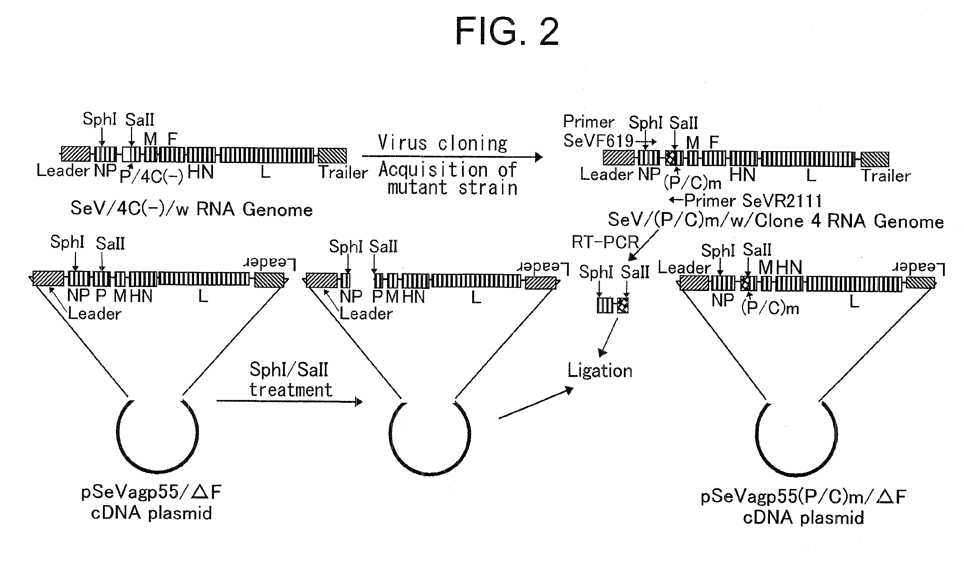

As shown in Fig. 2, the virus SeV/4C(-)/w was cloned using the limiting dilution method. The RNA genome was extracted from each clone. Following RT-PCR, the genomic sequences were determined. Thus, clone 4 that has a number of mutations in P/C genes was obtained. A fragment (SEQ ID NO: 10), which has the *Sph* I restriction site at one end and the *Sal* I restriction site at the other end, was obtained by PCR using as a template the RNA genome of clone 4 and the following primers: SeVF619 (5'-atgggtatcctgcatgcctaggag, 24 mer (SEQ ID NO: 8)) and SeVR2111 (5'-atcgattatcttgggtcgacg, 21 mer (SEQ ID NO: 9)). After treating with *Sph* I and *Sal* I*,* the fragment was purified and inserted into pSeVagp55/ΔF vector by replacing with the fragment the vector sequence which contains the *Sph* I site at one end and the *Sal* I site at the other end. Thus, pSeVagp55/(P/C)m/ΔF was obtained. In the above description, (P/C)m means that mutations have been introduced into P gene and/or C gene. The complete sequence of pSeVagp55/(P/C)m/ΔF is shown in SEQ ID NO: 11.

### [Example 2] Preparation of helper SeV (SeV/ΔF, SeVagp55/ΔF, and SeVagp55/(P/C)m/ΔF)

2.1 Preparation
(1) Cells: the day before, approximately 8e5 BHK-21 cells/well (6-well plate)
(2) 20% FBS/opti-MEM [Cat No. 31985-070, Lot No. 1183337, Invitrogen] (without P&S)
(3) Opti-MEM (stock solution)

2.2 Preparation of transfection solution
(1) DNA solution

| Per well | |
|---|---|
| Opti-MEM (stock solution without supplement) | 250 µl |
| pCAGGS-NP (Z), 1 µg/µl | 0.5 µl |
| pCAGGS-P (Z)/4C(-), 1 µg/µl | 0.5 µl |
| pCAGGS-L (TDK), 1 µg/µl | 2.0 µl |
| pCAGGS-F5R, 1 µg/µl | 0.5 µl |
| pCAGGS-T7, 1 µg/µl | 0.5 µl |
| SeV cDNA, 1 µg/µl | 5 µl |

(2) LipofectAMINE 2000 reagent [Cat No. 11668-019, Lot No. 1188609, Invitrogen] solution

| Per well | |
|---|---|
| Opti-MEM (stock solution without supplement) | 250 µl |
| LipofectAMINE2000 | 18 µl |

(3) Five minutes after preparation, solution (2) was mixed with solution (1). The mixture was left at room temperature for 20 to 30 minutes.
2.3 After washing once with opti-MEM (stock solution) and adding 0.5 ml/well of 20% FBS/opti-MEM, 0.5 ml of the above transfection solution was added to each well containing cells.
2.4 After incubation at 37°C for six hours, 1 ml/well of 10% FBS/G-MEM was added. On the following day, after washing once with VP-SFM, 5 µg/ml trypsin/VP-SFM was added, and the cells were cultured. After three days, the supernatant (P0 virus solution) was collected, and fresh LLC-MK₂/F/Ad cells were infected with the virus (passage from P0 to P1 virus). Subsequently, viruses are produced using LLC-MK₂/F/Ad cells. Supernatants after P1 were examined for viral titers by CIU Assay.

### [Example 3] Design of SeV minigenome and construction of cDNA

### 3.1 Design of SeV minigenome

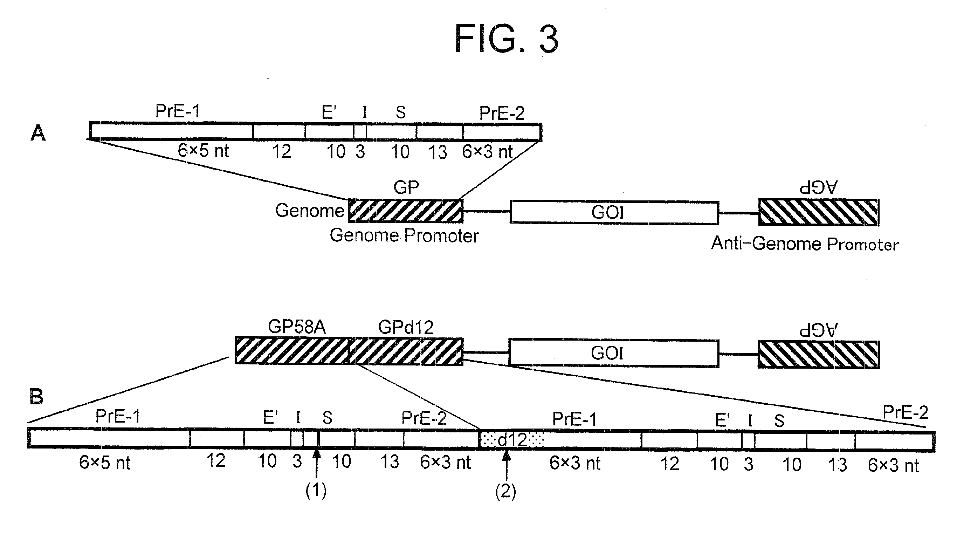

As shown in Fig. 3, the following two types of minigenomes were designed:
SeVminiSP/GOI having a conventional single promoter (GP); and SeVminiDP/GOI having dual promoters (GP58A-GPd12) (see "Nature of a paramyxovirus replication promoter influences a nearby transcription signal", Diane Vullienoz, et al., Journal of General Virology, p171-180, v86, 2005).

### 3.2 Construction of single-promoter (SP) SeV minigenome cDNA

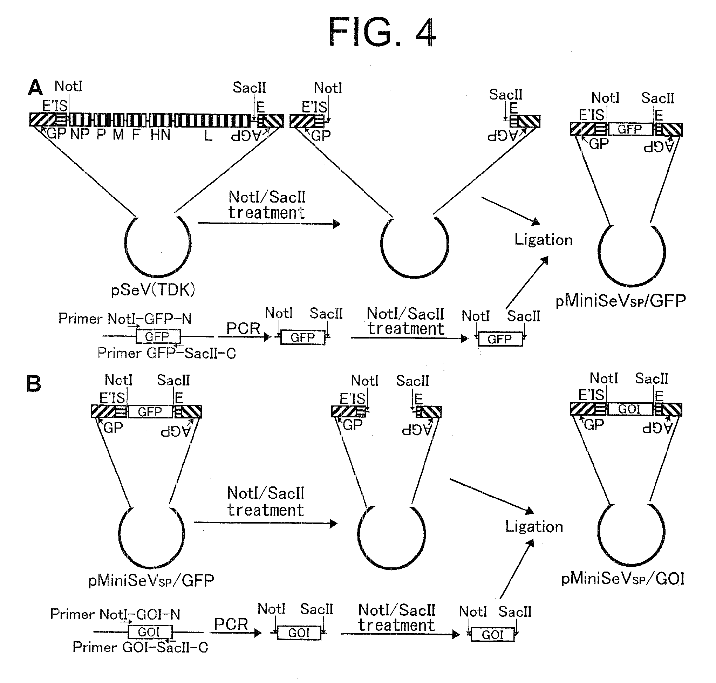

As shown in Fig. 4A, Not I-GFP-Sac II fragment was obtained by PCR using a commercially available GFP plasmid as a template and the following primers: Not I-GFP-N (5'-agttcacgcggccgcagatcttcacgatggtgagcaagggcgaggagctg, 50 mer (SEQ ID NO: 12)) and GFP-Sac II-C (5'-caggtaccgcggagcttcgatcgttctgcacgatagggactaattacttgtacagctcgtccatg, 65 mer (SEQ ID NO: 13)). The resulting fragment was digested with *Not* I and *Sac* II, and purified. Then, the purified fragment was introduced as a GFP insert into pSeV(TDK) vector (containing *Not* I and *Sac* II sites, and the Mini SeV genomic sequence at positions 437 to 670; SEQ ID NO: 14; Japanese Patent Application Kokai Publication No. (JP-A) 2002-272465 (unexamined, published Japanese patent application)), which had also been digested with *Not* I and *Sac* II to remove the fragment containing the NP, P, L, M, F, and HN sequences. Thus, pMiniSeV_{SP}/GFP was obtained.

The genomic sequence of MiniSeV_{SP}/GFP is shown in SEQ ID NO: 15. Positions 1 to 137 and positions 858 to 996 correspond to the MiniSeV genome sequence, while positions 138 to 857 correspond to the ORF sequence of carried GFP.

As seen in Fig. 4B, SeV minigenome cDNA that carries a gene(s) of interest (GOI) other than GFP can be readily constructed by introducing the *Not* I restriction site at one end of the GOI and the *Sac* II site at the other end by PCR using the primers Not I-GOI-N and GOI-Sac II-C, and then replacing the Not I-GFP-Sac II sequence of pMiniSeV_{SP}/GFP with the GOI.

The sequence of the primer Not I-GOI-N is designed to be:
5'-agttcacgcggccgcagatcttcacg (SEQ ID NO: 16) plus the sense sequence of initial 18 to 24 nucleotides including the start codon of the GOI ORF. The primer GOI-Sac II-C is designed to be: 5'-caggtaccgcggagcttcgatcgttctgcacgatagggactaa (SEQ ID NO: 17) plus a linker plus the antisense sequence for terminal 18 to 24 nucleotides including the stop codon of the GOI ORF (adjusted so that the linker length is zero to two nucleotides and the total nucleotide number of the genome is a multiple of six).

### 3.3 Construction of double-promoter (DP) SeV minigenome cDNA

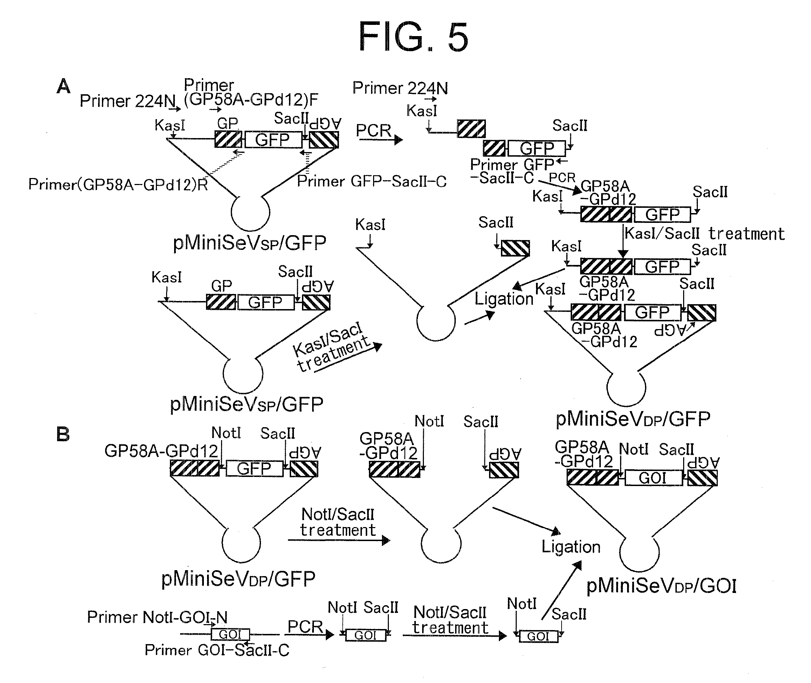

As shown in Fig. 5A, a fragment was obtained by PCR using pMiniSeV_{SP}/GFP as a template and the following primers: 224N (5'-ataccgcatcaggcgccattcg, 22 mer (SEQ ID NO: 18) and (GP58A-GPd12)R (5'-gttttttagggtctggaacctgctcctcagggtggatactttgacactaaaatcctg, 57 mer (SEQ ID NO: 19). Another fragment was obtained by PCR using the same template and the following primers: (GP58A-GPd12)F (5'-ggttccagaccctaaaaaacatgtatgggatatg, 34 mer (SEQ ID NO: 20)) and GFP-Sac II-C (the sequence is shown above). After the above two fragments were purified, PCR was carried out using the two fragments as templates and the primers 224N and GFP-Sac II-C. The resulting fragment was digested with *Kas* I and *Sal* I, and purifed. The purified fragment was inserted into pMiniSeV_{SP}/GFP vector, which had also been digested with *Kas* I and *Sal* I to remove the sequence containing the single promoter. Thus, pMiniSeV_{DP}/GFP was obtained. The sequence of pMiniSeV_{DP}/GFP is shown in SEQ ID NO: 21, positions 437 to 1516 of which correspond to the Mini SeV genome sequence. Of the sequence, positions 658 to 1377 correspond to the ORF sequence of GFP carried.

As shown in Fig. 5B, SeV minigenome cDNA that carries a gene(s) of interest (GOI) other than GFP can readily be constructed by introducing the *Not* I restriction site at one end of the GOI and the *Sac* II site at the other end by PCR using the primers Not I-GOI-N and GOI-Sac II-C (the designs are described above), and then replacing the Not 1-GFP-Sac II sequence of pMiniSeV_{DP}/GFP with the GOI.

### [Example 4] Mechanism of the MiniSeV and SeVΔF particle-coexisting protein expression system.

As shown in Fig. 6, once helper SeV/ΔF (SeVagp55/ΔF is preferred) and SeV minigenome cDNA plasmid that carry a gene(s) of interest (GOI) (pSeVmini/GOI) are introduced into production cells expressing the F protein (which can be F5R mutant), a fusion protein between T7 RNA polymerase and SeV, the RNA genome of MiniSeV/GOI is transcribed from pSeVmini/GOI by the action of T7 R-NA polymerase, and RNPs of MiniSeV/GOI are formed and replicated using the NP, P, and L proteins expressed from SeV/ΔF that serves as a helper. Preferred production cells are the BHK-21 cells, but are not limited thereto. The methods of expressing T7 RNA polymerase and F/F5R are not particularly limited. The methods include, for example, transient expression methods using plasmids or the like, and methods using cells with sustained expression of T7 RNA polymerase and F/F5R. Although cells with sustained expression of both T7 RNA polymerase and F/F5R proteins are preferred, cells expressing only one of the two proteins can be used. The RNPs of MiniSeV/GOI are packaged as infectious MiniSeV/GOI particles using M and HN expressed from helper SeV/ΔF and F/F5R expressed from the production cells, and released into culture supernatant together with infectious particles of helper SeV/ΔF. When the culture supernatant containing both infectious MiniSeV/GOI and helper SeV/ΔF particles is collected and target cells are infected with the supernatant, the gene(s) of interest (GOI) carried by MiniSeV/GOI is expressed using the transcription/replication system of helper SeV/ΔF (NP, P, and L proteins).

Herein, SeV/ΔF is an example of the helper SeV. The helper is not limited to this example. While the helper may be a type lacking any one or more of the NP, P, L, M, F, and HN proteins constituting SeV, the lacking proteins should be separately expressed by the production cells.

### [Examples 5] Influence of the design of helper SeV/ΔF on the productivity of coexisting MiniSeV and SeV/ΔF particles

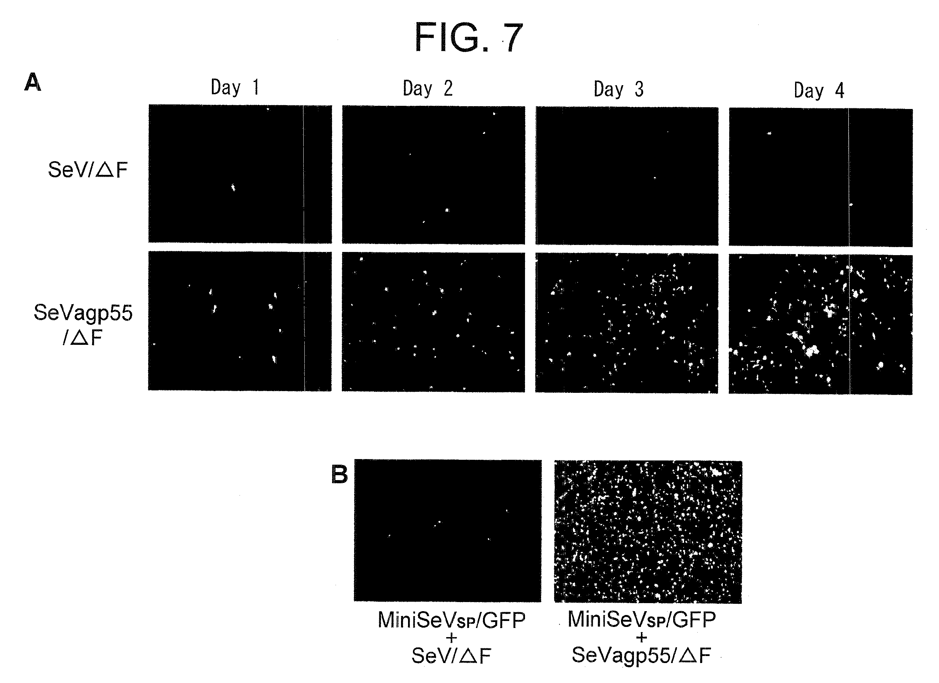

BHK/T7/Ad cells (cells with sustained expression of T7 RNA polymerase) plated at 3e5 cells/well (12-well collagen-coated plates, IWAKI) on the previous day were infected with SeV/ΔF or SeVagp55/ΔF (a leader/leader type in which the sequence of 55 nucleotides at the 5' end of the SeV genome had been replaced with the antisense sequence of 55 nucleotide of the 3' end) at moi 10 for one hour. Then, pCAGGS/F5R (1 µg/well) and pMiniSeV_{SP}/GFP (2 µg/well) were introduced into the cells (transfection reagent: Lipofectamine 2000, Invitrogen, Lipofectamine/DNA=2 µl/µg). After a two-day culture (10% FBS/G-MEM medium), the cells were washed once with the serum-free medium VP-SFM. Then, the medium was exchanged for VP-SFM supplemented with 5 µg/ml trypsin. The culture supernatant was collected on day 4. GFP images were photographed every day until day 4 (Fig. 7A). LLC-MK₂ cells (6-well plate) were infected with 100 µl/well of the collected culture supernatant. GFP images were photographed on day 2 (Fig. 7B).

As shown in Fig. 7A, the efficiency of protein expression (the ratio of GFP-expressing cells) by the minigenome was higher when SeVagp55/ΔF was used as a helper SeV, as compared to standard SeV/ΔF. Meanwhile, the number of GFP-expressing cells shown in Fig. 7B corresponds to the amount of MiniSeV in the coexisting particles produced. The productivity of the coexisting particles was much higher when SeVagp55/ΔF was used than when SeV/ΔF was used.

### [Example 6] Influence of the design of MiniSeV cDNA on the productivity of coexisting MiniSeV and SeV/ΔF particles

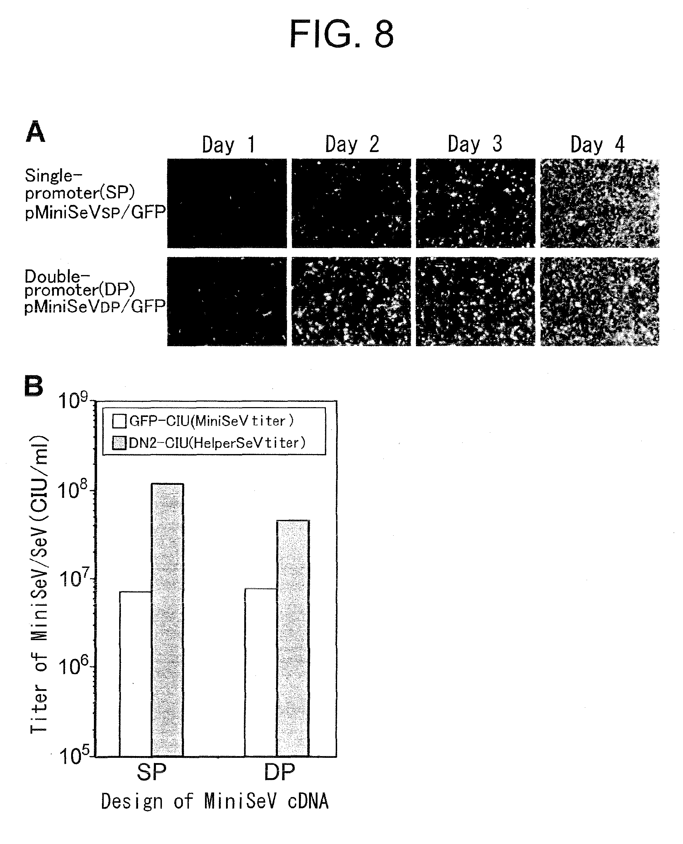

BHK/T7/Ad cells (cells with sustained expression of T7 RNA polymerase) plated at 3e5 cells/well (12-well collagen-coated plates, IWAKI) on the previous day were infected with SeVagp55/ΔF at moi 10 for one hour. Then, pCAGGS/F5R (1 µg/well) and the minigenome cDNA (2 µg/well) were introduced into the cells (transfection reagent: Lipofectamine 2000, Invitrogen, Lipofectamine/DNA=2 µl/µg). The two types of MiniSeV cDNAs, pMiniSeV_{SP}/GFP and pMiniSeV_{DP}/GFP, were used. After a two-day culture (10% FBS/G-MEM medium), the cells were washed once with the serum-free medium VP-SFM.
Then, the medium was exchanged for VP-SFM supplemented with 5 µg/ml trypsin. The culture supernatant was collected on day 4. GFP images were photographed every day until day 4 (Fig. 8A). CIU assay was carried out using the collected culture supernatant described above (Fig. 8B). Specifically, after 10-fold serial dilutions of the sample, LLC-MK₂ cells in 6-well plates were infected with 100 µl/well of the diluted samples. After infection for an hour, the cells were cultured for two days in serum-free medium. Then, the titer of MiniSeV was determined by counting GFP-expressing cells. Furthermore, antibody staining was carried out using the anti-SeV antibody DN2, and the titer of helper SeV was determined by counting DN2-positive cells.

As shown in Fig. 8, the efficiency of GFP expression was high in both the SP design and the DP design. With the DP design, the productivity for MiniSeV (GFP-CIU) was higher and the amount ratio of helper SeV/MiniSeV was lower.

### [Example 7] Comparison of the productivity of coexisting particles between SeVagp55/ΔF and SeVagp55/(P/C)m/ΔF

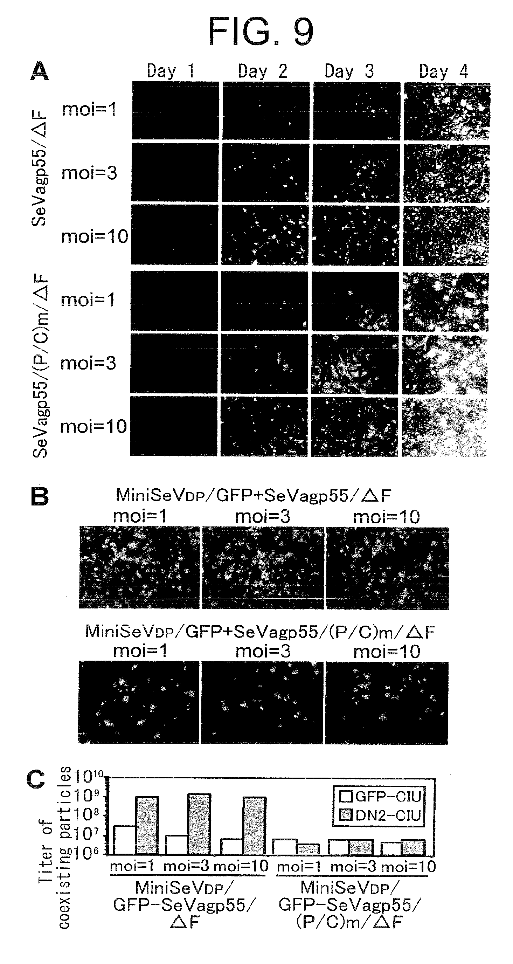

BHK/T7/Ad cells (cells with sustained expression of T7 RNA polymerase) plated at 3e5 cells/well (12-well collagen-coated plates, IWAKI) on the previous day were infected with SeVagp55/ΔF or SeVagp55/(P/C)m/ΔF at moi 1, 3, or 10 for one hour. Then, pCAGGS/F5R(1 µg/well) and pMiniSeV_{DP}/GFP (2 µg/well) were introduced into the cells (transfection reagent: Lipofectamine 2000, Invitrogen, Lipofectamine/DNA=2 µl/µg). After a two-day culture (10% FBS/G-MEM medium), the cells were washed once with the serum-free medium VP-SFM. Then, the medium was exchanged for VP-SFM supplemented with 5 µg/ml trypsin. The culture supernatant was collected on day 4. GFP images were photographed every day until day 4 (Fig. 9A). LLC-MK₂ cells (6-well plate) were infected with 100 µl/well of the collected culture supernatant. GFP images were photographed on day 2 (Fig. 9B). Furthermore, CIU assay was carried out using the collected culture supernatant described above (Fig. 9C).

As shown in Fig. 9, the productivity of MiniSeV was higher when the coexisting particles were produced using SeVagp55/ΔF as the helper SeV. The amount ratio of helper SeV/MiniSeV was also higher (about 10:1). Meanwhile, when the coexisting particles were produced using SeVagp55/(P/C)m/ΔF, the productivity of MiniSeV was comparatively lower, while the amount ratio of helper SeV/MiniSeV was very low (about 1:1). Thus, the two types of helper SeV can be selectively used depending on the purpose.

### [Example 8] Improvement of the amount ratio of helper SeV/MiniSeV

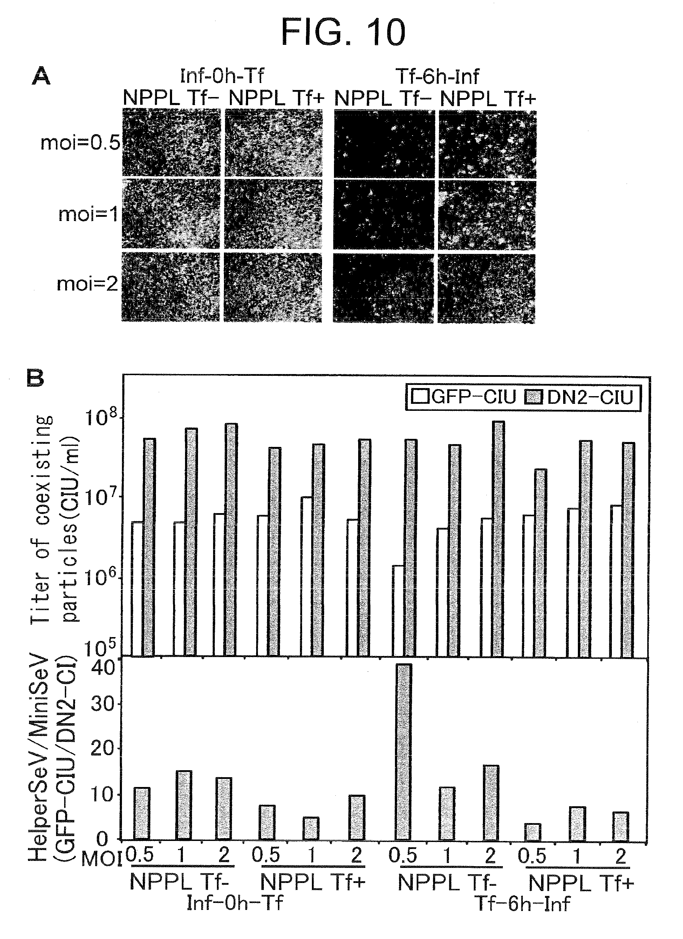

BHK-21 cells plated at 8e5 cells/well (6-well collagen-coated plates, IWAKI) on the previous day were infected with SeVagp55/ΔF at moi 0.5, 1, or 2 for one hour. Then, [pCAGGS/T7 (1 µg/well), pCAGGS/F5R (4 µg/well) and pMiniSeV_{DP}/GFP (5 µg/well)] or [pCAGGS/NP, P, L (0.5, 0.5, 2 µg/well), pCAGGS/T7 (1 µg/well), pCAGGS/F5R (4 µg/well), and pMiniSeV_{DP}/GFP (5 µg/well)] were introduced into the cells (transfection reagent: Lipofectamine 2000, Invitrogen, Lipofectamine/DNA=2 µl/µg). Separately, six hours after introduction of either of the above two plasmid combinations, BHK-21 cells were infected with SeVagp55/ΔF at moi 0.5, 1, or 2. After a one-day culture (10% FBS/G-MEM medium), the cells were washed once with the serum-free medium VP-SFM. Then, the medium was exchanged for VP-SFM supplemented with 5 µg/ml trypsin. On day 3, GFP images were photographed (Fig. 10A), and the culture supernatant was collected. CIU assay was carried out using the collected culture supernatant described above (Fig. 10B).

As shown in Fig. 10, when the plasmids that express NP, P, and L were co-introduced with the MiniSeV cDNA, there was no significant change in the amount of MiniSeV in the coexisting particles produced. However, the amount ratio of helper SeV/MiniSeV was observed to be decreased.

### [Example 9] In vitro expression using coexisting particles

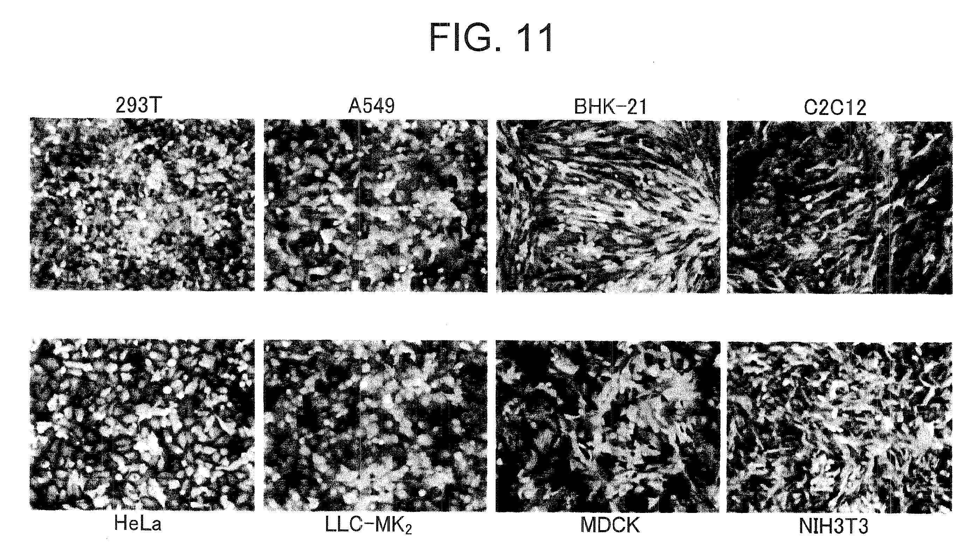

293T, A549, BHK-21, C2C12, HeLa, LLC-MK₂, MDCK, and NIH3T3 cells were cultured until about 90% confluency in 24-well collagen-coated plates. The cells were infected with the coexisting MiniSeV_{DP}/GFP and SeVagp55/ΔF particles (2e6 GFP-CIU/ml and 7.7e6 DN2-CIU/ml, respectively) at moi 3, and then cultured at 32°C. GFP images were photographed on day 2 (Fig. 11).

As shown in Fig. 11, with the coexisting MiniSeV_{DP}/GFP and SeVagp55/ΔF particles, the transfer and expression efficiency of GFP was high in all of the eight cell types tested.

### [Example 10] In vivo expression using coexisting particles

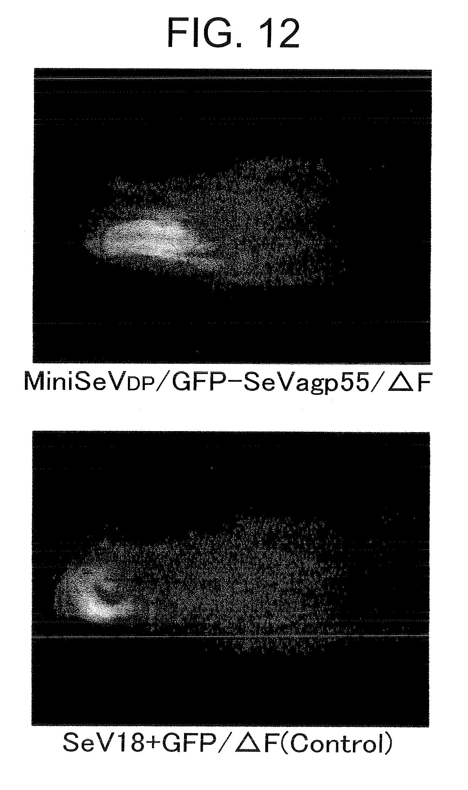

Intranasal administration to mice (n=3) was carried out using the coexisting MiniSeV_{DP} and GFP-SeVagp55/ΔF particles (5e6 GFP-CIU/head). The same dose of SeV18+GFP/ΔF was administered as a control. On day 2, the mice were dissected to obtain nasal tissues, and GFP images were photographed (Fig. 12).

As shown in Fig. 12, when the coexisting MiniSeV_{DP} and GFP-SeVagp55/ΔF particles were used, high *in vivo* expression (intranasal GFP expression) was achieved.

### [Example 11]

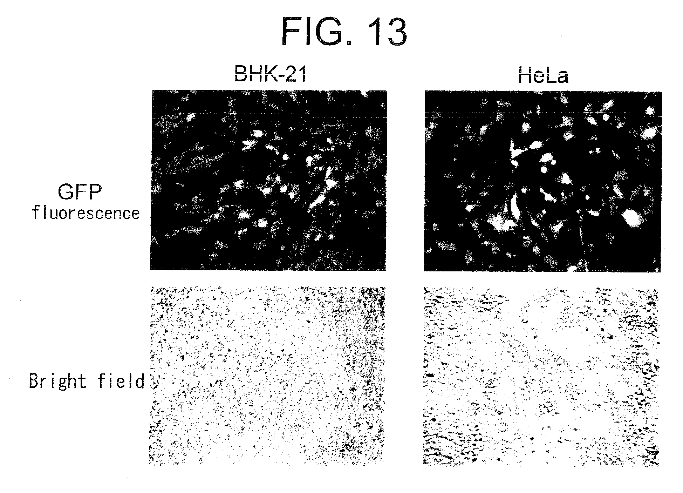

BHK-21 and HeLa cells were cultured until about 90% confluency in 12-well collagen-coated plates. The cells were infected with infectious SeVagp55/T7/ΔF particles as the helper-SeV at moi 20 for one hour. Then, MiniSeV_{DP}/GFP cDNA plasmid was introduced using Lipofectamine 2000 reagent [Lipofectamine 2000/DNA=2.0 (µl/µg), 2 µg DNA/well]. On day 2, GFP images were photographed (Fig. 13).

As shown in Fig. 13, high-efficiency expression of minigemome proteins was achieved by introducing MiniSeV_{DP}/GFP cDNA plasmid and infectious SeVagp55/T7/ΔF particles into target cells.

### [Example 12] Coexisting particle system using RNA polymerase I promoter

Since SeV vectors use the T7 promoter, T7 polymerase has to be supplied at the time of viral reconstitution. To produce the coexisting particles in the Examples described above, T7 polymerase has to be supplied by a method of introducing a minigenome cDNA into production cells together with an expression plasmid for T7 polymerase, or a method using helper SeV that carry the T7 polymerase gene. The former method may be disadvantageous in terms of cost and efficiency of production, since a number of different types of plasmids are introduced. In addition, the latter method can be disadvantageous in analysis of protein function since T7 polymerase carried by helper SeV and a gene(s) of interest (GOI) carried by MiniSeV are inevitably expressed simultaneously. To avoid the above problems, an alternative coexisting particle system using the RNA polymerase I promoter instead of the T7 promoter was developed.

### 12.1 Construction of a minigenome cDNA plasmid using the RNA polymerase I promoter

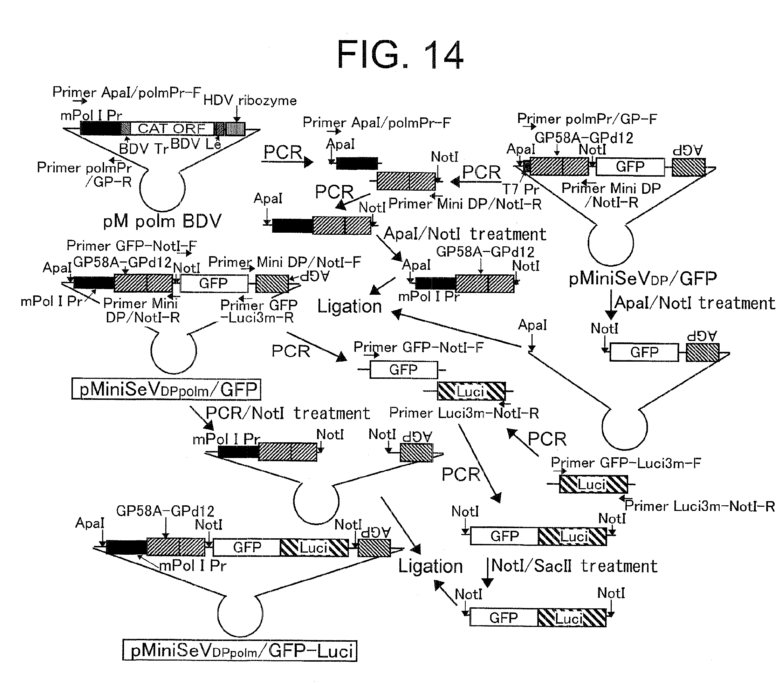

As shown in Fig. 14, the Apa I-polmPr fragment was obtained by PCR using pMpolBDV plasmid as a template and the following primers: Apa I/polmPr-F (5'-gtacgggcccgtacgtctgaggccgagggaaag, 33 mer (SEQ ID NO: 31)) and polmPr/GP-R (5'-ctcttgtttggtacctatctccaggtccaatagg, 34 mer (SEQ ID NO: 32)). In addition, the DP-Not I fragment was obtained by PCR using pMiniSeVDP/GFP as a template and the following primers: polmPr/GP-F (5'-gacctggagataggtaccaaacaagagaaaaaac, 34 mer (SEQ ID NO: 33)) and MiniDP/Not I-R (5'-gactagcggccgcgtgaactttggcag, 27 mer (SEQ ID NO: 34)). Then, the Apa I-polmDP-Not I fragment, which was obtained by PCR using the Apa I-polmPr fragment and the DP-Not I fragment as templates and the primers Apa I/polmPr-F and MiniDP/Not I-R, was digested with *Apa* I and *Not* I, and then purified. The purified fragment was inserted into pMiniSeVDP/GFP, which had also been digested with *Apa* I and *Not* I to remove the *Apa* I-*Not* I fragment. Thus, pMiniSeVDPpolm/GFP was obtained.

The size of the GFP gene is 0.7 kb, which is relatively short. To assess the productivity of coexisting particles with longer-sized genes, a fusion protein between GFP and luciferase (GFP-Luci, 2.3 kb) was constructed as a model. As shown in Fig. 14, the Not I-GFP fragment was obtained by PCR using pMiniSeVDP/GFP as a template and the following primers: GFP-Not I-F (5'-atatgcggccgcaatggtgagcaagggcgaggag, 34 mer (SEQ ID NO: 35)) and GFP-Luci3m-R (5'-tggcgtcttccttgtacagctcgtccatgccg, 32 mer (SEQ ID NO: 36)). In addition, the Luci3m-Not I fragment was obtained by PCR using pSeV18+Luci3m/ΔF as a template and the following primers: GFP-Luci3m-F (5'-acgagctgtacaaggaagacgccaaaaacataaag, 35 mer (SEQ ID NO: 37)) and Luci3m-Not I-R (5'-atatgcggccgcttacacggcgatctttccgc, 32 mer (SEQ ID NO: 38)). Then, the Not I-GFP-Luci-Not I fragment was obtained by overlap PCR using the Not I-GFP fragment and the Luci3m-Not I fragment as templates and the primers GFP-Not I and Luci3m-Not I. The resulting fragment was digested with *Not* I, then used as an insert. The Not I-MiniSeVDPpolm-Not I fragment was obtained by PCR using pMiniSeVDPpolm/GFP as a template and the following primers: MiniDP/Not I-F (5'-tcacgcggccgctagtccctatcgtgcagaacg, 33 mer (SEQ ID NO: 39)) and MiniDP/Not I-R (5'-gactagcggccgcgtgaactttggcag, 27 mer (SEQ ID NO: 40)). The resulting fragment was digested with *Not* I, then used as a vector. pMiniSeVDPpolm/GFP-Luci was obtained by introducing the GFP-Luci insert (2384nt (SEQ ID NO: 41)) into the MiniSeVDP vector.

### 12.2 Production of coexisting particles using the RNA polymerase I promoter

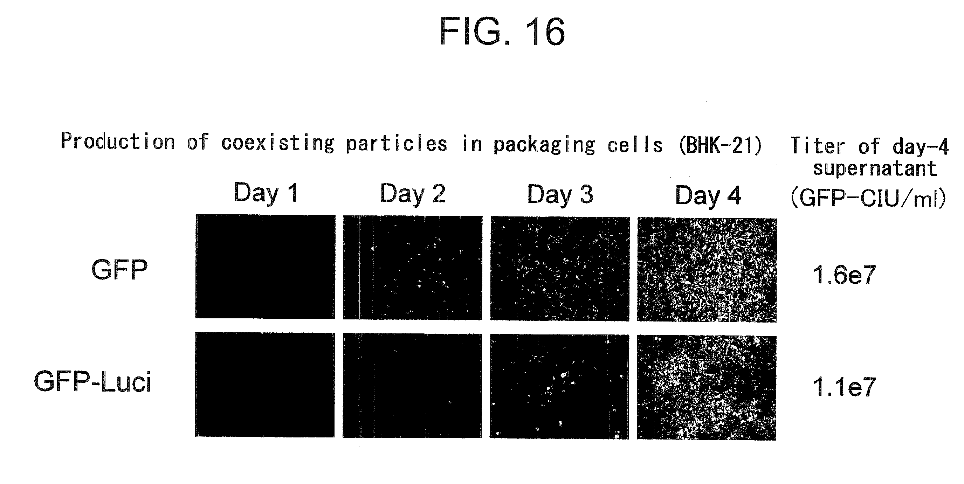

BHK-21 cells plated at 1e6 cells/well (6-well collagen-coated plates, IWAKI) on the previous day were infected with SeVagp55/ΔF at moi 10 for one hour. Then, [pCAGGS/F5R (5 µg/well) and pMiniSeV_{DPpolm}/GFP (5 µg/well)] were introduced into the cells (transfection reagent: Lipofectamine 2000, Invitrogen, Lipofectamine/DNA=2 µl/µg). Alternatively, a day after introduction of [pCAGGS/NP, P, L (0.5, 0.5, 2 µg/well), pCAGGS/F5R (4 µg/well) and pMiniSeV_{DPpolm}/GFP-Luci (5 µg/well)] (transfection reagent: Lipofectamine 2000, Invitrogen, Lipofectamine/DNA=1.5 µl/µg), the cells were infected with SeVagp55/ΔF at moi 10. After a one-day culture (10% FBS/G-MEM medium), the cells were washed once with the serum-free medium VP-SFM. Then, the medium was exchanged for VP-SFM supplemented with 3 µg/ml trypsin. Subsequently, every day, the medium was exchanged and GPF images were photographed. CIU assay was carried out using the Day-4 culture supernatant (Fig. 16).

As shown in Fig. 16, the coexisting particles containing MiniSeV with 1e7 GFP-CIU/ml or more were produced using both the shorter gene (GFP, 0.7kb) and the longer gene (GFP-Luci, 2.3kb). Thus, high productivity of coexisting particles was confirmed to be achieved with the RNA polymerase I promoter.

### [Example 13] System with coexisting particles that carry an HA-Tag-attached gene

When the system with coexisting particles of the present invention carries a marker gene such as GFP or a gene encoding an antibody that enables immunostaining, the titer of MiniSeV in the coexisting particles can be readily determined. However, when the system carries a gene whose function is unknown or the system does not carry a gene encoding an antibody that enables immunostaining, the titer of MiniSeV in the coexisting particles is difficult to determine. To solve this problem, a system with coexisting particles that carry an HA-Tag-attached gene (GFP) was developed.

### 13.1 Construction of minigenome cDNA that carries an HA-Tag-attached gene (GFP)

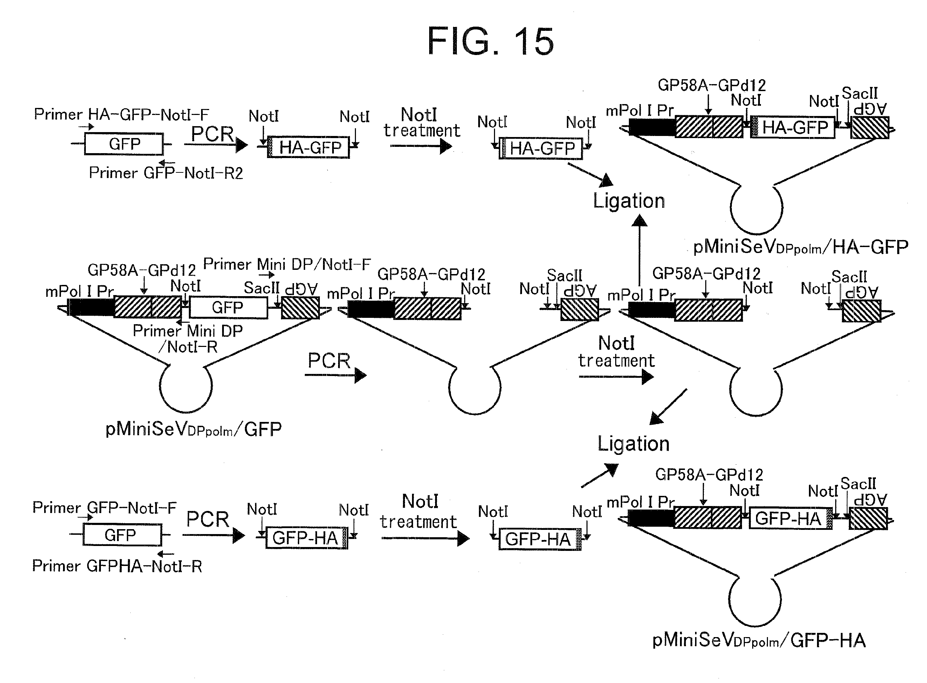

As shown in Fig. 15, a minigenome cDNA was constructed to attach an HA-Tag to the N terminus of GFP. First, the Not I-HA-GFP-Not I fragment was obtained by PCR using a GFP-carrying plasmid as a template and the following primers: HA-GFP-Not I-F (5'-atagcggccgcaatgtacccatacgacgtcccagactacgctgtgagcaagggcgaggagctg, 63 mer (SEQ ID NO: 42)) and GFP-Not I-R2 (5'-tatgcggccgcttacttgtacagctcgtccatg, 33 mer (SEQ ID NO: 43)). In addition, the Not I-GFP-HA-Not I fragment was obtained by PCR using the same template and the primers GFP-Not I-F (see Example 12 above) and GFP-HA-Not I-R (5'-tatgcggccgcttaagcgtagtctgggacgtcgtatgggtacttgtacagctcgtccatgccg, 63 mer (SEQ ID NO: 44)). The two types of fragments (Not I-HA-GFP-Not I and Not I-GFP-HA-Not I) were digested with *Not* I, and introduced as an insert into the Not I-MiniSeVDPpolm-Not I fragment obtained in Example 12 above, which had also been digested with *Not*I. Thus, pMiniSeVDPpolm/HA-GFP and pMiniSeVDPpolm/GFP-HA were obtained.

### 13.2 Production of coexisting particles that carry an HA-Tag-attached gene (GFP)

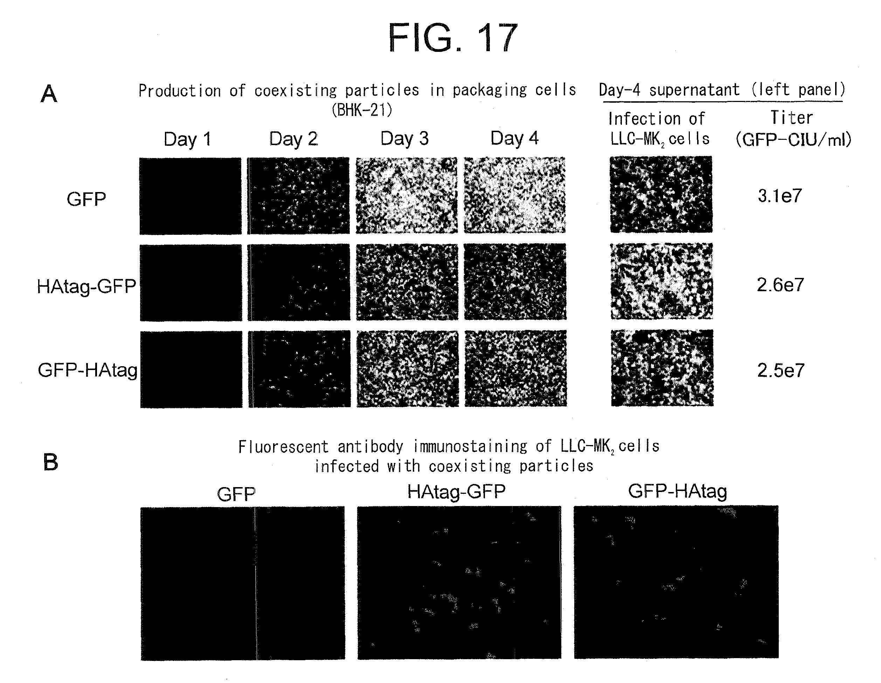

BHK-21 cells plated at 1e6 cells/well (6-well collagen-coated plates, IWAKI) on the previous day were infected with SeVagp55/ΔF at moi 10 for one hour. Then, [pCAGGS/F5R (5 µg/well) plus any one of pMiniSeV_{DPpolm}/GFP (5 µg/well), pMiniSeV_{DPpolm}/HA-GFP (5 µg/well), and pMiniSeV_{DPpolm}/GFP-HA (5 µg/well)] were introduced into the cells (transfection reagent: Lipofectamine 2000, Invitrogen, Lipofectamine/DNA=1.5 µl/µg). After a-day culture (10% FBS/G-MEM medium), the cells were washed once with the serum-free medium VP-SFM. Then, the medium was exchanged for VP-SFM supplemented with 3 µg/ml trypsin. Subsequently, every day, the medium was exchanged and GPF images were photographed. CIU assay was carried out using the Day-4 culture supernatant (Fig. 17). Furthermore, LLC-MK2 cells infected with the coexisting particles were immunostained using a rabbit anti-HA-Tag antibody as a primary antibody and an Alexa Fluor 594 goat anti-rabbit IgG antibody as a secondary antibody (Fig. 17).

As shown in Fig. 17A, when an HA-Tag was attached to the N or C terminus of GFP, the coexisting particles were produced at the same level as that of the control (carrying GFP without any HA-Tag). Furthermore, as shown in Fig. 17B, it was found that cells infected with the coexisting particles that carry an HA-Tag-attached gene (GFP) could be specifically stained by fluorescent antibody staining using an HA-Tag antibody.

### Industrial Applicability

The protein expression system with coexisting MiniSeV and SeV particles, which was developed by the present inventors, shares the advantages of conventional viral vectors, *i.e.,* high efficiency of gene transfer and expression, as well as the advantages of non-viral vectors, *i.e.,* simplicity in preparation, capability of large-scale production in a short term, and low cost. That is, the expression system of the present invention is expected to be applicable to a wide variety of fields such as production of useful proteins, antibody preparation, functional analysis of proteins, gene therapy, and gene vaccination, since the system has advantages of both conventional non-viral vectors and viral vectors.

Furthermore, the use of protein-defective types of helper viral vectors is superior in biosafety, since there is no risk of re-release of infectious particles after infection of target cells with the coexisting particles.

## Claims

1. A method of expressing a foreign gene(s) in target cells, said method comprising the steps of:
(a) introducing into production cells a helper minus-strand RNA viral vector and a minus-strand RNA viral minigenome that carries the foreign gene(s);
(b) collecting viral particles in which either the helper minus-strand RNA viral vector or the minus-strand RNA viral minigenome is packaged, wherein the vector and the minigenome are produced in said production cells; and
(c) introducing into the target cells the viral particles collected in step (b).

2. The method of claim 1, wherein step (a) comprises further introducing into the production cells a vector that expresses NP, P, and L proteins.

3. The method of claim 1, wherein the helper minus-strand RNA viral vector is a ribonucleoprotein (RNP) complex of a virus-like particle (VLP).

4. The method of claim 1, wherein the minus-strand RNA viral minigenome has one or more promoters.

5. The method of claim 4, wherein at least one of the promoters is the RNA polymerase I promoter.

6. The method of any one of claims 1 to 5, wherein the minus-strand RNA virus is a paramyxovirus.

7. The method of claim 6, wherein paramyxovirus NP, P, and L proteins are expressed from the helper minus-strand RNA viral vector or the minus-strand RNA viral minigenome.

8. The method of claim 6, wherein the helper minus-strand RNA viral vector is a vector comprising a single-stranded minus-strand RNA that has been altered so as not to express one or more of paramyxovirus F, M, and HN proteins, and wherein the 5'-end trailer region of the single-stranded minus-strand RNA has been replaced with a leader region.

9. The method of claim 8, wherein the production cells express one or more of F, M, and HN proteins, and wherein the helper minus-strand RNA viral vector has been altered so as not to express proteins expressed by the production cells.

10. The method of claim 8 or 9, wherein the helper minus-strand RNA viral vector has been altered so as not to express F protein.

11. The method of any one of claims 8 to 10, wherein the helper minus-strand RNA viral vector has a mutation(s) in P gene and/or C gene.

12. The method of claim 11, wherein the helper minus-strand RNA viral vector comprises:
(a) a single-stranded minus-strand RNA comprising the nucleotide sequence of SEQ ID NO: 10; or
(b) a single-stranded minus-strand RNA that hybridizes under stringent conditions to the complementary strand of a minus-strand RNA comprising the nucleotide sequence of SEQ ID NO: 10.

13. The method of claim 6, wherein the paramyxovirus is Sendai virus.

14. The method of claim 6, wherein the helper minus-strand RNA viral vector is a wild type Sendai virus vector.

15. The method of any one of claims 1 to 14, wherein the production cells express T7 RNA polymerase.

16. The method of any one of claims 1 to 15, wherein the foreign gene(s) is expressed in target cells *in vitro.*

17. The method of claim 16, wherein the target cells are 293T cells, A549 cells, BHK-21 cells, C2C12 cells, HeLa cells, LLC-MK₂ cells, MDCK cells, or NIH3T3 cells.

18. The method of any one of claims 1 to 15, wherein the foreign gene(s) is expressed in target cells *in vivo.*

19. A helper minus-strand RNA viral vector comprising a single-stranded minus-strand DNA that has been altered so as not to express one or more of paramyxovirus F, M, and HN proteins, and wherein the 5'-end trailer region of the single-stranded minus-strand RNA has been replaced with a leader region.

20. A minus-strand RNA viral minigenome that carries a foreign gene(s).

21. A method of expressing a foreign gene(s) in target cells, said method comprising the step of introducing into the target cells the helper minus-strand RNA viral vector of claim 19 and the minus-strand RNA viral minigenome of claim 20.

22. A method of producing viral particles, said method comprising the steps of:
(a) introducing into production cells a helper minus-strand RNA viral vector and a minus-strand RNA viral minigenome that carries a foreign gene(s); and
(b) collecting viral particles in which either the helper minus-strand RNA viral vector or the minus-strand RNA viral minigenome is packaged, wherein the vector and the minigenome are produced in said production cells.
